(19) Europäisches Patentamt
European Patent Office
Office européen des brevets

(11) **EP 2 487 248 A1**

(12) **EUROPEAN PATENT APPLICATION**

(43) Date of publication:
**15.08.2012 Bulletin 2012/33**

(51) Int Cl.:
***C12Q 1/00*** (2006.01)  ***G01N 33/574*** (2006.01)
***C12Q 1/02*** (2006.01)

(21) Application number: **12166973.3**

(22) Date of filing: **10.05.2007**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **10.05.2006 US 799558 P**

(62) Document number(s) of the earlier application(s) in accordance with Art. 76 EPC:
**07762107.6 / 2 021 491**

(71) Applicant: **The Board of Regents of the University of Texas System Austin, TX 78701 (US)**

(72) Inventors:
• **Weigum, Shannon Austin, TX 78748 (US)**

• **Floriano, Pierre N. Austin, TX 78749 (US)**
• **Christodoulides, Nicolaos Austin, TX 78749-4250 (US)**
• **McDevitt, John T. Austin, TX 78735 (US)**

(74) Representative: **Dehmel, Albrecht Dehmel & Bettenhausen Patentanwälte Herzogspitalstrasse 11 80331 München (DE)**

Remarks:
This application was filed on 07-05-2012 as a divisional application to the application mentioned under INID code 62.

(54) **Detecting tumor biomarker in oral cancer**

(57) Methods and device for detecting the presence of tumor biomarkers in oral squamous cell carcinoma. A membrane based cell capture device allows deliver of cell samples and reagents to the membrane.

FIG. 30

**Description**

**I. FIELD OF THE INVENTION**

The present invention relates generally to cellular biology, medicine, and diagnostic assays and systems. The present invention relates to detecting tumor biomarkers tumor biomarkers in oral cancers.

**II. BACKGROUND**

The development of smart sensors capable of discriminating different analytes, toxins, and bacteria has become increasingly important for clinical, environmental, health and safety, remote sensing, military, food/beverage, and/or chemical processing applications. Some sensors have been fashioned for single analyte detection. Other sensors are capable of solution phase multi-analyte detection. Latex agglutination tests ("LATs") are used to detect many different types of analytes in clinical analyses. LATs employ colloidal polymer microspheres to determine the presence (or absence) of analytes. Commercially available LATs for more than 60 analytes are used routinely for the detection of infectious diseases, illegal drugs, and pregnancies. LATs generally operate on the principle of agglutination of latex particles (*e.g.*, colloidal polymer microspheres). LATs are set up such that agglutination occurs when antibody-derivatized latex particles become effectively "cross-linked" by a foreign antigen, resulting in the attachment of the particle to, or the inability of the particle to pass through, a filter. The cross-linked latex particles are then detected colorimetrically upon removal of the antigen carrying solution.

More recently, "taste chip" sensors have been employed that are capable of discriminating mixtures of analytes, toxins, and/or bacteria in medical, food/beverage, and environmental solutions. Certain sensors of this type are described in U.S. Patent Application Publication No. 20020197622 to McDevitt et al.*,*

Cancer has surpassed heart disease as a leading cause of death in people under 85 years of age. Beyond prevention, early detection is a crucial determinant for successful treatment and survival of cancer.

High-risk human papilomavirus (HPV) infection has recently been associated with oral cancer development.

**SUMMARY OF THE INVENTION**

Various embodiments of the invention include systems, methods, and apparatuses to analyze one or more biological samples containing one or more analytes, in particular tumor biomarkers. Samples may be cell suspensions or fluid samples. In some embodiments, an analyte-detection system is capable of analysis of a sample that includes individual analytes and/or mixtures of analytes. In some embodiments, the analytes include lymphocytes, epithelial cells or other cells or parts thereof that may be present in body fluids. The analyte-detection system may include a cartridge.

Disclosed is an optical biosensor for rapid, non-invasive analysis of body fluids for biomarkers, such as saliva for oral epithelial tumor biomarkers. This lab-on-a-chip membrane-based sensor is designed to capture cells or particles from biological fluids or biopsy suspensions directly into a flow cell imaging chamber where the cells undergo assay-specific immunolabeling and optical imaging. Intensity contouring using custom image analysis macros identifies cells for measurement and correlation of multiple parameters, factors, or indicators on a single-cell basis. The detection assay integrates recently identified early tumor biomarkers, such as epidermal growth factor receptor and DNA aneuploidy, with traditional cellular examination, to provide a cancer-risk profile that encompasses a large range of tumor progression phenotypes, potentially increasing the method's sensitivity over conventional pathology.

Unlike immunophenotyping using a flow cytometer, the lab-on-a-chip optical biosensor can provide both a molecular and morphological pattern within individual cells and improve spatial resolution for high complexity measurements using subcellular features. In addition, the fixed position of cells on the membrane allows individual cells to be relocated and reanalyzed following additional staining procedures, thereby expanding the capacity of the biosensor for multiplexing. Ongoing efforts at sensor and analyzer miniaturization would make these immunophenotyping assays rapid, highly sensitive, and cost effective for point-of-care use, thus increasing access to diagnostic testing for optimal cancer treatment and recovery.

In some embodiments, the cartridge includes one or more collection regions, one or more fluid delivery systems, one or more channels, one or more reagent regions, one or more reservoirs, a detection region, or combinations thereof. The detection region may include one or more detection systems. In some embodiments, one or more collection regions, one or more detection systems, one or more fluid delivery systems, one or more channels, one or more reagent regions, and one or more reservoirs are: coupled to; at least partially positioned on; or at least partially positioned in the cartridge. In some embodiments, one or more collection regions, one or more detection systems, one or more fluid delivery systems, one or more channels, one or more reagent regions, and one or more reservoirs are at least partially contained in a body of the cartridge. In some embodiments, a body of the cartridge includes a plurality of layers coupled together.

In some embodiments, the body of the cartridge includes openings. The openings may be configured to receive one or more components used to facilitate analyte detection. One or more channels may couple the openings together. In some embodiments, one or more collections regions, one or more of the detection systems, one or more fluid packages, or combinations thereof are at least partially placed in one or more of the openings.

The collection region of a cartridge may receive a fluid and/or sample. In some embodiments, a collection region may include a cover.

Detection systems may include membrane-based detection systems and/or particle-based detection systems. The detection systems are configured to interact with at least a portion of a sample to allow detection of an analyte.

In some embodiments, a membrane of a membrane-based detection system, when one or more samples are applied to the membrane, at least partially retains desired matter in or on the membrane. In some embodiments, one or more viewing windows are optically coupled to the membrane, the viewing window being configured to allow one or more detectors to view at least a portion of the membrane.

In some embodiments, an anti-reflective material is coupled to the membrane. In some embodiments, the anti-reflective material is configured to inhibit reflection of light applied to the sample on the membrane, such that an image of at least a portion of the sample in or on the membrane is improved with respect to an image taken of the sample in the absence of the anti-reflective material.

One or more fluid delivery systems are configured to transport fluid from a first location to a second location in or on the cartridge. In some embodiments, a fluid delivery system includes one or more fluid packages and/or one or more syringes configured to facilitate transport of fluid. In some embodiments, at least one fluid delivery package is configured to create a partial vacuum, when opened, in one or more of the channels during use.

Fluid may be transported through one or more channels of the cartridge from a first location to a second location in or on the cartridge. Channels may couple one or more collection regions, one or more detection regions, and one or more fluid delivery systems to each other. In some embodiments, one or more channels are part of a fluid delivery system. In some embodiments, a shape or elevation of at least a portion of one or more of the channels is configured such that fluids flowing in or through one or more channels during use are selectively directed through the one or more channels. In some embodiments, an inside material of or on at least a portion of one or more of the channels is configured to selectively direct fluids flowing in or through one or more of the channels during use.

Valves positioned in or on one or more of the channels and/or a cartridge may control fluid flow. In some embodiments, one or more pinch valves are coupled to one or more of the channels and/or the cartridge. In some embodiments, applying pressure to one or more pinch valves positioned in or on the cartridge controls fluid flow through one or more of the channels.

One or more vents may be coupled to one or more of the channels. In some embodiments, gas is released from the cartridge through vents as fluids flow through one or more of the channels.

One or more reagent regions may include a reagent pad, at least a portion of a channel, and at least a portion of a surface of a cartridge. At least one of the reagent regions may deliver one or more reagents from the reagent region to a fluid flowing through one or more of the reagent regions during use. In some embodiments, flowing fluid through one or more reagent regions allows at least one reagent from at least one of the reagent regions to be delivered to a sample.

In some embodiments, one or more reservoirs include an overflow reservoir, a waste reservoir, or a both an overflow reservoir and a waste reservoir. The overflow reservoir and/or waste reservoir may collect excess sample or fluid. In some embodiments, a portion of fluids or samples in a cartridge is directed to an overflow reservoir of the cartridge.

In some embodiments, an analyte-detection system includes one or more cartridge-control systems. The cartridge-control systems include one or more control analytes. The cartridge-control systems may be coupled to one or more of the detection systems. One or more of the detection systems are configured to interact with at least a portion of the control analytes to allow detection of the control analyte.

A method of detecting analytes in a sample may include applying a sample on or to a collection region of a cartridge. In some embodiments, a cover is positioned over the collection region.

In some embodiments, a sample flows from a collection region to one or more detections systems, and one or more images of at least a portion the detection system are provided. In some embodiments, fluid flows through channels to and from reagent regions with the assistance of one or more fluid delivery systems. Fluids from reagent regions may flow in and/or through one or more detection systems.

A method for detection of an analyte in a sample may include applying at least a portion of a sample to a detection system of a cartridge and interacting at least a portion of the sample with the detection system to allow detection of the analyte.

A method of detecting analytes in a fluid includes applying one or more control analytes from one or more control analyte reservoirs in or on an analyte-detection cartridge to one or more detection systems in or on the analyte-detection cartridge and assessing a result from the detection system to determine whether the analyte-detection cartridge is working within a selected range.

A method for detecting and/or analyzing cells such as lymphocytes, epithelial cells, precancer cell, or cancer cell in a sample includes applying a sample to one or more membranes in or on a cartridge and applying one or more visualization agents from one or more visualization agent locations in or on a cartridge to a least a portion of the lymphocytes retained in or on the one or more membranes.

A method for assessing precancer, cancer, epithelial, or CD4$^+$ cells in a sample includes: applying a sample to a membrane in or on a cartridge; applying a first visualization agent to material retained on a membrane to stain any precancer, cancer, epithelial, or CD4$^+$ cells; applying one or more additional visualization agents to the material retained on the membrane to stain any target cell, such as a precancer cells, cancer cells, epithelial cells, T-cells, NK-cells, and B-cells retained on the membrane; providing a first image of the precancer, cancer, epithelial, or CD4$^+$ cells; providing a second image of the retained material; and assessing a number of precancer, cancer, epithelial, or CD4$^+$ cells by assessing the number of stained cells in the first image that are also depicted as stained cells in the second image. In some embodiments, a ratio of precancer, cancer, epithelial, or CD4$^+$ cells is assessed by comparing the number of stained cells that are depicted in both the first image and the second image, to the number of stained cells that are depicted in the second image.

A method of assessing precancer, cancer, epithelial, or CD4$^+$ cells in a sample includes: applying a fluid sample to a membrane; providing a first image of material of the sample retained on the membrane; applying one or more visualization agents to the material retained on the membrane to stain at least a portion of the material retained on the membrane that does not include precancer, cancer, epithelial, or CD4$^+$ cells; providing a second image of material retained on the membrane; assessing a number of precancer, cancer, epithelial, or CD4$^+$ cells by assessing the number of cells that are depicted in the first image but are not depicted in the second image.

A method of analyzing a body fluid, such as saliva or blood sample includes introducing the sample into an analyte-detection system, assessing a number of at least a portion of the cellular components collected by a membrane, and assessing an amount and/or identity of proteins that interact with the particle-based detection system.

An apparatus for analyzing a body fluid sample such as saliva or blood sample includes a membrane-based detection system and a particle-based detection system. The membrane-based detection system includes a membrane. The membrane collects at least a portion of a first analyte in the sample as the sample passes through the membrane during use. The particle-based detection system includes one or more particles. At least a portion of the particles is configured to interact with a second analyte in the sample during use.

## DESCRIPTION OF THE DRAWINGS

Features and advantages of the methods and apparatus of the present invention will be more fully appreciated by reference to the following detailed description of presently preferred but nonetheless illustrative embodiments in accordance with the present invention when taken in conjunction with the accompanying drawings in which:

FIG. 1 depicts a perspective view of an embodiment of a cartridge.

FIG. 2 depicts an exploded view of an embodiment of a cartridge.

FIG. 3 depicts an embodiment of a cartridge with channels.

FIG. 4 depicts an embodiment of a cartridge with fluid delivery systems with fluid packages.

FIG. 5 depicts an alternate embodiment of a cartridge.

FIG. 6 depicts a cross-sectional view of a valve.

FIG. 7 depicts a top view of an actuation system coupled to a cartridge.

FIG. 8 depicts a cross-sectional side view of an embodiment of a fluid package.

FIG. 9 depicts a top view of an embodiment of the fluid package depicted in FIG. 8.

FIG. 10 depicts a cross-sectional side view of an embodiment of a fluid package positioned in a cartridge.

FIG. 11 depicts a cross-sectional side view of rupturing the fluid package depicted in FIG. 10.

FIG. 12 depicts a cross-sectional side view of an embodiment of a fluid package in a cartridge.

FIG. 13 depicts a perspective view of a fluid delivery system that includes a fluid package and a reservoir.

FIG. 14 depicts an exploded view of the fluid delivery system depicted in FIG. 13.

FIG. 15 depicts a perspective cut-away view of the fluid delivery system depicted in FIG. 13.

FIG. 16 depicts a cut-away perspective view of the bottom of the fluid delivery system depicted in FIG. 13.

FIG. 17 depicts a top view of a seal offset from a top layer opening of the fluid delivery system depicted in FIG. 13.

FIG. 18 depicts a perspective view of an alternate embodiment of a fluid delivery system.

FIG. 19 depicts an exploded view of the fluid delivery system depicted in FIG. 18.

FIG. 20 depicts an embodiment of a fluid package used in the fluid delivery system depicted in FIG. 18 and FIG. 19.

FIG. 21 depicts an exploded view of an alternate embodiment of a fluid delivery system.

FIGs. 22A and 22B depict embodiments of fluid packages.

FIG. 23 depicts an embodiment of a fluid bulb for fluid delivery.

FIG. 24 depicts an alternate embodiment of fluid bulb for fluid delivery.

FIGs. 25A-25H depict embodiments of syringes.

FIG. 26A - 26B depicts an embodiment of syringes coupled to a cartridge. FIG. 26B depicts a magnified view of a portion of the cartridge depicted in FIG. 26A.

FIG. 27 depicts an embodiment of a cartridge that includes more than one detection system.

FIG. 28 depicts a top view of an embodiment of a multi-functional cartridge.

FIG. 29 depicts an exploded view of the multi-functional cartridge depicted in FIG. 28.

FIG. 30 depicts an exploded view of a membrane-based detection system.

FIG. 31 depicts an exploded view of a membrane-based detection system with directed fluid flow.

FIG. 32 depicts a top view of a membrane support with a parallelogram shape.

FIG. 33 depicts a top view of a membrane support with a euclidian shape.

FIG. 34 depicts a cross-sectional view of an embodiment of an open area of a membrane support.

FIG. 35 depicts a cross-sectional view of an alternate embodiment of an open area of a membrane support.

FIG. 36 depicts a schematic diagram of a cartridge positioned in an optical platform with two light sources.

FIG. 37 depicts a schematic diagram of a cartridge positioned in an alternate optical platform with two light sources.

FIG. 38 depicts a schematic diagram of a cartridge positioned in an optical platform with a single light source.

FIGs. 39A and 39B depict schematic diagrams of a cartridge positioned in an optical platform that includes movable filters.

FIGs. 40A-40C depict representations of images of cells obtained using an analyte-detection system.

FIGs. 41A-41D depict representations of images of cells obtained using an analyte-detection system.

FIG. 42 further illustrates the method and apparatus as applied to the detection tumor biomarkers in oral squamous cell carcinoma.

FIG. 43 further illustrates the method and apparatus as applied to the detection tumor biomarkers in oral squamous cell carcinoma.

FIG. 44 further illustrates the method and apparatus as applied to the detection tumor biomarkers in oral squamous cell carcinoma.

FIG. 45 further illustrates the method and apparatus as applied to the detection tumor biomarkers in oral squamous cell carcinoma.

FIG. 46 further illustrates the method and apparatus as applied to the detection tumor biomarkers in oral squamous cell carcinoma.

FIG. 47 further illustrates the method and apparatus as applied to the detection tumor biomarkers in oral squamous cell carcinoma.

FIG. 48 further illustrates the method and apparatus as applied to the detection tumor biomarkers in oral squamous cell carcinoma.

FIG. 49 further illustrates the method and apparatus as applied to the detection tumor biomarkers in oral squamous cell carcinoma.

FIG. 50 further illustrates the method and apparatus as applied to the detection tumor biomarkers in oral squamous cell carcinoma.

FIG. 51 further illustrates the method and apparatus as applied to the detection tumor biomarkers in oral squamous cell carcinoma.

FIG. 52 further illustrates the method and apparatus as applied to the detection tumor biomarkers in oral squamous cell carcinoma.

FIGs. 53A-53C depict structure of membrane-based LOC sensor. (FIG. 53A) Schematic diagram of layered LOC device with embedded track-etch membrane designed for cellular capture, imaging and analysis. (FIG. 53B) Cross-section view shows continuous fluid flow path supporting cell capture and delivery of reagents for "on-membrane" assays. (FIG. 53C) SEM micrograph of a LOC sensor membrane containing captured cancer cells.

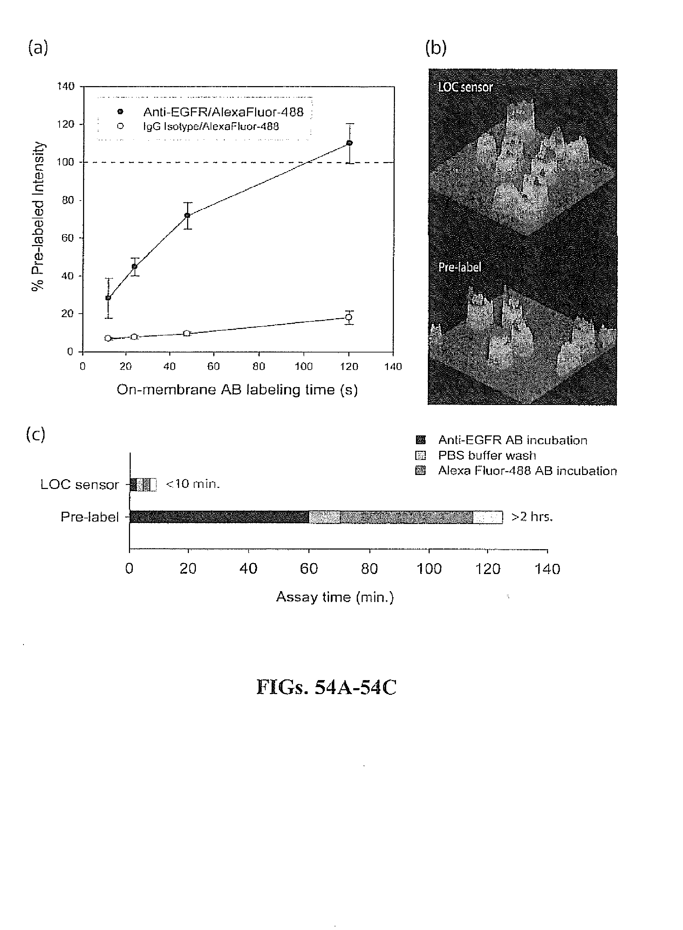

FIGs. 54A-54C depict the optimization of EGFR immunoassay in LOC sensor. (FIG. 54A) Line graph of EGFR fluorescent labelling intensity obtained using LOC sensor "on-membrane" staining of A253 cells at various antibody incubation times from 10-120 seconds. Results are expressed as a percentage of the EGFR intensity found when cells were pre-labelled according to a standard flow cytometry protocol. (FIG. 54B) A surface intensity plot of select cells from the LOC sensor assay at 120 seconds (top) and pre-labelled cells (bottom) shows relative EGFR intensity and membrane localization. (FIG. 54C) Bar graph comparing stepwise immunolabelling time in LOC sensor and pre-label protocol.

FIG. 55 depicts the comparison of immunolabelling homogeneity. Frequency distributions of bead standards labelled "on-membrane" in LOC sensor and pre-labelled in centrifuge tube. A rectangular gate according to object area and max pixel intensity (inset) eliminated debris and doublets from histogram analysis. Geometric mean and CV are reported for each population containing approximately 1000 events each.

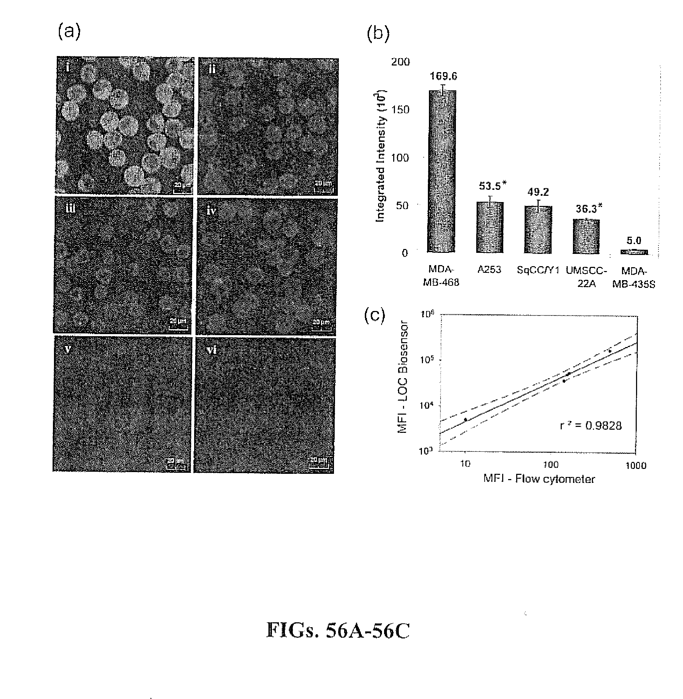

FIGs. 56A-56C depicts the detection of EGFR over-expression using LOC sensor. (FIG. 56A) Fluorescent micrographs of EGFR sensor immunoassays in cancer cell lines (i) MDA-MB-468; (ii) A253; (iii) SqCC/Y1; (iv) UMSCC-22A; (v) MDA-MB-435S; and (vi) isotype control. (FIG. 56B) Examination of EGFR expression using automated image analysis macros shows mean integrated intensity $\pm$ SD of triplicate EGFR assays with statistical differences found between all cell lines and MDA-MB-435S negative control ($p < 0.05$). Among the oral cancer cell lines, A253 and UMSCC-22A also exhibited statistical differences in EGFR expression ($p < 0.01$) denoted with "*". (FIG. 56C) Correlation of LOC expression analysis with standard flow cytometry shows a high degree of correlation ($r^2 = 0.9828$) at the 95% confidence interval.

FIG. 57 depicts an example of quantitative flow cytometry. Standard curve generated from flow cytometric analysis of QIFI bead standards for interpolation and quantification of EGFR receptors per cell in tumor-derived cell lines ranging from 0.2-8.0 x $10^5$ EGFR/cell.

## DETAILED DESCRIPTION OF THE INVENTION

In various embodiments, an analyte-detection system may be used to analyze a sample containing one or more analytes. Samples may be fluid samples, *e.g.*, a liquid sample or a gaseous sample. The analyte-detection system may, in some embodiments, generate patterns that are diagnostic for both the individual analytes and mixtures of the analytes. In some embodiments, the analyte-detection system includes a membrane capable of retaining a portion of the sample. The analyte-detection system, in certain embodiments, may include a plurality of chemically sensitive particles, formed in an ordered array, capable of simultaneously detecting different analytes. In some embodiments, the analyte-detection system may be formed using a microfabrication process, thus allowing the analyte-detection system to be economically manufactured.

Terms used herein are as follows:

"Analyte" refers one or more substances undergoing analysis. Examples of analytes include, but are not limited to, organic molecules, inorganic molecules, cells, bacteria, viruses, fungi, and parasites.

"Anti-reflective" refers to inhibiting the reflection of light at predetermined wavelengths.

"Cartridge" refers to a removable unit designed to be placed in a larger unit.

"Couple" refers to either a direct connection or an indirect connection (*e.g.*, one or more intervening connections) between one or more objects or components.

"CRP" refers to C-reactive protein.

"Detection system" refers to one or more systems designed to interact with one or more analytes during use.

"Detector" refers to one or more devices capable of detecting the presence of one or more analytes, one or more signals produced by one or more of the analytes, one or more signals produced by the interaction of one or more analytes with a detection system, or combinations thereof. Signals produced by analytes include, but are not limited to, spectroscopic signals. Spectroscopic signals include, but are not limited to, signals produced at wavelengths detectable in an ultraviolet ("UV") region, a visible region and an infrared ("IR") region of the electromagnetic spectrum. Spectroscopic signals also include signals produced by fluorescence of an analyte or a component of a detection system. The detector may be, but is not limited to an optical digital camera, a charge-coupled-device ("CCD"), a complementary-metal-oxide-semiconductor ("CMOS") detector, or a spectrophotometer capable of detecting UV, visible and/or IR wavelengths of light.

"Fluid" refers to a substance in a gas phase or a liquid phase.

"Fluid delivery system" refers to one or more systems or devices capable of causing a fluid to flow. A fluid delivery system may include a plurality of components. Components that may be part of a fluid delivery system include, but are not limited to, reservoirs containing fluids, flexible chambers containing fluids, channels, reagent reservoirs, buffer reservoirs, fluid packages, syringes, fluid bulbs, and/or pipettes.

"Fluid package" refers to a pouch, a container, or a chamber configured to contain one of more fluids.

"Fluorophore" refers to one or more fluorescent molecules or compounds.

"Hydrophilic material" refers to one or more materials having the ability to hydrogen bond with water. Hydrophilic materials may have an affinity for aqueous solutions.

"Hydrophobic material" refers to one or more materials ineffective at hydrogen bonding with water. Hydrophobic materials may lack an affinity for water.

"LED" refers to light emitting diode.

"Membrane" refers to one or more thin sheets or layers capable of retaining matter from a fluid and/or a sample.

"Positioned in" or "positioned on" refers to placing one or more substances at least partially or fully in or on an opening or a surface of a substrate.

"RBCs" refer to red blood cells.

To "stain" refers to applying one or more compounds to a substance to alter the absorbance and/or fluorescence of the substance.

"Visualization agent" refers to one or more compounds capable of altering an appearance of a material. Visualization agents may, in some embodiments, stain a material.

"WBCs" refer to white blood cells.

Analytes in a sample may be analyzed using an analyte-detection system. In some embodiments, a sample is a bodily fluid (*e.g.*, saliva, urine, and/or blood). A blood sample may be obtained from any species. A blood sample may be human blood or mammalian blood. Collection of a sample may be accomplished by making an incision (*e.g.,* a prick or cut) in a part of (*e.g.,* a finger) a human body to allow collection of the sample (*e.g.*, blood).

The sample may be collected with a tube, a fluid bulb, a syringe, or a pipette. The sample may be directly transferred to a cartridge of the analyte-detection system (*e.g.*, transfer to a collection region of the cartridge) using the fluid bulb, the syringe, or the pipette. For example, a sample is collected in a tube or a vacuum tube and transferred to a collection region of the cartridge. In some embodiments, a cartridge may include a conduit coupled to a disposable tip. The disposable tip may puncture a portion of a human body and draw a sample into the cartridge. In some embodiments, a sample is reacted with one or more reagents and/or one or more visualization agents in a sample collection device prior to being transferred to the cartridge.

The sample may be diluted before it is applied to a cartridge or after it is applied to the cartridge. For example, a sample of human blood may be diluted before applying it to a collection region of a cartridge. The use of a sample collection device may limit health and safety risks associated with exposure to pathogens present in a sample. Using a sample collection device, may allow a sample to be directly transported from the source to the instrument without further handling.

Sample collection devices are described by McDevitt et al., in U.S. Patent Application Nos. 11/022,176 entitled "INTEGRATION OF FLUIDS AND REAGENTS INTO SELF-CONTAINED CARTRIDGES CONTAINING SENSOR ELEMENTS"; 11/020,443 entitled "INTEGRATION OF FLUIDS AND REAGENTS INTO SELF-CONTAINED CARTRIDGES CONTAINING SENSOR ELEMENTS"; 11/020,442 entitled "INTEGRATION OF FLUIDS AND REAGENTS INTO SELF-CONTAINED CARTRIDGES CONTAINING SENSOR ELEMENTS"; 11/022,365 "INTEGRATION OF FLUIDS AND REAGENTS INTO SELF-CONTAINED CARTRIDGES CONTAINING SENSOR ELEMENTS"; 11/021,123 entitled "PARTICLE ON MEMBRANE ASSAY SYSTEM"; and 11/022,219 entitled "MEMBRANE ASSAY SYSTEM INCLUDING PRELOADED PARTICLES", all of which were filed on December 22, 2004.

The analyte-detection system may include, but is not limited to, one or more apparatuses (*e.g.*, cartridges), an optical platform, one or more detectors, an analyzer, or combinations thereof. The cartridge may include, but is not limited to, one or more sample collection devices, one or more collection regions, one or more fluid delivery systems, one or more reagent regions, one or more detection regions, or combinations thereof. The detection regions may include one or more detection systems. The optical platform may include, but is not limited to, one or more detectors, one or more light sources, one or more lenses, one or more filters, one or more dichroic mirrors, one or more shutters, one or more actuators, or combinations thereof. The analyzer may include one or more computer systems and/or one or more microscopes. In some embodiments, the analyte-detection system includes a housing. The housing may include the optical platform and/or one or more cartridges.

In some embodiments, a cartridge is self-contained and/or disposable. The cartridge may include all reagents and/or fluids necessary for the detection of one or more analytes in a sample. Use of a self-contained and/or disposable cartridge may limit environmental and health risks associated with handling of fluids and/or samples.

In some embodiments, one or more barcodes or other readable indicia are positioned on a cartridge. A detector and/or an analyzer of the analyte-detection system may read the barcode to determine hardware and/or software specifications for the assay. Using barcodes or other readable indicia may allow a user to analyze a plurality of cartridges using the same analyte-detection system. When the cartridge is positioned in an analyte-detection system, a reader in the analyte-detection system may read the indicia on the cartridge and set the system specifications for the indicated test. A bar code or indicia may represent information such as, but not limited to, the type of analyte to be detected, light sources which should be used, process time, sample number or code, detector settings, or combinations thereof. System specifications include, but are not limited to: which light sources, filters, or lenses to use; detector settings; fluid delivery system activation order and/or times; actuator activation sequence; actuator positions; exposure times; sample incubation time; and/or which visualization agents are used in the cartridge.

A cartridge may include indicia that tell a user which direction to insert the cartridge into the analyte-detection system. For example, a body of a cartridge may include a notch, arrow and/or a barcode to indicate the proper placement of the cartridge.

In some embodiments, a cartridge includes a viability indicator (*e.g.*, a temperature indicator). A viability indicator may indicate if the cartridge has been exposed to conditions that could damage the cartridge and/or one or more chemical components of the cartridge. For example, a temperature-based indicator indicates if the cartridge has been exposed to temperatures that are above or below a temperature that would cause decomposition of one or more chemical components in the cartridge. An analyte-detection system may read the viability indicator to determine if the cartridge is viable prior to initiating any detection operations with the cartridge.

The cartridge may be formed of an inert and/or biodegradable material. The cartridge may be sized to allow the cartridge to be hand-held and/or portable. In some embodiments, a cartridge has dimensions, which allows the cartridge to be inserted into a housing of an analyte-detection system.

In some embodiments, a cartridge body is substantially planar. A width (w) of the cartridge may range from about 30 mm to about 100 mm, from about 40 mm to about 90 mm, from about 50 mm to about 80 mm, or from about 60 mm to about 70 mm. A length (1) of the cartridge may range from about 50 mm to about 300 mm, 60 mm to about 200 mm, 70 mm to about 150 mm, or from about 80 mm to about 100 mm. A height (h) of the cartridge may range from about 1 mm to about 30 mm, from about 5 mm to 20 mm, or from about 10 mm to 15 mm. In some embodiments, a cartridge is about 35 mm wide and 125 mm long, about 35 mm wide and about 75 mm long, or about 50 mm wide and about 75 mm long.

A cartridge body may include one or more openings designed to receive one or more components used to facilitate analyte detection. Components include, but are not limited to, a collection region (*e.g.*, a sample collection pad), a fluid delivery system (*e.g.*, a fluid package, a fluid bulb, a syringe, and/or a fluid reservoir), reservoirs, a membrane-based detection system, a particle-based detection system, or combinations thereof. Components may be positioned in one or more cartridge body openings. Adhesive may be used to secure the components to the cartridge body and/or within the openings formed in the cartridge body. Openings may be designed to receive a specific component. For example, an opening designed for a collection region may have a specific shape that is different than an opening designed for a fluid delivery system component. In some embodiments, openings for components have the same dimensions and/or shape. In some embodiments, a cartridge body includes channels coupling one or more of the openings in or on the cartridge together. The ability to customize the cartridge body may allow many different configurations of a cartridge to be produced.

In some embodiments, collection regions, fluid delivery systems, reagent regions, and/or detection systems may be coupled to the cartridge, directly attached to the cartridge, positioned in the cartridge, or positioned on the cartridge. Collection regions, reagent regions, fluid delivery systems, and/or detection systems may be incorporated in a cartridge body. Collection regions, reagent regions, fluid delivery systems, and detection systems may be at least partially contained in a cartridge body.

In some embodiments, components are at least partially positioned in different layers of a body of the cartridge. For example, the collection region may be positioned in a different layer of the cartridge than the detection system. In some embodiments, reservoirs (*e.g.*, sample collection reservoir, overflow reservoir, and/or waste reservoir) are positioned in the same layer or in more than one layer. For example, a waste reservoir is positioned in a different layer of the cartridge than the detection system and/or the collection region. Fluid delivery systems may be positioned in one or more of the same layers of the cartridge body. The cartridge body may include one or more layers that retain fluid in at least a portion of the cartridge. In some embodiments, a top layer includes an opening coupled to the sample collection region to allow application of the sample to the sample collection region, while retaining fluid in other portions of the cartridge.

In certain embodiments, a cartridge with one or more openings has a variety of configurations. For example, a cartridge includes a detection region and one or more openings. A collection region, one or more fluid delivery systems and/or one or more reservoirs may be positioned in the openings of the cartridge. Alternatively, a cartridge includes a sample collection region and one or more openings. A detection system and/or at least one fluid package may be positioned in the openings. In another example, a cartridge includes one or more fluid delivery systems and one or more openings. Components (*e.g.*, a sample collection region and/or detection system) may be inserted the openings.

The collection region of a cartridge may be coupled to, positioned in, or positioned on the cartridge. The collection region may collect sample from a sample collection device. In some embodiments, fluids other than sample are collected in the collection region.

The collection region may include a channel positioned at a predetermined height with respect to the region. When a sample is deposited in the collection region, any sample excess sample will flow through the channel into an overflow reservoir and/or waste reservoir of the cartridge. The height at which the channel is positioned with respect to the region will determine the amount of sample that is collected in the collection region. Inclusion of the channel may inhibit sample from spilling out of a collection region. Inhibiting a sample from overflowing from the collection region may lessen exposure to potentially hazardous material. In some embodiments, a collection region of a cartridge includes and/or is a sample collection reservoir and/or a collection pad.

One or more fluid delivery systems may be coupled to, positioned in, positioned on, or embedded in a cartridge. In some embodiments, fluid delivery systems containing appropriate reagents, buffers, and/or visualization agents are positioned in openings in the cartridge body. Some fluid delivery systems are described in U.S. Patent Nos. 5,096,660 to Lauks et al.*;* 5,837,199 to Dumschat; and 6,010,463 to Lauks et al. In some embodiments, gravity, elevation changes within the cartridge and/or channel, capillary forces, or combinations thereof, promotes and/or facilitates the transport of fluids in the cartridge. In certain embodiments, pumps and/or vacuums are coupled to the cartridge, in addition to fluid delivery systems, to assist fluid flow.

A cartridge may include one or more reagent regions. One or more reagent regions may be at least partially coupled to, positioned on, or positioned in the cartridge. In some embodiments, a reagent region includes one or more reagents, visualization agents, and/or buffers that are disposed on one or more reagent pads, one or more surfaces of a channel, one or more surfaces of a cartridge, or a combination of these locations.

The reagents, visualization agents, and/or buffers may be in solid, liquid, or gaseous state. In some embodiments, a reagent region includes one or more reagents, visualization agents, and/or buffers entrained in a dissolvable material. When a fluid contacts (*e.g.*, passes over) the dissolvable material, at least a portion of the reagents, visualization agents, and/or buffers entrained in the dissolvable material may be released. For example, dried reagents may be positioned in or on a dissolvable material. Fluid passing over the dissolvable material may at least partially dissolve the dissolvable material and partially reconstitute the dried reagents.

A reagent pad of a reagent region may be, but is not limited to, a filter, absorbent pad, or container. Reagents including, but not limited to, visualization agents, anti-coagulants, and/or particles may be positioned in the reagent pad and/or on a surface of the reagent pad such that fluid passing over and/or through the reagent pad may at least partially reconstitute the reagents contained in or on the pad. In some embodiments, a reagent pad performs as a filter to remove large particles from a fluid flowing through the reagent pad.

In certain embodiments, dried reagents, lyophilized reagents, and/or solid reagents are positioned in or coated on a surface of a reagent region (*e.g.*, surfaces of a channel or a cartridge). As fluid passes through the channel, reagents and/or visualization agents may be reconstituted. Dried, lyophilized, or solid reagents may be more stabile. Using reagents that are dried, lyophilized, or are in a solid state may increase the shelf life of a cartridge. Using dried, lyophilized, or solid reagents may allow a cartridge to be stored at ambient temperatures rather than in a controlled temperature storage unit (*e.g.*, a refrigerator).

In some embodiments, one or more reservoirs (*e.g.*, one or more overflow reservoirs and/or one or more waste reservoirs) are coupled to, positioned in, or positioned on a cartridge. The overflow reservoir and/or waste reservoir may collect excess fluid (*e.g.*, excess sample, excess visualization agent, and/or excess reagents).

The overflow reservoir is, in some embodiments, coupled to a collection region, a detection region, a detection system, and/or one or more reagent regions. The overflow reservoir may be coupled to the collection region to allow an excess amount of sample (*e.g.*, an amount of sample greater than a predetermined amount of sample) applied to the collection region to flow to the overflow reservoir. Coupling the overflow reservoir to the collection region may allow a predetermined amount of sample to be collected. Coupling the overflow reservoir to the collection region may inhibit overfilling the collection region. Inhibiting overfilling of the collection region may inhibit release of potentially hazardous material.

In some embodiments, the overflow reservoir is coupled to the detection region and/or detection system to inhibit excess fluid from entering the detection region and/or detection system. If excess fluid enters the detection region and/or detection system, it may disturb matter and/or particles retained in or on the detection region and/or detection system. Disturbance of retained matter and/or particles may cause the matter and/or the particles to leave the detection region and/or detection system. For example, if too much fluid flows onto a membrane positioned in or on a detection region and/or a detection system, matter retained on a surface of the membrane may be disturbed and a portion of the retained matter may flow into proximate channels or regions before analysis.

One or more detection regions of a cartridge include areas of the cartridge where one or more detection systems are located. Detection systems may be coupled to, positioned in, or positioned on, a cartridge. It should be understood, that various combinations of detection systems in, on, or coupled to the cartridge are possible. For example, one detection system may be positioned in an opening of the cartridge, while another detection system is positioned on the cartridge. A detection system may be coupled to the cartridge, while another detection system is positioned in the cartridge. Detection systems may include, but are not limited to, a membrane-based detection system and/or a particle-based detection system. A detection system is selected based on the analyte of interest. For example, a membrane-based detection system may be selected to assess cells or bacteria in a fluid and/or sample.

Detection systems and methods of using the detection systems are described herein and in U.S. Patent Application Nos. 11/020,442; 11/022,365; 11/021,123; and 11/022,219, and in the following U.S. Patents, U.S. Published Patent Applications, and Patent Applications to McDevitt et al.: U.S. Patent Nos. 6,908,770; 6,680,206; 6,602,702; 6,589,779; 6,649,403; and 6,713,298; U.S. Patent Application Publication Nos. 20020160363; 20040029259; 20030064422; 20030186228; 20040053322; 20050136548; 20050164320; 20050214863; U.S. Patent Application Nos. 09/616,731 entitled "METHOD AND APPARATUS FOR THE DELIVERY OF SAMPLES TO A CHEMICAL SENSOR ARRAY" filed July 14, 2000; 10/522,499 entitled "CAPTURE AND DETECTION OF MICROBES BY MEMBRANE METHODS" filed January 24, 2005; 10/470,646 entitled "CAPTURE AND DETECTION OF MICROBES BY MEMBRANE METHODS" filed January 24, 2005; 10/522,926 entitled "CAPTURE AND DETECTION OF MICROBES BY MEMBRANE METHODS" filed January 24, 2005; 10/544,864 entitled "MICROCHIP-BASED SYSTEM FOR HIV DIAGNOSTICS" filed August 5, 2005; and 10/544,954 entitled "MULTI-SHELL MICROSPHERES WITH INTEGRATED CHROMATOGRAPHIC AND DETECTION LAYERS FOR USE IN ARRAY SENSORS" filed on August 8, 2005.

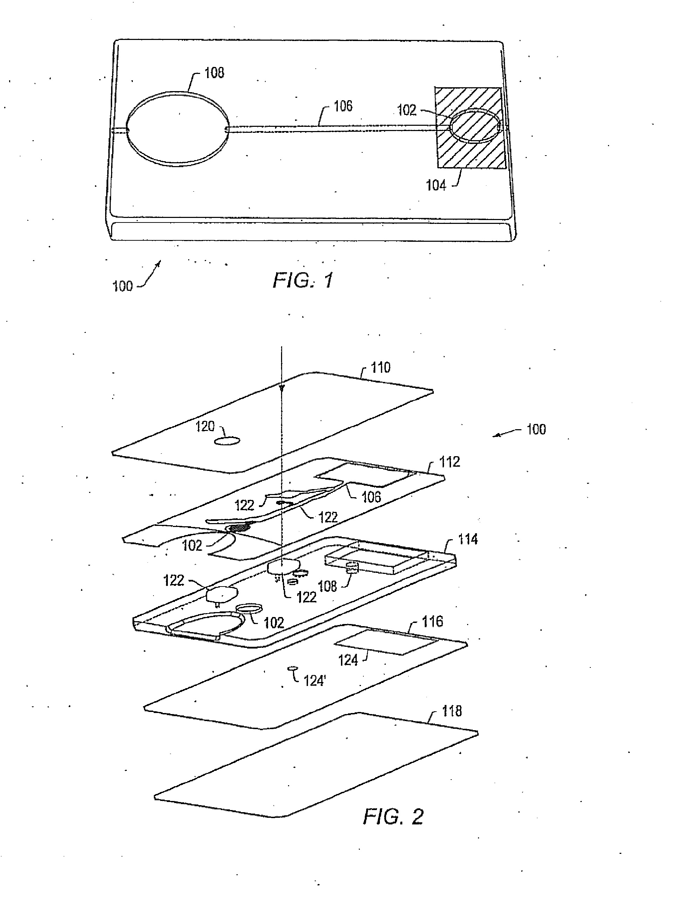

FIG. 1 depicts a perspective top view of an embodiment of a cartridge. Cartridge 100 includes collection region 102, cover 104, fluid channel 106, and detection region 108. A sample may be placed in collection region 102. In some embodiments, other fluids (e.g., reagents and/or buffer solutions) may be added to the collection region and mixed with the sample. The sample may flow from collection region 102 through channel 106 to detection region 108.

Collection region 102 may include, but is not limited to, a reservoir, a pad, a channel, a capillary, a tube, a vacuum collection tube (*e.g.*, a Vacutainer® commercially available from Becton, Dickinson Company Franklin Parks, New Jersey, USA), an opening in the cartridge, or combinations thereof. In some embodiments, collection region 102 is a portion of the detection system on which sample is applied. In certain embodiments, collection region 102 is a membrane.

In some embodiments, cover 104 is removable. Cover 104 may cover a portion or all of collection region 102. The use of cover 104 is optional. Cover 104 may be positioned manually or automatically. In some embodiments, an analyte-detection system automatically positions the cover over the collection region after the cartridge is positioned in the system. Cover 104 may be a flap coupled to the cartridge that may be moved to uncover or cover the collection region, as desired. Cover 104 may be moved in a sliding motion to cover or uncover the sample collection region. Cover 104 may seal the sample collection region and inhibit contaminants from entering the sample collection region. In some embodiments, the cover may include an opening. Cover 104 may at least partially contain biological waste and/or hazardous materials in the cartridge. In some embodiments, the cover may substantially contain biological waste and/or hazardous materials in the cartridge. In some embodiments, the cover may include an adhesive strip, an absorbent pad, a non-removable plug, a swinging window, a film, a nylon filter or combinations thereof.

In some embodiments, it may be desirable to inhibit sample from flowing towards a detection region. For example, after a predetermined amount of sample flows towards the detection region, it may be desirable to inhibit more of the sample from flowing towards the detection region. Cover 104 may inhibit undesired additional sample from flowing towards a detection region by absorbing sample from the collection region.

In some embodiments, a cartridge and/or a body of the cartridge are formed of one or more layers. In certain embodiments, one or more layers seal one or more components in the cartridge. Layers may be coupled, sealed, and/or bonded together to form the cartridge. The cartridge body may include more than three layers or more than four layers coupled together.

FIG. 2 depicts an exploded view of an embodiment of a cartridge formed of layers. Cartridge 100 may include top layer 110, channel layer, 112, sample layer 114, reservoir layer 116, and support layer 118.

Top layer 110 may include opening 120. Samples may be deposited on sample layer 114 through opening 120. Top layer 110 and support layer 118 may seal cartridge 100. In some embodiments, each of the layers may include more than one layer coupled together.

In some embodiments, sample layer 114 may be positioned between one or more channel layers 112 and reservoir layer 116. Sample layer 114 may include collection region 102 and/or one or more reagent regions 122. Collection region 102, one or more fluid channels 106, and/or reagent regions 122 may be at least partially contained in more than one layer of a body of cartridge 100.

Reservoir layer 116 may be positioned proximate sample layer 114. Reservoir layer 116 may collect sample and/or one or more fluids passing through the cartridge during use. Reservoir layer 116 may include one or more reservoirs 124, 124' that collect sample and/or fluid passing through the cartridge (e.g., an overflow reservoir and/or a waste reservoir). In some embodiments, reservoirs may extend through more than one layer. For example, reservoir 124 may extend through channel layer 112 and sample layer 114.

Channel layer 112 may be positioned above sample layer 114. In some embodiments, an additional channel layer may be positioned below a reservoir layer. In certain embodiments, one or more channel layers may be positioned above or below one or more sample layers and/or one or more reservoir layers. Channel layer 112 may include a plurality of channels coupling various components of cartridge 100. One or more channels 106 may allow fluid to flow within a layer and/or from one layer to another layer.

In some embodiments, channels are positioned in more than one layer of a cartridge. Positioning a channel in more than one layer may change an elevation of the channel enough to enhance sample and/or fluid to flow in and/or through the cartridge. Channels may be coupled to two or more locations in or on a cartridge. In some embodiments, one or more channels are a part of one or more fluid delivery systems.

In some embodiments, one or more channels couple a collection region to a detection region, one or more detection systems, and/or one or more overflow reservoirs. Channels may couple one or more fluid delivery systems to a collection region, a detection region, one or more detection systems, and/or one or more reservoirs (*e.g.*, overflow reservoirs and/or one or more waste reservoirs). Two or more channels may be coupled such that they intersect and fluid may optionally flow through more than one channel; however, the size, the elevation, and/or the inside material of the intersecting channel may affect which channel a fluid may flow through and/or may selectively direct fluid flow. Channels or a portion of a channel may promote and/or inhibit fluid flow in or on the cartridge.

The size and/or the elevation of a channel may selectively direct fluid flow through the channel. Fluid may flow preferentially through a channel that is wider before flowing through narrower channels, thus the fluid may be inhibited from flowing in channels narrower than other proximate channels. In some embodiments, a portion of the fluid may flow into a narrower channel, while another portion of the fluid flows into a channel wider than the narrow channel. In some embodiments, some channels may have a cross-sectional area larger than a cross-sectional area of other channels of a cartridge. Fluid may flow through the channel with the largest cross-sectional areas prior to flowing through channels with smaller cross-sectional areas. Fluid may be inhibited from flowing into a channel, when the channel has a smaller cross-sectional area than proximate channels.

In some embodiments, channels include changes in elevation. A portion of a channel may be positioned in a first layer of a cartridge while another portion may be positioned in a second and/or third layer of a cartridge. A channel may have an elevation gradient along an axis parallel to fluid flow. Changes in elevation of a channel may promote, facilitate, and/or increase fluid flow in or on a channel. Elevation changes may inhibit fluid from flowing into a channel.

In some embodiments channel properties may affect fluid flow in the channels. At least a portion of a channel may selectively direct fluid flow in one or more channels. A channel may be formed of a material, coated with a material or have material deposited on a surface of a portion of the channel that selectively directs fluid flow in one or more channels. For example, a channel may be at least partially formed of a hydrophilic material to promote aqueous fluid flow in the channel. A channel may be at least partially formed of a hydrophobic material to inhibit aqueous fluid flow in the channel. In some embodiments, portions of a channel may be coated with a hydrophilic and/or hydrophobic material. A material that defines at least a part of the channel may be hydrophilic. A channel coupled to a collection region may be partially made of a hydrophilic material to allow an aqueous sample to be drawn from the collection region. In some embodiments, channels partially made of a hydrophobic material may inhibit aqueous fluid flow, thus a waste region may not be needed.

Channels may be formed of or coated with a hydrophilic material and/or the elevation of the channel may promote fluid flow towards the detection region. In some embodiments, a channel releasing fluid into the detection regions and/or a detection system is at least partially formed of a hydrophilic material to promote laminar flow in the channel. Laminar flow of fluid in the channel may cause matter (*e.g.*, particles, cells, or other matter) in the sample to be evenly distributed across a surface of a portion of a detection system (*e.g.*, a membrane of a membrane-based detection system).

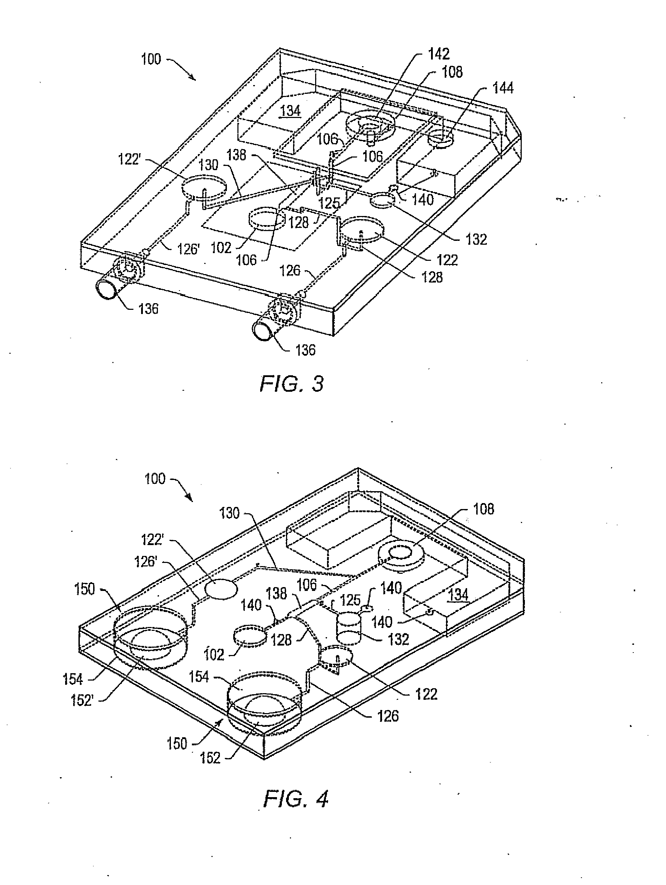

FIG. 3 depicts an embodiment of a cartridge that includes channels having different elevations. Cartridge 100 may include channels 106, 125, 126, 126', 128, 130, collection region 102, reagent regions 122, 122', detection region 108, overflow reservoir 132, waste reservoir 134, and connectors 136.

Sample deposited in collection region 102 may flow through channel 106 toward detection region 108. Channel 106 includes metered volume portion 138. Metered volume portion 138 may be a part of the channel. In some embodiments, the metered volume portion is coupled to the channel and/or the collection region. Metered volume portion 138 may have a diameter greater than diameters of proximate channels. If metered volume portion 138 reaches a predetermined amount of fluid (*e.g.* sample), fluid may flow towards overflow reservoir 132 through channel 125. In some embodiments, substantially all of an introduced sample flows out of collection region 102, into metered volume portion 138. Excess introduced sample will enter overflow reservoir 132 if the metered volume portion is filled. In some embodiments, overflow region 132 is coupled a waste region. Overflow reservoir 132 includes vent 140 to promote fluid flow.

Vents 140 may be positioned proximate one or more collection regions, metered volume portions, waste reservoirs, overflow reservoirs, and/or in channels coupled to fluid delivery systems. Vents 140 may allow gas to escape from cartridge 100 as fluids pass through or on one or more channels or layers of the cartridge. Vents 140 may inhibit pressure in the channels of the cartridge from becoming greater than ambient pressure. Vents 140 may promote fluid flow in cartridge 100 by releasing pressure associated with the passage of pressurized fluids through the channels. Vents 140 may facilitate laminar flow of fluids in cartridge 100. In some embodiments, vents 140 are designed to inhibit release of fluids through the vent. It may be desirable to limit release of liquids while allowing gas to escape from the cartridge to contain fluids (hazardous reagents and/or biological samples) in the cartridge.

Channel 106 has different elevations. Different elevations in the channel may inhibit fluid from flowing into detection region 108. It may be desirable to require a sample to be pushed towards a detection system rather than allowing a sample to flow towards a detection system without applied pressure for many reasons. For example, it may be desirable to allow the sample to mix and interact with reagents prior to entering the detection region. Channel 106 may promote fluid flow towards the overflow region. In certain embodiments, channel 106 may have a negative pressure so that fluids are drawn into the channel. In some embodiments, a channel coupled to a collection region may have a negative pressure to draw the sample into the channel.

Fluid may be delivered to cartridge 100 from one or more fluid delivery systems connected to the cartridge by connectors 136. Connectors may include, but are not limited to, tubing, quick-disconnect connections, and/or locking connectors. It should be understood that any of the various embodiments of fluid delivery systems described herein and/or other fluid delivery systems known in the art may be incorporated with or coupled to cartridge 100.

Fluid enters channel 126, 126' and passes through and/or over reagent regions 122, 122'. In some embodiments, the reagent region may be a pad, a channel, a depression and/or a reservoir. In some embodiments, the reagent regions may be a part of the fluid delivery system. In some embodiments, the reagent regions are channels, which are a part of a fluid delivery system. Reagent regions 122, 122' may include dried reagents, anti-coagulants, and/or visualization agents. In some embodiments, reagents, buffers and/or visualization agents are dried on or in a pad positioned in or on reagent regions 122, 122'. In some embodiments, reagents and/or visualization agents on and/or in the reagent regions 122, 122' may be reconstituted by fluid passing over and/or the through reagent region.

Channels 128, 130 may allow fluid to flow from the bottom surface of reagent regions 122, 122' to other components of cartridge 100. In some embodiments, inlet and outlet channels to the reagent regions may be positioned such that fluid is forced to pass through, on, and/or over reagent regions 122, 122'. In some embodiments, additional fluid delivery systems are positioned proximate the reagent regions.

The fluid delivery system may be controlled to allow fluid to pass across the reagent region 122, enter metered volume portion 138, and then enter detection region 108. Reagents and/or visualization agents in reagent region 122 may be reconstituted by the fluid from the fluid delivery system and may react with the sample. The fluid delivery system may be controlled to allow a predetermined volume of fluid to pass through detection region 108. In some embodiments, fluid from a fluid delivery system may pass over a detection system of the cartridge while the sample incubates on the detection system and/or a membrane of the detection system.

Channels 128, 130 intersect channel 106, and fluid and/or sample from these channels enters detection region 108 via channel 106. Detection region 108 may include viewing window 142. Viewing window 142 may be optically coupled to a detection system. Viewing window 142 may be positioned in or on the cartridge. Viewing window 142 may be a portion of a detection system. For example, viewing window 142 may be a portion of a top member of a membrane-based detection system located in the detection region. Viewing window 142 may be made of a material transparent to visible or ultraviolet light. Viewing window 142 may include or be composed of a material that acts as a filter that only allows certain wavelengths of light to pass. Viewing window 142 may include a lens that assists in focusing light onto a portion of a detection system and/or onto one or more detectors. A detector may capture an image or light from a detection system through viewing window 142.

Detection region 108 and/or a detection system in the detection region may be coupled to waste reservoir 134 to allow fluids flowing through the detection system to pass into the waste region. Waste reservoir 134 may be, but is not limited to, a container, a depression, or an opening. Waste reservoir 134 may be coupled to, positioned in, or positioned on the cartridge. By allowing fluids to flow towards a waste reservoir after use, all fluids in the cartridge may be contained within the cartridge. A contained waste reservoir may minimize health and safety hazards due to handling of and/or exposure to the sample and/or fluid.

Waste reservoir 134 may include cap 144. Cap 144 allows a user to remove fluids from the waste region and/or release pressure from the waste region. All or a portion of cap 144 may be removable. Cap 144 may have a variety of shapes and/or configurations (*e.g.*, round, oval, threaded and/or tapered). A cap on a waste reservoir may allow the waste reservoir to be pressurized so that fluids may be drawn towards the detection system and/or waste reservoir. A waste reservoir may include vent 140 that may inhibit a build up of pressure in the waste reservoir.

In some embodiments, a fluid delivery system facilitates transport of fluid or sample from one location to another location in or on the cartridge (*e.g.*, from a first location in or on the cartridge to a second and/or third location in or on the cartridge). In certain embodiments, a fluid delivery system delivers reagents, buffer, and/or visualization agents to the detection system. The fluid delivery system may facilitate transport of at least a portion of the sample from the sample collection region to the detection system. The fluid delivery system may couple and/or include channels that couple different regions of the cartridge. For example, the fluid delivery system couples the collection region to the detection system. The fluid delivery system may couple the collection region to the detection system and/or to one or more waste reservoirs. In some embodiments, the fluid delivery system includes channels that couple components of the analyte-detection system to each other.

FIG. 4 depicts an embodiment of cartridge 100 with two fluid delivery systems. Cartridge 100 may include channels 106, 125, 126, 126', 128, 130, collection region 102, reagent regions 122, 122', detection region 108, overflow reservoir 132, waste reservoir 134, fluid delivery systems 150, and vents 140. Fluid delivery systems 150 include fluid packages 152, 152' and reservoirs 154. During use, sample may be released from collection region 102, flow through channel 106 and enter detection region 108. Channel 106 may include metered volume portion 138.

Fluid packages 152, 152' may be opened at predetermined times (*e.g.*, simultaneously or one at a time) to allow fluid (*e.g.*, a buffer, reagent solution or visualization agents) in the fluid package to be released into channel 126, 126'. The released fluids may pass over reagent regions 122, 122' before a portion of sample in channel 106 reaches detection system 108. For example, a portion of a sample is placed in collection region 102 and released into channel 106 after fluid from one of fluid packages 152 flows over and/or through reagent region 122. Alternatively, a portion of sample is placed in collection region 102 and released into channel 106 before and/or simultaneously as fluid from one of fluid packages 152' flows over and/or through reagent region 122'. In some embodiments, substantially the entire excess introduced sample flows out of collection region 102 and into overflow reservoir 132 via channel 125. A size of overflow reservoir 132 may allow fluid from more than one assay to be collected during use.

Fluid from reagent region 122 flows through channel 128, enters into channel 106, and then enters detection region 108. In some embodiments, channel 128 and channel 106 are the same channel. Channel 126' delivers and/or directs fluid flow from fluid delivery system 150, across and/or through the reagent region 122', and into channel 130.

Channel 130, which intersects channel 106, directs fluid from reagent region 122' to a position in channel 106 such that the reagents from reagent region 122' mix with a portion of the sample and/or fluid in channel 106 prior to entering detection region 108. In some embodiments, channel 130 is a part of channel 106.

Vents 140 may be positioned in or on cartridge 100. Vents 140 may be a part of waste reservoir 134 or a part of one or more channels (*e.g.*, channel 106).

In some embodiments, valves are used to control fluid flow through the cartridge. Valves may be positioned on or in the cartridge. Valves may direct, control, and/or restrict fluid flow. Active or passive valves may be positioned in channels. Valves may include, but are not limited to, pinch valves, pressure valves, electromagnetic valves, and/or temperatures valves.

In some embodiments, a temperature-controlled valve may be used. A temperature-controlled valve may include a fluid, such as but not limited to, water that is at least partially frozen in a channel to prevent further fluid from passing through the channel. To open the valve, heat is applied to the frozen fluid to melt the fluid. A temperature-controlled valve includes, in some embodiments, a material that is a solid at room temperature (*e.g.*, paraffin or wax). To open a channel, heat may be applied to the solid in the channel to melt the solid material.

In certain embodiments, a valve is hydraulically activated. In some embodiments, pressurized fluid (*e.g.*, air or water) is used to open or close a valve. Pressure may be transferred via a gas or liquid in a channel to another location in the cartridge. The gas or liquid may be used to compress a drum and/or close a valve. In some embodiments, valves surrounding a portion of a channel having negative pressure inhibit equalizing the negative pressure until desired.

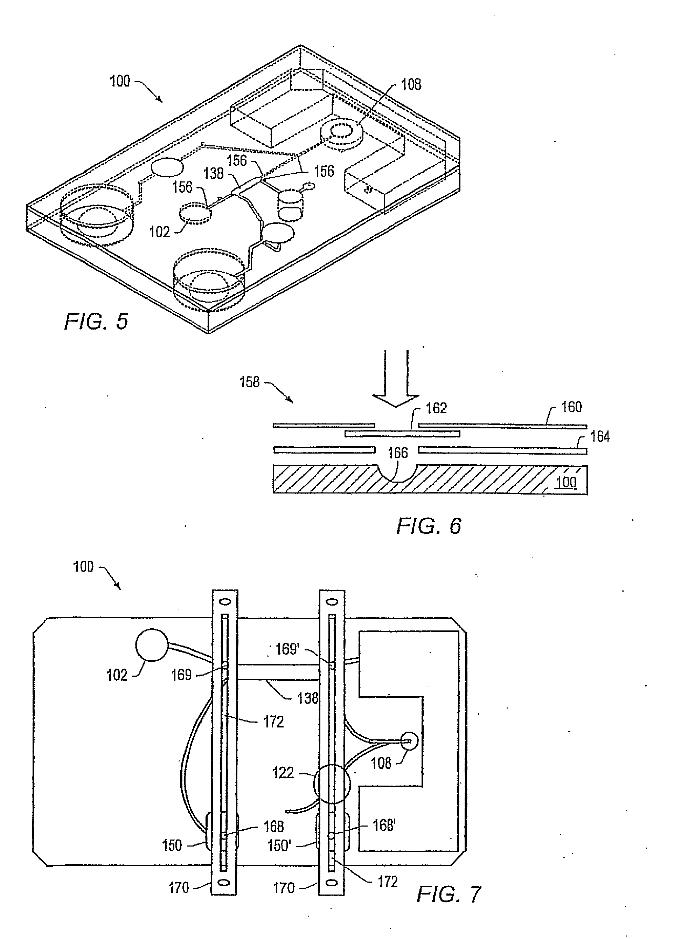

FIG. 5 depicts cartridge 100 depicted in FIG. 4 with valves 156. Valves 156 are positioned after collection region 102 and after metered volume portion 138. Valves 156 may be used to direct fluid flow from collection region 102 to detection region 108. Valves 156 may be positioned at various other locations in or on cartridge 100.

FIG. 6 depicts an embodiment of a pinch valve. Pinch valve 158 may include one or more layers 160, 162, 164 and channel 166. Layers 160, 162, 164 may be positioned over a surface of cartridge 100. In some embodiments, the layers are incorporated into the cartridge. Channel 166 may be an opening in cartridge 100.

Layer 162 may be coupled to layer 160 and layer 164. Surfaces of layers 160, 164 may be composed of materials including, but not limited to, thermal bond film, pressure sensitive adhesive, or other adhesive materials. Layer 162 may be adhered to layers 160, 164 (*e.g.*, using a heat sealing process). In some embodiments, layer 164 forms a wall of channel 166. Layer 162 may be designed so that pressure applied to a surface of layer 162 causes the layer to deform (*e.g.*, layer 162 flexes). Deformation of at least a portion of layer 162 may at least partially obstruct channel 166 as layer 162 is forced into channel 166 by the applied pressure. Layer 162 may be formed of any material that exhibits flexibility when pressure is applied to the layer (*e.g.*, formed of an elastomer material).

Valves may be activated manually or automatically. In some embodiments, an analyzer system automatically opens or closes the valves. Actuators may be coupled to the analyte-detection system to open and/or close the valves. In some embodiments, an actuator is positioned above the cartridge to apply pressure to a valve through an opening in the cartridge. In some embodiments, an actuator is positioned below the cartridge to apply pressure to a valve through an opening in the cartridge. In some embodiments, actuators are designed to open fluid delivery systems. In some embodiments, a metered volume of a sample including particulate components (*e.g.*, cellular components) may be defined within a cartridge by actuation of one or more valves (*e.g.*, pinch valves).

In some embodiments, actuation is used to release liquids or gas from a fluid delivery system. Liquids and/or gas may be pressurized into or in the fluid delivery system. An actuated fluid delivery system may be actuated from a top surface, a bottom surface, and/or a side surface of the cartridge. For example, a cartridge may be loaded in a housing of an analyte-detection system with actuators. Actuators are then automatically, semi-automatically, or manually aligned with actuation points of the cartridge. A cartridge positioning system may facilitate cartridge placement into a position such that actuation points are aligned with actuators. Actuation points may be positioned on top, bottom, and/or side surfaces of a cartridge. For example, when a cartridge is positioned in the housing of an analyte-detection system, actuators may be positioned below the cartridge.

FIG. 7 depicts a perspective top view of a cartridge 100 with an actuator system. The actuator system may include actuators 168, 168', 169, 169' and structure 170. Structure 170 may be designed to move from one side of a cartridge to another side a cartridge 100, along a surface of the cartridge, to facilitate actuation of various valves and/or fluid delivery systems. Structures 170 may be positioned at various points on cartridge 100. As shown, structures 170 are positioned between collection region 102 and detection region 108. Structures 170 may include openings 172. In some embodiments, opening 172 is a track. Actuators 168, 168',169, 169' may be positioned at various points on or in structure 170 or opening 172. Actuators 168, 168', 169, 169' may move along opening 172 in structure 170, as needed.

Actuators 168, 168' are positioned over fluid delivery systems 150, 150'. Actuation of fluid delivery system 150 by actuators 168 may force fluid to flow towards metered volume portion 138. Actuation of fluid delivery system 150' by actuator 168' may allow fluid to flow towards reagent region 122.

Actuators 169, 169' may be positioned over valves proximate metered volume portion 138. Actuation of one or more of the valves proximate meter volume portion 138 may allow a metered volume of sample to flow into and/ out of metered volume portion 138. For example, actuator 169 may open the valve between collection region 102 and metered volume portion 138 to allow a portion of a sample to flow into the metered volume portion. Actuator 169' may at least partially open the valve between metered volume portion 138 and detection region 108 to allow a portion of the sample to flow towards the detection region.

Structure 170 may then be moved to a different location, as desired. In some embodiments, sample in a channel may be inhibited from flowing back towards a collection region by actuating a valve. In some embodiments, one or more actuators may be moved along an opening or a track of the structure until the actuator aligns with a valve. The actuator may then actuate the valve.

In some embodiments, fluid delivery systems include one or more fluid packages. A fluid package is a package that contains a fluid used by a fluid delivery system. Fluid packages may include liquids or gas under pressure. Fluid packages contain a fluid until the package is opened. Upon opening of the package, fluid in the fluid package may be at least partially released. A fluid package may contain a fluid until an activation pressure is applied to the fluid package. An activation pressure may be the pressure required to release at least a portion of fluids from the fluid package. An activation pressure may be the pressure required to rupture the package of the fluid package. Upon application of an activation pressure to the fluid package, at least a portion of the fluid contained in the fluid package will be released. In some embodiments, a fluid package is activated (*e.g.*, opened) by heat or an electromagnetic signal.

In some embodiments, fluid packages contain liquids, such as one or more buffers (*e.g.*, phosphate buffers), one or more solvents (*e.g.*, water, methanol, ethanol, and/or THF), one or more reagents, and/or one or more visualization agents. Positioning one or more liquids required for analysis in or on a cartridge may make the fluids more accessible during use and enhance usage of the cartridge. Pre-packaged liquids may limit exposure to the liquids resulting from selection and/or mixing of solutions during use. Pre-packaged liquids may enhance time of analysis from sample collection to analysis of the sample. Placing the liquids required for analysis in fluid packages may increase stability and/or shelf life of a cartridge that includes an actuated fluid delivery system. Additionally, fluid packages may allow the cartridge to be stored at room temperature rather than requiring refrigeration.

In some embodiments, a fluid package includes a solvent. The solvent in a fluid package may be released from the fluid package and flow over one or more reagent pads that include buffer chemicals, reagents, and/or visualization agents. A cartridge including solvent filled fluid packages and dried buffers, reagents, and/or visualization agents may increase the stability of the cartridge since dried buffers, reagents, and visualization agents may be more stable and/or may have a greater shelf life than aqueous solutions.

In some embodiments, a fluid package delivers air or another gas to the cartridge. Gas released from a fluid package may assist in transporting a fluid and/or a sample through and/or in the components and/or channels of the cartridge.

In certain embodiments, a fluid package is designed to be filled with fluid with substantially few or no air bubbles. A fluid package may be designed to inhibit release of air bubbles or gas within a fluid package into a cartridge channel or component during partial or full compression and/or actuation of the fluid delivery system.

In some embodiments, a fluid package is designed to release at least 80 percent of liquid or gas contained in the fluid package. A fluid package may include about 1 mL to about 500 mL of fluid. In certain embodiments, a fluid package has a shelf life of at least 2 years and/or has a volume loss of less than 5 percent of the original volume during a 2-year period.

A fluid package may be, but is not limited to, a pouch, container, and/or chamber. The fluid package may be formed from plastic materials. Plastic material may allow the fluid package to deform and release fluid. Once the fluid is released the plastic fluid package does not attempt to reform, thus creation of at least a partial vacuum is inhibited. Creation of at least a partial vacuum may draw fluids and/or gas back into the fluid package.

In some embodiments, a fluid package may be deformable in a controlled manner. The fluid package may be formed of a material that allows the fluid package to be deformed and/or compressed (*e.g.*, elastomeric material). A deformable/compressible material may allow a fluid package to be transported, stored, and/or positioned without breakage.

A fluid package may be made of materials including, but not limited to, polyvinyl chloride (PVC), polyvinylidene chloride (PVDC), polyethylene (PE), rubber, polypropylene (PP), polyacrylonitrile (PAN), cyclic olefin copolymer (COC), fluoropolymer films, foil (*e.g.*, aluminum foil or plastic foil), adhesive tapes, or combinations thereof.

In some embodiments, a fluid package may be formed of a first material and a second material, where a second material is designed to rupture or break before the first material when pressure is applied to the fluid package. In some embodiments, a wall of the fluid package may be formed of layers of polypropylene and cyclic olefin copolymer.

A fluid package may be formed of a material compatible with the fluid it is designed to contain. A fluid package may be formed of a material that will not leach into the fluid contained within the fluid package. In certain embodiments, a fluid package includes a layer that couples the fluid package to the cartridge. The layer may be formed of a material capable of bonding (*e.g.*, adhesive material) to acrylic, plastics, and/or other materials used to form a cartridge body.

A wall of a fluid package may be designed to have a weak portion (*e.g.*, a burst point). The weak wall portion may rupture when a predetermined amount of pressure is applied to the fluid package. Fluid may be released from a fluid package when by applying sufficient pressure to the package to cause the weak wall portion to rupture. The location of the weakened wall portion may be aligned with or coupled to a channel and/or component opening. A fluid package may be designed with a burst point or point at which fluid is released of about 3 psi to about 7 psi.

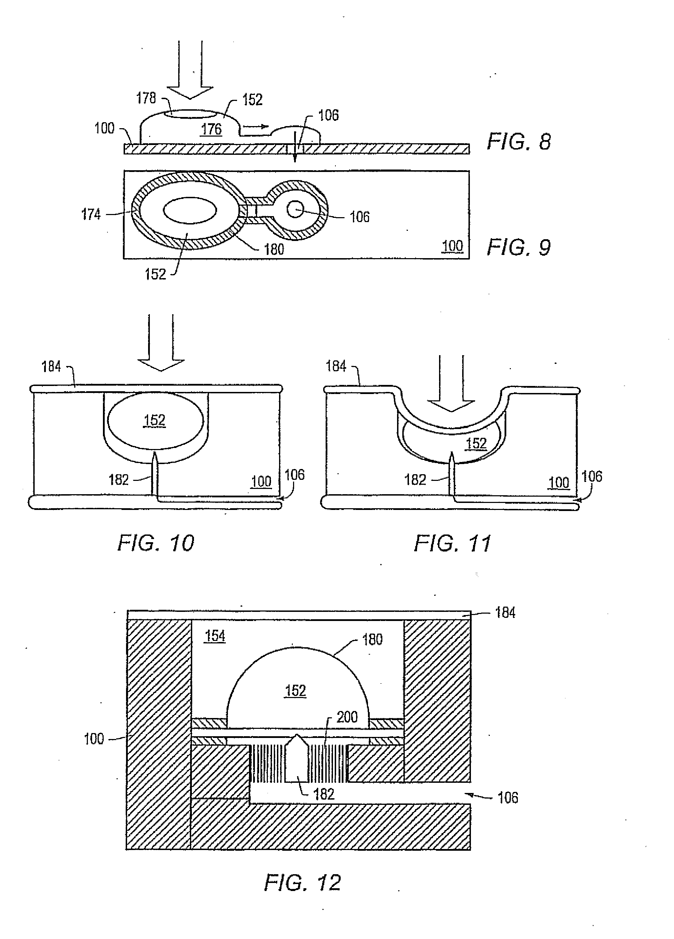

FIG. 8 depicts a side view of an embodiment of a fluid package. FIG. 9 depicts a top view of the embodiment of the fluid package depicted in FIG. 8. Fluid package 152 may be coupled to, at least partially positioned in, or at least partially positioned on cartridge 100. As depicted in FIG.9, fluid package 152 may include layer 174. Layer 174 may be made of material (*e.g.*, adhesive) that allows fluid package 152 to couple to cartridge 100. Fluid package 152 may be at least partially filled with liquid. Fluid package 152 may include liquid 176 and gas 178. Examples of gas 178 are air, nitrogen, and/or argon. A portion of a wall of fluid package 152 may include a burst point. As pressure is applied to the fluid package 152, wall 180 of fluid package 152 may rupture at the burst point. Once fluid package 152 ruptures, fluid may be released from the fluid package into channel 106. The rigidity of fluid package 152 may be modified to accommodate various applications and/or storage or transport conditions. In some embodiments, fluid and/or air may be contained in the fluid package by a removable adhesive strip. Removal of the adhesive strip may allow fluid and/or air from the fluid package to be released from the fluid package.

In some embodiments, a cartridge includes a projection to rupture a portion of the fluid package. The projection may be needle shaped or any other shape capable of perforating a fluid package. The projection may be formed from any suitable material such as metal, plastic, and/or silicon. FIG. 10 depicts a side view of an embodiment of a fluid package positioned in a cartridge with a projection. Projection 182 may be positioned proximate to a surface of fluid package 152 and/or cartridge 100. Cover 184 may be positioned over fluid package 152. FIG. 11 depicts an embodiment of rupturing the fluid package depicted in FIG. 10. When pressure is applied to cover 184, the cover contacts the fluid package 152 causing the fluid package to contact projection 182. Projection 182 may rupture a portion of fluid package 152 causing fluids to be released channel 106.

FIG. 12 depicts cross-sectional view of a fluid package positioned in cartridge 100. Fluid package is positioned in opening 154 of cartridge 100. In some embodiments, fluid package is positioned on the cartridge. In some embodiments, one or more walls of the opening are capable of being deformed (*e.g.*, the walls flex). Cover 184 may be positioned above opening 154. Cover 184 may be formed of an adhesive so that fluid package 152 is retained in opening 154. Projection 182 may be coupled to cartridge 100. Pressure applied to cover 184 may cause wall 180 of fluid package 152 to contact projection 182 and rupture. Fluid from fluid package 152 may be released into channel 106. Baffles 200 positioned proximate the bottom of opening 154 may assist in controlling flow rate of the fluid from fluid package 152.

In some embodiments, a fluid delivery system includes one or more fluid packages and a reservoir. The one or more fluid packages may be sealed and/or positioned in the reservoir. The reservoir may be coupled to, positioned in or positioned on the cartridge.

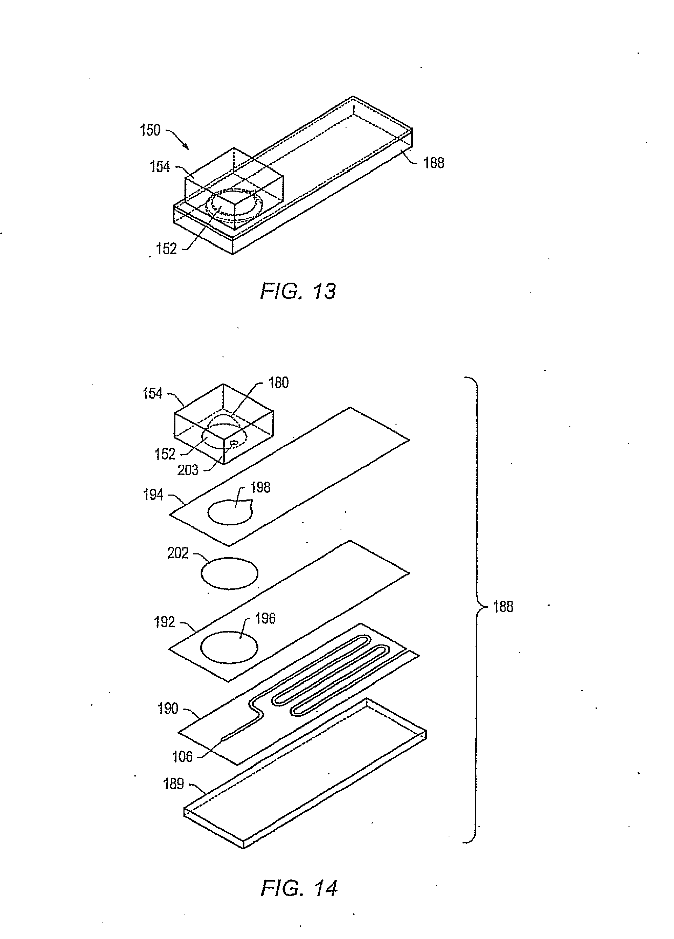

FIG. 13 is a perspective view of a fluid delivery system with a fluid package and a reservoir. Fluid delivery system 150 may include fluid package 152, reservoir 154, and support 188. In some embodiments, support 188 is part of a cartridge body. Portions of the fluid delivery system may be formed of several layers. In some embodiments, portions of the fluid deliver system may be formed of silicon resin, double-sided adhesive, thermo-bond film, and/or metal foil.

FIG. 14 depicts an exploded view of fluid delivery system 150 depicted in FIG. 13. Support 188 may include support layer 189, channel layer 190, middle layer 192, and top layer 194. Support layer 189 and/or middle layer 192 may assist in retaining fluids in channel layer 190. Support layer 189 may be a portion of a cartridge. Support layer may be formed of plastic and/or glass. Channel layer 190 may be coupled to, or be a part of, support layer 189. Channel 106 of channel layer 190 directs fluid flow to a collection region and/or a detection region of the cartridge. Channel layer 190 may include reagent regions and/or have properties described herein. In some embodiments, the layers of fluid delivery system 150 may be the same as the layers in cartridge 100.

Middle layer 192 may be coupled to or be a part of channel layer 190. Portions of middle layer 192 may include coupling agents (*e.g.*, adhesive or adhesive film) that couple the middle layer to channel layer 190. Middle layer 192 may include opening 196. Opening 196 may direct fluid into channel 106. Middle layer 192 may be coupled to top layer 194 using generally known coupling techniques (*e.g.*, adhesive, pins, and/or screws).

Top layer 194 may seal or contain fluids in fluid package 152 and/or reservoir 154. Top layer 194 may include opening 198. Opening 198 may direct fluid from fluid package 152 and/or reservoir 154 to channel layer 190. Top layer 194 may include seal 202. Seal 202 may be positioned between middle layer 192 and top layer 194. Seal 202 may cover opening 198 of top layer 194. Seal 202 may seal fluid and/or gas in fluid package 152 and/or reservoir 154. Seal 202 may be formed from a variety of materials (e.g., thermo-bond film, and/or foil). Seal 202 may rupture when pressure is applied to fluid package 152 and/or reservoir 154. In some embodiments, seal 202 may be a part of top layer 194.

Top layer 194 may be coupled to or be a part of reservoir 154 using generally known coupling techniques. Reservoir 154 may include opening 203. Reservoir opening 203 may be aligned with top layer opening 198. Top layer 194 may coupled to or be a part of reservoir 154 and/or fluid package wall 180.

Fluid package 152 may be positioned in reservoir 154. A wall of fluid package 152 may be aligned with reservoir opening 203 and top layer opening 196. A portion of a wall of fluid package 152 includes a burst point to allow the fluid package to rupture when a predetermined amount of pressure is applied to the fluid package and/or reservoir 154. In some embodiment, the fluid package and the reservoir are one unit. In some embodiments the reservoir does not include the fluid package.

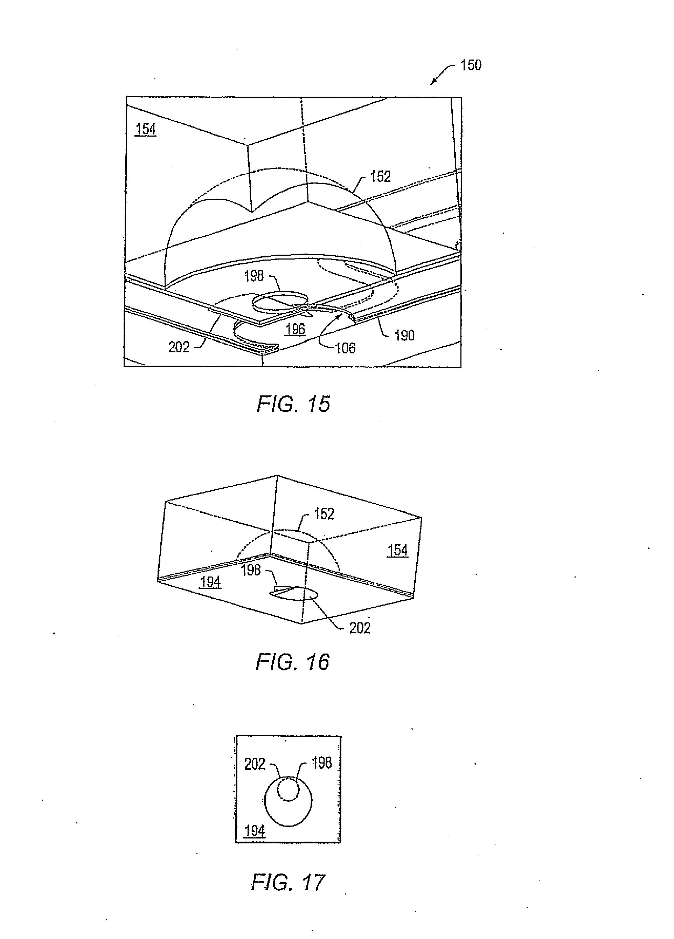

FIG. 15 depicts a perspective cut-away view of the reservoir of fluid delivery system 150 depicted in FIG. 13. A diameter of top layer opening 198 and/or the reservoir opening may be less than, equal to, or greater than a diameter of than middle layer opening 196. As depicted, seal 202 has been torn to allow fluid to flow to channel 106 in channel layer 190. A center of seal 202 may be directly aligned or offset with a center of top layer opening 198.

FIG. 16 depicts a cut-away perspective view of top layer 194 and reservoir 154 containing fluid package 152 as depicted in FIG. 13. FIG. 17 depicts a top view of fluid reservoir 154. As seen in FIG. 17, seal 202 is offset from top layer opening 198 in top layer 194. Offsetting seal 202 may facilitate the rupturing of the seal when a predetermined amount of pressure is applied to the fluid package and/or reservoir by creating a weak point in the seal.

A center of the seal may be offset from the center of the top layer opening by a distance ranging from about 0.2 mm to about 2 mm, about 0.3 mm to about 1.5 mm, or about 0.4 mm to about 1 mm. When the center of the seal is offset from the center of the top cover opening by about 0.25 mm, a burst point of the seal may rupture at a pressure of about 1 psi to at most 10 psi, from about 3 psi to about 8 psi, or from about 5 psi to about 7 psi. In contrast, the burst point of the seal may rupture at a pressure of greater than 10 psi when a center of the seal is aligned with the center of the top cover opening.

In some embodiments, the pressure required to rupture a fluid package is lowered by varying the materials used to create the seal, decreasing the surface area of the seal in a strategic location, decreasing the bonding temperature of the seal, and/or decreasing the time of heat sealing the seal to the top layer and/or the reservoir. Application of force to the reservoir and/or the fluid package may change the internal pressure in the reservoir and/or the fluid package enough to cause the seal to rupture or separate from the top layer. Rupturing or separating the seal from the top layer allows fluids in the reservoir to pass through the reservoir opening, the top layer opening, and/or the cover layer opening and into the channel layer.

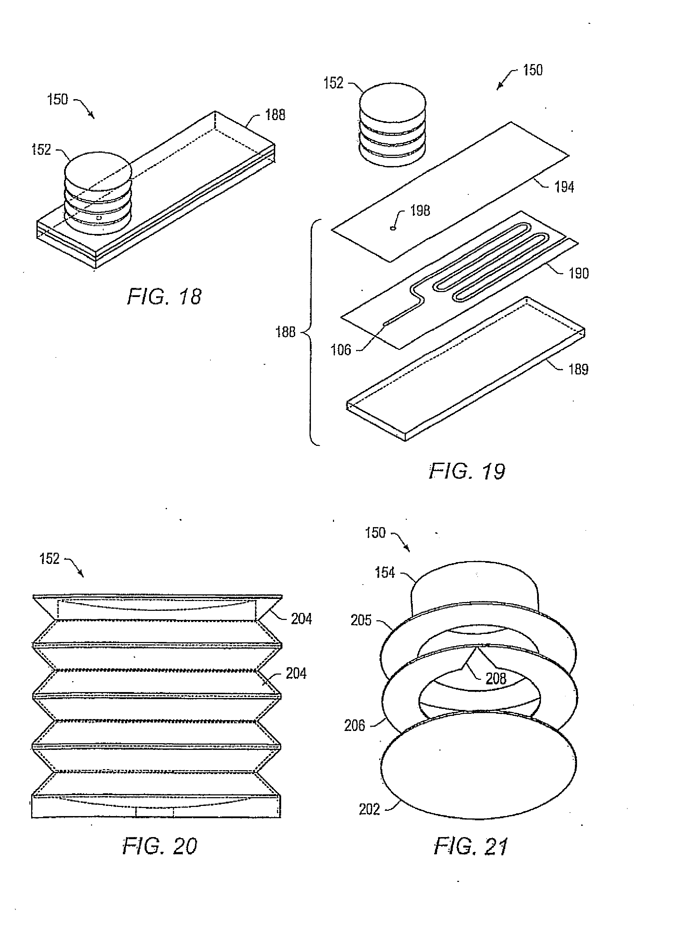

In some embodiments, a fluid package is coupled to a structure (*e.g.*, a planar support or a cartridge). The structure may provide support for the fluid package. FIG. 18 depicts an embodiment of fluid delivery system 150 that includes fluid package 152 coupled to support 188 (*e.g.*, a cartridge). FIG. 19 depicts an exploded view of fluid delivery system 150 depicted in FIG. 18. Support 188 may include support layer 189, channel layer 190 and top layer 194. Channel layer 190 may be coupled to support layer 189 and top layer 110. Channel layer 190 may be at least partially formed from double-sided adhesive. Channel layer may include channel 106.

Top layer 194 and support layer 189 may seal fluids in channel layer 190. Top layer 194 may include opening 198. Top layer opening 198 may direct fluid from fluid package 152 to channel layer 190. Top layer 194 or a portion of the top layer may include a material capable of coupling the top layer to fluid package 152 (*e.g.*, vinyl adhesive or other types of adhesive). In some embodiments top layer 194 and fluid package 152 are formed as one unit.

FIG. 20 depicts an embodiment of the fluid package depicted in FIG. 18 and FIG. 19. Fluid package 152 may include walls 204. Walls 204 may be formed of a material that allows the walls to be rigid while being able to collapse. Walls 204 may be corrugated and designed to fold. For example, walls 204 may form a shape similar to an accordion. Walls 204 may have limited outward flexibility under pressure. A corrugated fold may maximize the efficiency of the fluid package to deliver fluid. Walls 204 may be designed such that compression (full or partial) of the fluid package will not cause the base of the fluid package to flex upwards and/or cause the walls of the fluid package to flex outwards. In some embodiments, a diameter of the fluid package base is larger than a diameter of the fluid package opening and the top layer opening. The larger base may enhance bonding of the fluid package to the top layer. In some embodiments, fluid package 152 may have a rigid and/or ridged top surface. The rigid and/or ridged top surface may allow an actuator to contact the fluid package without puncturing the fluid package. The actuator may apply pressure to the top surface to force fluid from the fluid package.

FIG. 21 depicts an exploded view of a fluid delivery system that may be coupled to a support. Fluid delivery system 150 may include reservoir 154, gasket 206, and seal 202. Reservoir 154 includes one closed end and one open end. In some embodiments, the reservoir is formed from a mold made from Delrin® (DuPont, Wilmington, DE), an inflexible polymer, brass, stainless steel, and/or aluminum. For example, reservoir 154 may be molded from polydimethylsiloxane. The open end of reservoir 154 may include flange 205. Gasket 206 may couple flange 205 to seal 202. Seal 202 may be coupled to an opening in a top layer. Gasket 206 may include burst point 208. When a predetermined pressure is applied to reservoir 154, gasket 206 may rupture at burst point 208 causing seal 202 to rupture and/or tear. Rupturing of seal 202 allows fluid from reservoir 154 to flow through the opening in the top layer to a channel layer of the cartridge. In some embodiments, gasket 206 is a double-sided adhesive layer.

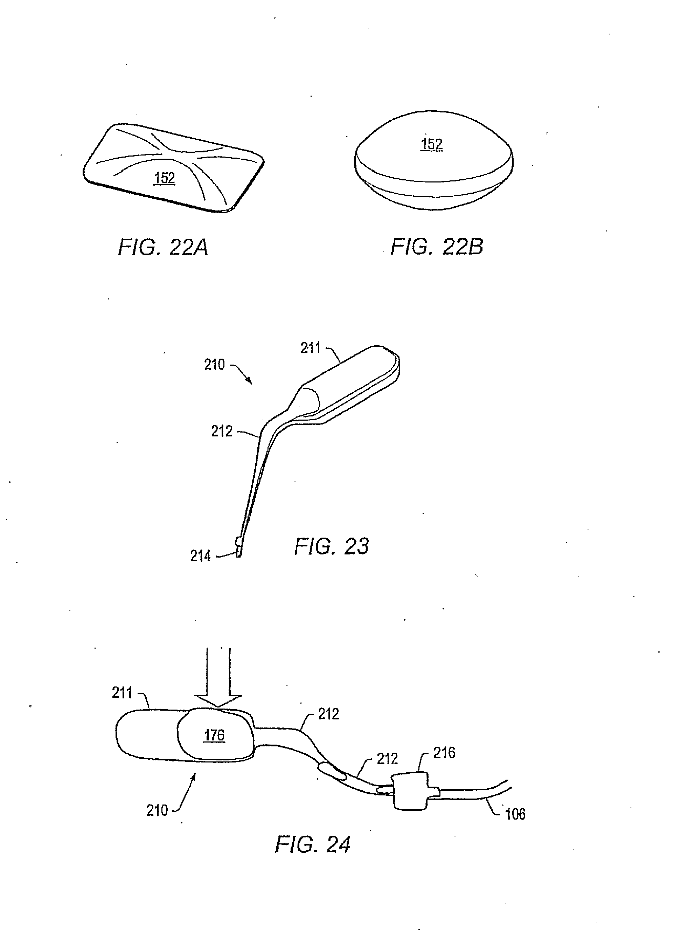

In some embodiments, a fluid delivery system includes a flexible conduit with a negative pressure source. The negative pressure source may be a fluid package. The negative pressure source may have a pressure less than ambient pressure. FIG. 22A depicts fluid package 152 as a negative pressure source before actuation. FIG. 22B depicts fluid package 152 as a negative pressure after actuation. When a negative pressure source is actuated (*e.g.*, a seal is removed, a seal is ruptured, or a conduit is inserted in a wall or seal of the negative pressure source), air and/or fluid are drawn towards the negative pressure source until the pressure equalizes (the negative pressure source inflates). Actuating or opening a negative pressure source may create at least a partial vacuum in one or more channels.

A fluid delivery system may include a fluid bulb coupled, integrated, or embedded into the cartridge. A cartridge may be designed to incorporate commercially available fluid bulbs or custom designed fluid bulbs. Fluid bulbs may have various dimensions depending on dispensing volumes required and/or cartridge specifications.

FIG. 23 depicts an embodiment of a fluid bulb. Fluid bulb 210 may include body 211, and conduit 212. Conduit 212 may be straight, angled and/or tapered. Conduit 212 may include tip 214. In some embodiments, tip 214 may be a breakaway sealed tip. Tip may be angled 214. Tip 214 may couple or removably couple to a cartridge.

FIG. 24 depicts an embodiment of a fluid bulb 210 coupled or removably coupled to a channel in the cartridge. Body 211 may release liquid 176 upon actuation. Body 211 may be coupled, via conduit 212, to connector 216. Connector 216 may connect fluid bulb 210 to channel 106 of the cartridge. In some embodiments, tip 214 may be positioned in connector 216. In certain embodiments, the connector may include one or more openings to allow more than one fluid delivery system to be attached to the connector. Connector 216 may be permanently affixed to conduit 212. In some embodiments, connector 216 may be removably coupled to conduit 212 and/or channel 106.

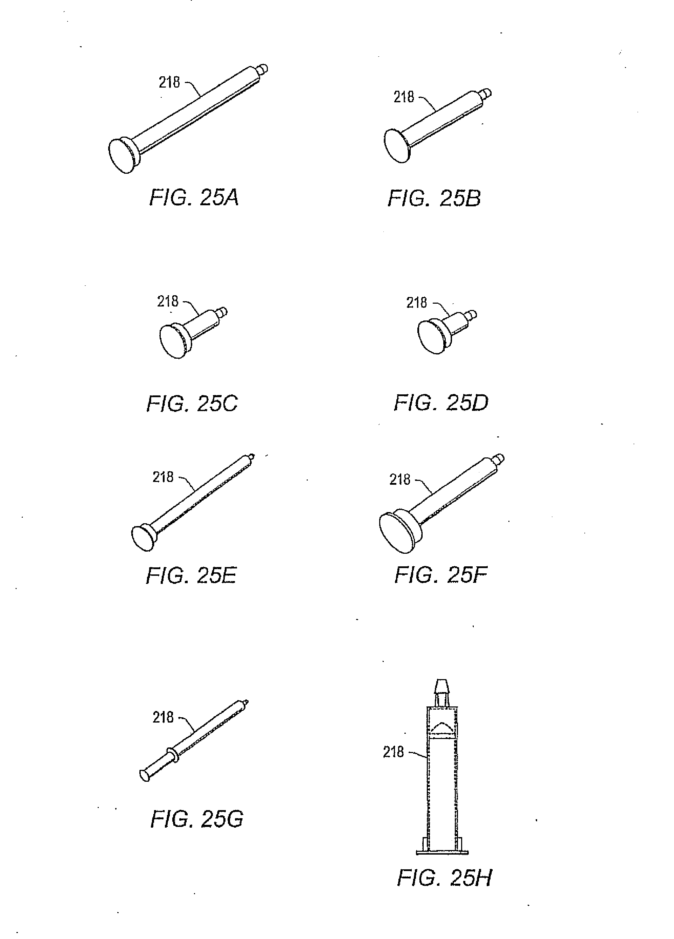

In some embodiments, a fluid delivery system may include one or more syringes coupled, embedded, or integrated into the cartridge. Syringes may be used to provide fluid delivery control, volume control, and/or a secure fluid seal to a cartridge. A syringe may be formed from a biocompatible material. Syringes may have a variety of designs, such as but not limited to, the embodiments depicted in FIGS. 25A-25H. The dimensions of syringes 218 may vary depending on dispensing volumes required and/or cartridge specifications. Use of a syringe in a fluid delivery system may offer accurate and/or precise fluid delivery. In some embodiments, prefilled syringes may be positionable in a cartridge prior to use.

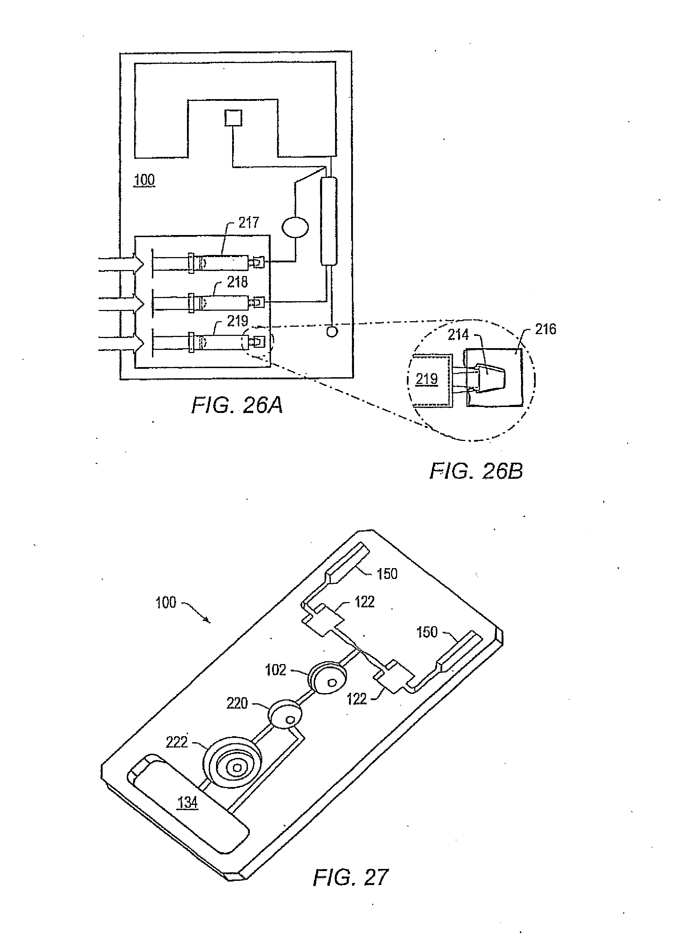

FIG. 26A depicts an embodiment of a cartridge that includes syringes 217, 218, 219. Syringes 217, 218, 219 may be linearly activated simultaneously or sequentially. Syringes 217, 218, 219 may be actuated when a prong contacts the fluid delivery system. In some embodiments, an actuator with three prongs of different lengths may be actuated to release fluid from the syringes. Using an actuator with prongs of different lengths may allow actuation of different syringes at different times using a single actuation of the prongs. Since the prongs are of different lengths, the actuation system may be set up such that each prong contacts a syringe at a different, predetermined, time. As each prong of the actuator depresses a syringe, fluid may be released. Syringes 217, 218, 219 may deliver fluid to various portions of the cartridge. For example, syringe 217 may deliver a fluid toward reagent region 122, while syringe 218 delivers fluid towards metered volume portion 138.

An expanded view of one the end of syringe 219 is depicted in FIG. 26B. Syringe 219 includes tip 214 positionable in connector 216. In some embodiments, connector 216 is coupled to the cartridge. Tip 214 may be designed to mate with connector 216. In some embodiments, a tip may include adhesive and/or a gasket to seal the syringe to the connector. A cartridge may include a spring mechanism that holds the syringes in position.

In some embodiments, a metered syringe pump is used to push and pull fluids through the system. During use, a capillary containing sample may be inserted into the cartridge coupled to a fluid bus. The system may then be filled with buffer through two lines. Using a third line, sample may be pushed into a trap that releases air trapped in the sample. A line may then be used to draw a predetermined amount of sample into the detection system. After sample analysis, the system may be washed with a buffer solution and waste may be transferred to a waste reservoir positioned in the cartridge or coupled to the cartridge.

In some embodiments, an analyte-detection system may be used to test for multiple analytes. The analyte-detection system may include a multi-functional cartridge. The multi-functional cartridge may include two or more detection systems. In some embodiments, a single cartridge or system may include a membrane-based detection system and a particle-based detection system. The membrane-based detection system may be positioned upstream from the particle-based detection system. A sample may be introduced into the cartridge or system and passed through the membrane-based detection system where a portion of the sample is retained by the membrane. The material passing through the membrane may be passed to the particle-based detection system. Particles in the particle-based detection system may interact with one or more analytes in the fluid passed over the particles. In alternate embodiments, a particle-based detection system may be positioned upstream from a membrane-based detection system. In certain embodiments, particles may be coupled to (*e.g.*, at least partially embedded in) at least a portion of a membrane of a membrane-based detection system. In combination, the two detection systems allow the presence of at least two analytes to be assessed in a single sample at about the same time.

FIG. 27 depicts perspective top view of an embodiment of a cartridge that includes two detection systems. Cartridge 100 may include fluid delivery systems 150, reagent regions 122, collection region 102, membrane-based detection system 220, particle-based detection system 222, and waste reservoir 134.

Sample may be deposited in and/or delivered to collection region 102. In some embodiments, a filter may be positioned proximate the collection region to allow removal of large particles and/or coagulated matter from the sample. In some embodiments, fluid may be released from fluid delivery systems 150 directly into channel 106. In some embodiments, fluid from the fluid delivery system may flow directly to one of the detection systems (*e.g.*, flow directly to the membrane-based detection system).

Fluid may be released from fluid delivery systems 150 and pass through reagent region 122. Reagent region 122 may include dried reagents, anti-coagulants, and/or visualization agents. In some embodiments, reagents and/or visualization agents on and/or in the reagent pad may be reconstituted by fluid passing over and/or through reagent region 122. In some embodiments, reagent region 122 includes reagent pads that contain dried reagents, anti-coagulants, and/or visualization agents. A reagent pad acts, in some embodiments, as a filter and removes large particles and/or coagulated matter from the sample.

In some embodiments, a reagent region may be positioned proximate the collection region so that sample from the collection region may pass over the reagent pad and reconstitute reagents and/or visualization agents in the reagent region. Directly flowing sample over and/or through a reagent region may facilitate the time of reaction between sample and reagents and/or visualization agents.

After fluid flows through and/or over reagent region 122, fluid may flow over and/or through collection region 102. A combined fluid and sample flows toward the membrane-based detection system 220 and particle-based detection system 222. In some embodiments, a combined fluid and sample passes through the particle-based detection system first. In certain embodiments, a combined fluid and sample may first pass through a first detection system for a first test and only pass through the second detection system based on the results of the first test.

Membrane-based detection system 220 and/or particle-based detection system 222 may be coupled to waste region 134. Fluid may flow from membrane-based detection system 220 and then to particle-based detection system 222 to waste region 134.

In some embodiments, a cartridge of an analyte-detection system may be multi-functional (*e.g.*, used to analyze two or more analytes in a sample). In some embodiments, the analysis may be done simultaneously, or substantially simultaneously. For example, a cartridge may be used to assess WBC count and CRP levels in a whole blood sample.

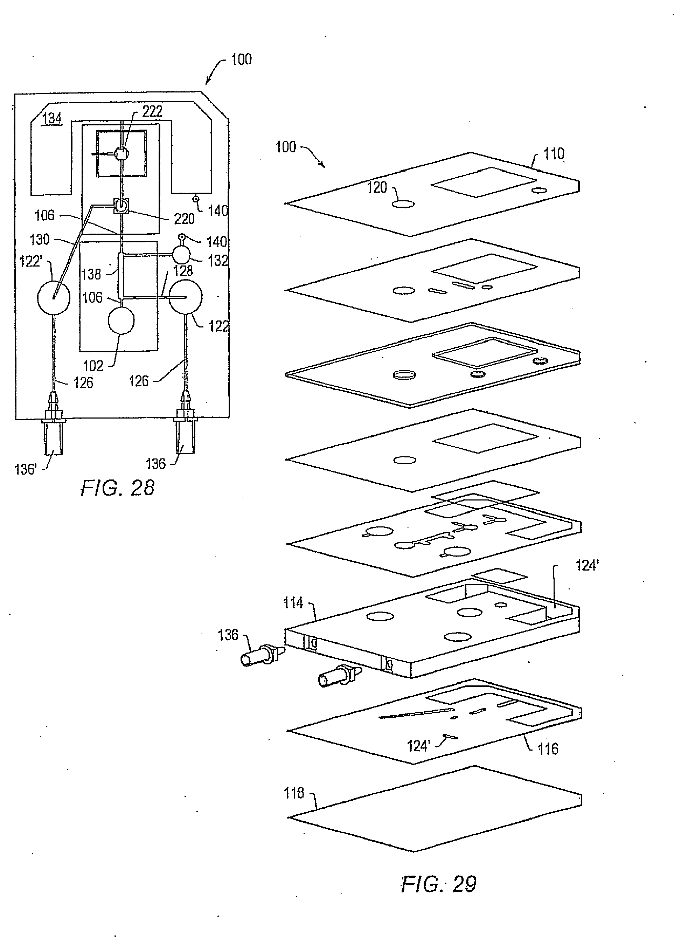

FIG 28 depicts a top view of an embodiment of multi-functional cartridge 100. Cartridge 100 may include connectors 136, 136', channels 106, 126, 128, 130, metered volume portion 138, collection region 102, reagent regions 122, 122', overflow reservoir 132, membrane-based detection system 220, particle-based detection system 222, waste reservoir 134, and vents 140.

Sample may be deposited in collection region 102. Sample flows from collection region 102 through channel 106 and enters metered volume portion 138. Sample may then be delivered to membrane-based detection system 220 from metered volume portion 138. Excess sample may be collected in overflow reservoir 132.

Connectors 136, 136' may connect one or more fluid delivery systems to the cartridges. Fluid from the fluid delivery systems flows through channels 126 to reagent regions 122, 122', respectively. Fluid may be delivered at different time intervals or substantially simultaneously to the reagent regions from separate fluid delivery systems. In some embodiments, fluid from the fluid delivery system may flow directly to one of the detection systems (e.g., flow directly to the membrane-based detection system).

Fluid may pass through or over reagent region 122, through channel 128 and enter metered volume portion 138. Fluid may be delivered to membrane-based detection system 220 from metered volume portion 138. Excess fluid and/or sample may be collected in overflow reservoir 132.

A similar fluid or different fluid that passed through or over reagent region 122 may pass through or over reagent region 122'. Fluid from reagent region 122' flows toward membrane-based detection system 220 through channel 130. In some embodiments, an additional amount of sample is delivered from metered volume portion 138 to membrane-based detection system 220 before fluid from reagent region 122' reaches the membrane-based detection system. In some embodiments, fluid from reagent region 122' may flow directly to particle-based detection system 222.

Sample and/or fluid that pass through or over membrane-based detection system 220 is transported to particle-based detection system 222. The detection systems may be optically coupled to a detector and the analytes in the sample may be analyzed. In some embodiments, the analytes in the sample retained in membrane-based detection system 220 may be analyzed prior to sending the remainder of the sample to the particle-based detection system 222. In some embodiments, the sample may be transported to the particle-based detection system 222 before being delivered to the membrane-based detection system 220.

Membrane-based detection system 220 and/or particle-based detection system 222 may be coupled to waste region 134. Fluid may flow from membrane-based detection system 220, to particle-based detection system 222, and then to waste region 134.

FIG. 29 depicts an exploded view of the embodiment of cartridge 100 depicted in FIG. 28. Cartridge 100 includes top layer 110, top layer opening 120, sample layer 114, reservoir layer 116, reservoirs 124, support layer 118, and connectors 136 designed to couple to fluid delivery systems. In certain embodiments, one or more additional fluid delivery systems (*e.g.*, fluid packages) may be coupled to, positioned on or positioned in cartridge 100 to provide fluid for sample processing during use.

Cartridges described herein may include a membrane-detection system. A membrane-detection system may include a membrane and, optionally, a membrane support. The membrane may retain at least a portion of matter in the sample, while allowing other portions of the sample to pass through the membrane. For example, with blood samples, a membrane may be selected that will allow red blood cells and plasma to pass through the membrane, while the membrane retains white blood cells.

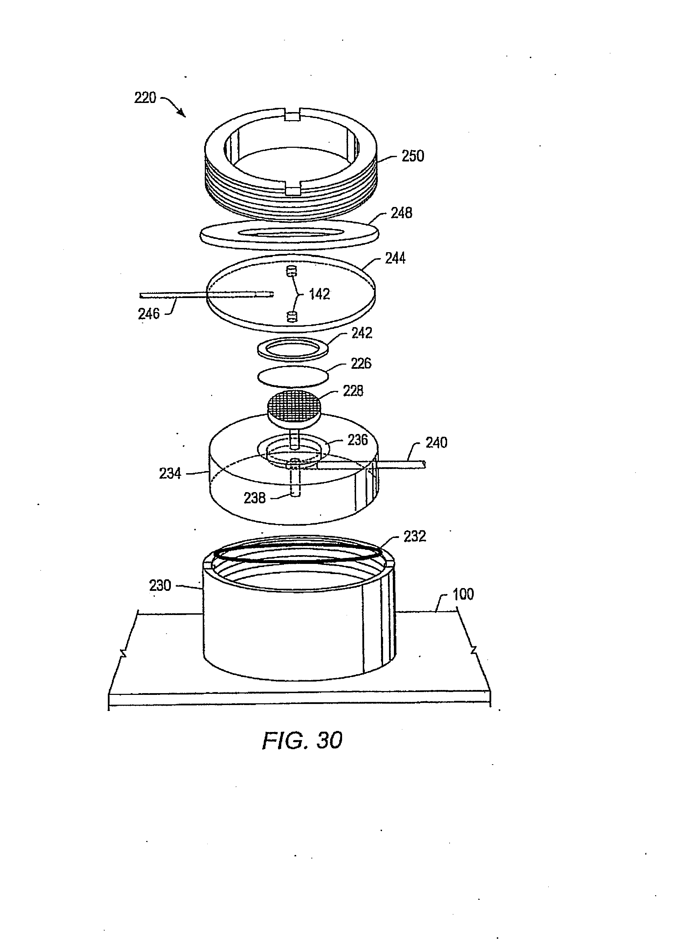

FIG. 30 depicts an embodiment of a membrane-based detection system. The membrane-based detection system may be coupled to, positioned in, or positioned on cartridge 100. The membrane-based detection system may be integrated within a cartridge.

Membrane-based detection system 220 includes membrane 226 and membrane support 228. In some embodiments, a membrane may be designed such that a membrane support is not necessary. For example, a thickness of a membrane may be selected so that a membrane remains substantially planar. In some embodiments, the membrane is porous.

The membrane-based detection system 220 may include housing 230 positioned on a cartridge 100. Bottom spacer 232 may position bottom member 234 in housing 230. Bottom member 234 may include indentation 236 to receive membrane 226 and membrane support 228. Channel 238 in bottom member 234 may receive fluids flowing through membrane 226 and conduct the fluids to outlet 240. In some embodiments, the outlet is coupled to a waste reservoir of the cartridge. Gasket 242 may be positioned between top member 244 and membrane 226. Gasket 242 may reduce leaks from the membrane-based detection system. Inlet 246 coupled to top member 244 may allow fluids to enter the membrane-based detection system. Top spacer 248 may be positioned between top member 244 and fastening member 250. Top member 244 may include viewing windows 142. Viewing windows 142 may be transparent to visible light and/or ultraviolet light. Fastening member 250 may keep the components of the membrane-based detection system coupled during use. Fastening member 250 may be machined (*e.g.*, threaded and/or tapered) to mate with housing 230.

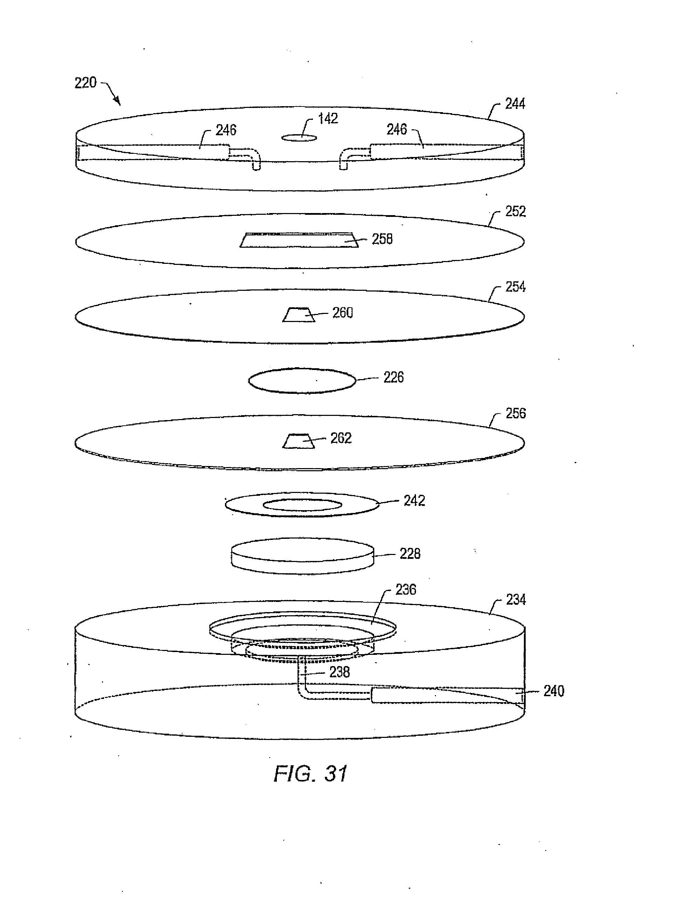

In some embodiments, a membrane-based detection system may include layers to direct fluid flow. FIG. 31 depicts an exploded view of an embodiment of a membrane-based detection system with directed fluid flow. The membrane-based detection system may include a plurality of layers positioned in the cartridge or on a surface of the cartridge. Membrane-based detection system 220 includes top member 244, top layer 252, middle layer 254, membrane 226, bottom layer 256, and membrane support 228. Layers of the membrane-based detection system may be coupled to each other. Top layer 252, middle layer 254, and bottom layer 256 may include openings 258, 260, and 262, respectively. Fluid may flow from inlet 246 through openings 258 and 260 to and/or through membrane 226. A portion of analytes in the fluid flowing to the membrane 226 may be retained on the membrane. Light may be directed to a portion of the membrane to detect analytes in the fluid. Fluid may flow through membrane 226, through opening 262 and out through outlet 240 to one or more reservoirs.

In some embodiments, a cavity is formed between the top member and the membrane. The top member may be spaced at a distance above the membrane to form the cavity and/or the top member may have a shape such that a cavity is formed between the top member and the membrane.

Top member 244 may be at least partially transparent to visible light and/or ultraviolet light. Top member 244 is, in some embodiments, formed of PMMA. Top member 244 may include viewing window 142. In some embodiments, a portion of top member 244 may be opaque or translucent to visible light and/or ultraviolet light while viewing window 142 may be substantially transparent to visible light and/or ultraviolet light.

Fluid may be directed towards membrane 226 through top layer 252 positioned below top member 244. A portion of top layer 252 may be formed of a material or materials (*e.g.*, vinyl material and/or an adhesive) capable of coupling the top layer to middle layer 254. Top layer 252 may direct flow of fluid from top member 244 through opening 258 and towards membrane 226.

Middle layer 254 may be positioned below top layer 252. Middle layer 254 may be formed of a vinyl material and/or adhesive. A portion of middle layer 254 may be formed of a material or materials (*e.g.*, vinyl material and/or an adhesive) capable of coupling the middle layer to top layer 252 and/or bottom layer 256. Middle layer 254 may be opaque or translucent to visible light and/or ultraviolet light. Middle layer 254 may direct fluid to flow through opening 260 toward membrane 226.

Fluid that flows through membrane 226 passes through opening 262 in bottom layer 256. Bottom layer 256 may direct fluid flow through opening 262. A portion of bottom layer 256 may be formed of a material or materials (e.g., vinyl material and/or an adhesive) capable of coupling the bottom layer to middle layer 254. In some embodiments, opening 262 in bottom layer 256 has a size similar to the size of opening 260. Openings with similar sizes may allow fluid to be retained in the area of membrane 226 between the middle layer 254 and bottom layer 256.

Gasket 242 may be positioned below bottom layer 256 to inhibit leaks from the membrane-based detection device. Membrane support 228 may be positioned below gasket 242. In some embodiments, membrane support 228 may inhibit sagging of membrane 226. Membrane support 228 may be positioned in bottom member 234 and/or an opening of the cartridge. Bottom member 234 may include indentation 236 to receive membrane 226 and/or membrane support 228. Channel 238 in bottom member 234 may receive fluids flowing through membrane 226 and conduct the fluids to outlet 240.

In some embodiments, a membrane is selected depending on the analyte of interest. The membrane may capture or retain matter in the sample (*e.g.*, particles, cells, or other matter). Matter may be retained on a surface of the membrane and/or in the membrane. The membrane may include a thin film or layer capable of separating one or more components from a liquid passing through the film or layer. The surface of a membrane may be hydrophilic to promote cell proliferation across the surface of the membrane. A membrane may have a variety of shapes including, but not limited to, square, rectangular, circular, oval, and/or irregularly shaped. In some embodiments, a membrane includes openings (*e.g.*, pores) that inhibit an analyte of interest from passing through the membrane. A membrane designed to capture substantially all of an analyte of interest may be selected depending on the analyte of interest.

In some embodiments, a membrane is a monolithic microchip with a plurality of high-density holes. The monolithic microchip membrane may be formed from materials including, but not limited to, glass, silica/germanium oxide doped silica, inorganic polymers, organic polymers, titanium, silicon, silicon nitride, and/or mixtures thereof. Organic polymers include, but are not limited to, PMMA, polycarbonate (PC) (*e.g.*, NUCLEOPORE® membranes, Whatman, Florham Park, NJ), and resins (*e.g.*, Delrin®). A membrane formed of polymeric material may include pores of a selected range of dimensions. In certain embodiments, a membrane is an acrylic frit. In some embodiments, a membrane is formed of multiple layers (*e.g.*, at least 2 layers, at least 3 layers, at least 4 layers, or at least 5 layers) of etchable and/or non-etchable glass. In some embodiments, a membrane is formed from an anti-reflective material and/or a material that does not reflect light in the ultraviolet-visible light range. In some embodiments, a membrane includes one or more locking mechanisms to assist in securing placement of the membrane in or on the cartridge or membrane support.

In some embodiments, membranes are microsieves. Microsieves may be manufactured from silicon materials and/or plastic materials. In some embodiments, a microsieve is a layered plastic microsieve.

Membranes may have a thickness from about 0.001 mm to about 25 mm, from about 1 mm to about 20 mm, or from about 5 mm to 10 mm. In some embodiments, a thickness of the membrane ranges from about 0.001 mm to about 2 mm. Membranes may have a diameter from about 1 mm to 500 mm, from about 5 mm to about 100 mm, or from about 10 mm to about 50 mm.

Pores of a membrane may have various dimensions (*e.g.*, diameter and/or volume). In some embodiments, pores of the membrane may have approximately the same dimensions. In some embodiments, membrane pores have a pore diameter ranging from about 0.0001 mm to about 1 mm; from about 0.0002 mm to about 0.5 mm; from about 0.002 mm to about 0.1 mm. The membrane pores have, in some embodiments, a pore diameter of at most 0.005 mm or at most 0.01 mm.

Pores of the membrane may be randomly arranged or arranged in a pattern (*e.g.*, a hexagonal close-packed arrangement). Pores of the membrane may occupy at least 10 percent, at least 30 percent, at least 50 percent, or at least 90 percent of the surface area of a membrane. The pores may assist in selectively retaining matter in a sample and/or a fluid.

In some embodiments, a membrane is positioned from about 0.3 mm to about 0.5 mm below a top surface of the cartridge. In some embodiments, the membrane includes a support. In some embodiments, a membrane is designed such that a membrane support is not needed (*e.g.*, utilizing a membrane having a thickness of at least 5 mm). In some embodiments, one or more layers separate the membrane and the membrane support. The membrane support may facilitate positioning of the membrane in or on the cartridge.

A membrane support may be coupled to the cartridge or integrated within a cartridge. In some embodiments, a membrane support is used to maintain a membrane in a substantially planar orientation. In certain embodiments, a membrane support is integrated with one or more membranes. The membrane support may be formed of the same material as the membrane. The membrane support may be formed of materials including, but not limited to, glass, polymers, metal, silicon, PC, cyclic olefin copolymer (COC), nylon, and/or nitrocellulose. The membrane support may be, but is not limited to, a stainless steel filter or a plastic mesh.

A support assembly may be coupled to the membrane support to allow the membrane and membrane support to withstand backpressures of at least 10 psi. The membrane support may be selected to produce a predetermined backpressure. When backpressure is controlled, cells may be more uniformly distributed across a surface of a membrane. Uniform distribution of cells across a membrane surface may facilitate imaging of a region containing cells and/or analyte detection.

In some embodiments, a membrane support includes open areas (*e.g.*, pores or holes). Open areas in the membrane support may have any shape, such as substantially square and/or substantially circular. The shape of the open areas in the membrane support may be different than the shape of pores in the membrane. Open areas of the membrane support may be equal to or greater than the diameter of the pores of the membrane. In some embodiments, a membrane support has open areas with diameters ranging from about 0.0001 mm to about 1 mm, from about 0.0002 mm to about 0.5 mm, or from about 0.002 mm to about 0.1 mm. The open areas have, in some embodiments, diameters of at most 0.005 mm or at most 0.01 mm.

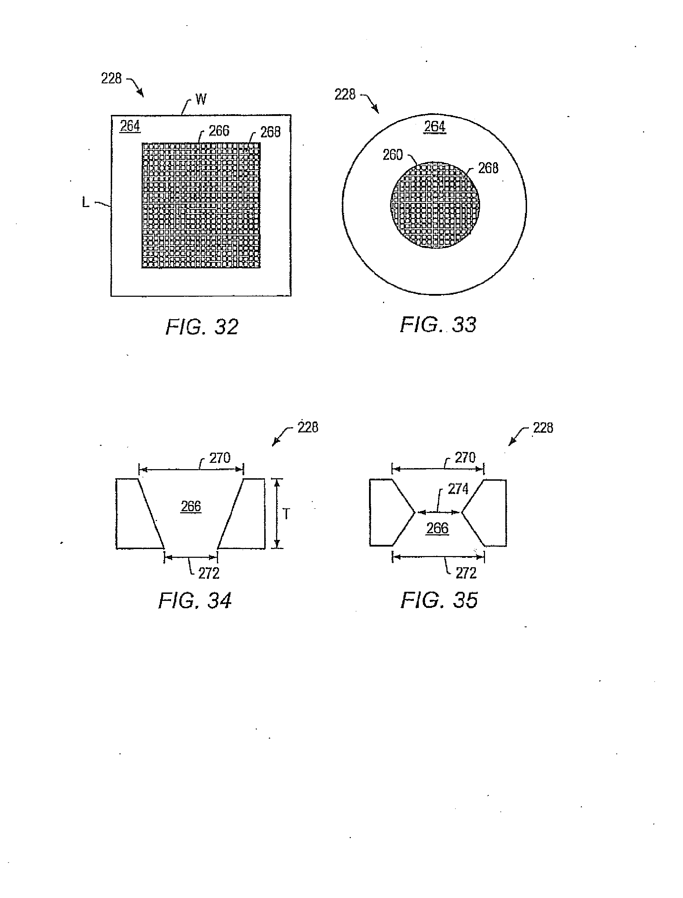

FIG. 32 depicts a top view of an embodiment of a membrane support having a parallelogram shape. Membrane support 228 may include outer area 264 and open area 266. Open area 266 may include openings 268. Membrane support 228 may be machined and/or fabricated such that open area 266 has various shapes. Various shapes of open area 266 may allow particles of different sizes to be removed during analysis of the analyte. Length (L) of outer area 264 may be greater than or about equal to width (W) of the outer area (*e.g.*, outer area 264 may have a substantially square shape or a substantially rectangular shape). A length of open area 266 may be greater than, or about equal to a width of the open area (*e.g.*, open area 266 may have a substantially square shape or a substantially rectangular shape). Open area 266 may have dimensions that are less than the dimensions of outer area 264. In some embodiments, an outer area of a membrane support may have a length about 4 mm to about 6 mm and a width from about 4 mm to about 6 mm. An open area of a membrane support may have a length from about 2.5 mm to about 4 mm and a width from about 2.5 mm to about 4 mm. FIG. 33 depicts a top view of an embodiment of membrane support 228 having an euclidian shape (*e.g.* membrane support 228 have a substantially oval shape or a substantially circular shape). Open area 266 may have dimensions that are less than the dimensions of outer area 264.

FIG. 34 depicts a perspective cross-sectional view of open area 266 of membrane support 228. Open area 266 includes top portion 270 and bottom portion 272. Bottom portion 272 may be equal to or less than the top portion 270. In some embodiments, a membrane support may include a top portion formed from a silicon nitride film and a bottom portion formed from silicon. A membrane support may be formed from a hydrophilic and/or anti-reflective material. Forming a membrane support from a hydrophilic material may reduce the formation of air bubbles across the membrane and membrane support. Use of a hydrophilic material may also inhibit nonspecific binding of analytes. Using a membrane support made at least partially of anti-reflective material may enhance analyte detection.

In embodiments where the membrane support is formed from silicon, a bottom portion of the membrane support has a thickness (T) ranging from about 0.001 mm to about 5 mm. For silicon membrane supports, a thickness of the membrane support is related to a length (Lt) of the top portion 270 and a length (Lb) of the bottom portion 272 as represented by the equation:

$$T = \tan(54.7) \ x \ (Lt\text{-}Lb)/2.$$

FIG. 35 depicts a perspective cross-sectional view of open area 266 of membrane support 228. Open area 266 includes top portion 270, middle portion 274, and bottom portion 272. A length of middle portion 274 may less than a length of top portion 270 and a length bottom portion 272. Thus, an hourglass shaped opening is formed.

In a membrane-detection system, a fluid and/or sample in the detection region of the cartridge may be treated with a light. Interaction of the light with the fluid and/or sample may allow the analyte to be detected. Light from one or more light sources may shine on or in at least the detection region of a cartridge, such as the portion of the membrane where the fluid and/or sample is retained. The light may allow a signal from the retained fluid and/or sample to be detected. When light shines on a membrane surface, some of the light may be reflected. Areas proximate the detection region may also reflect some of the light that shines on a sample. Light reflecting from the membrane surface and/or membrane support may interfere with obtaining an accurate reading from the detector and so it may be advantageous to optically couple an anti-reflective material to the membrane and/or the membrane support.

In some embodiments, an anti-reflective material is optically coupled to the membrane and/or the membrane support. Alternatively, an anti-reflective material may be a coating on a surface of the membrane and/or membrane support. For example a black coating on a surface of the membrane and/or membrane support may act as an anti-reflective coating.

In certain embodiments, a portion of the membrane and/or membrane support may be made of an anti-reflective material. The anti-reflective material may be positioned above or below a membrane. An anti-reflective material may inhibit the reflection of light applied to analytes retained in or on the membrane. The anti-reflective material may absorb one or more wavelengths of light that are emitted by an analyte of interest. The anti-reflective material may improve the contrast of an image of at least a portion of the analyte retained in or on the membrane by inhibiting reflection of light.

In some embodiments, materials that form the components of the cartridge control flow of fluids through the cartridge. In some embodiments, hydrophilic material is coupled to the membrane and/or membrane support. Alternatively, hydrophilic material may be a coating on a surface of a membrane and/or membrane support. In certain embodiments, a portion of the membrane and/or membrane support is made from hydrophilic material. Hydrophilic material may enhance flow of a fluid through the membrane. Hydrophilic material may reduce the formation of air bubbles across the membrane and membrane support and/or inhibit nonspecific binding of analytes. Hydrophilic material may attract or have an affinity for aqueous fluids flowing through the membrane. Hydrophilic material may be positioned downstream of the membrane.

In some embodiments, hydrophobic material is positioned in or on the cartridge. Hydrophobic material may repel aqueous fluid away from surfaces of the cartridge and cause the fluid to flow towards the membrane. For example, positioning a top member above the membrane forms a cavity between the top member and the membrane. Hydrophobic material may be coupled to the top member. The hydrophobic material may be a coating on a surface of the top member, and/or the hydrophobic material may form a portion of the top member. As an aqueous sample or fluid enters the cavity, it is repelled away from the hydrophobic top member and flows towards the membrane.

A membrane-based detection system may be used alone or in combination with a particle-based detection system. In some embodiments, a particle-based detection system includes a supporting member with one or more cavities. One or more particles may be positioned in the cavities of the supporting member. In some embodiments, a particle-based detection system detects one or more analytes simultaneously using reactive particles that interact with the analytes.

In a particle-based detection system, a particle may produce a signal in the presence of an analyte. Particles may produce optical (e.g., absorbance or reflectance) or fluorescence/phosphorescent signals upon exposure to the analyte. Particles include, but are not limited to, functionalized polymeric beads, agarose beads, dextrose beads, polyacrylamide beads, control pore glass beads, metal oxides particles (e.g., silicon dioxide ($SiO_2$) or aluminum oxides ($Al_2O_3$)), polymer thin films, metal quantum particles (e.g., silver, gold, and/or platinum), and semiconductor quantum particles (e.g., Si, Ge, and/or GaAs).

The particles may include a receptor molecule coupled to a polymeric bead. The receptors, in some embodiments, are chosen for interacting with analytes. This interaction may take the form of a binding/association of the receptors with the analytes. A particle, in some embodiments, possesses both the ability to bind the analyte of interest and to create a modulated signal. The particle may include receptor molecules, which possess the ability to bind the analyte of interest and to create a modulated signal. Alternatively, the particle may include receptor molecules and indicators. The receptor molecule may posses the ability to bind to an analyte of interest. Upon binding the analyte of interest, the receptor molecule may cause the indicator molecule to produce the modulated signal. The receptor molecules may be naturally occurring or synthetic receptors formed by rational design or combinatorial methods. Natural receptors include, but are not limited to, DNA, RNA, proteins, enzymes, oligopeptides, antigens, and antibodies. Either natural or synthetic receptors may be chosen for their ability to bind to the analyte molecules in a specific manner.

Some particle-based detection systems and particles for use in particle-based detection systems are described U.S. Patent Application No.: 09/616,731; U.S. Application Publication Nos.: 20020160363; 20020064422; 20040053322; 20030186228; 20020197622; 20040029259; 20050136548; and 20050214863; and U.S. Patent Nos.: 6,680,206; 6,602,702; 6,589,779; 6,649,403; 6,713,298; and 6,908,770.

In some embodiments, components necessary to obtain and assist in the analysis of a fluid and/or sample are included in a single package as a kit. In some embodiments, a package includes a cartridge, a sample collection device (*e.g.*, a lancet, a syringe, or a needle), and one or more disinfectant wipes. Disinfectant wipes may be used prior to using the sample collection device to draw a sample from a person. A disinfectant wipe may also be used by a user to wipe portions of the analyte-detection system before or after sample analysis. Packaging a cartridge and a sample collection device together may make collection and analysis of samples easier for an operator. Packaging a cartridge and a sample collection device together may inhibit contaminants from entering the cartridge and the sample collection device.

A package may be sealed to inhibit entrance of air (*e.g.* vacuum sealed). A package may be formed from a material that has at least one of the following properties: is waterproof, is water resistant, controls static electricity, kills microbes that enter the package, blocks sunlight, and blocks UV light. Materials that have these properties include polymeric materials or metal foils. A package may have a positive pressure to protect items in the package. Insulating materials, such as polyurethane or bubble wrap, may be placed inside a package to protect items in the package.

It may be desirable for the analyte-detection cartridge and/or system to include a control to ensure that the cartridge and/or system are operating correctly. Long storage times and/or less than ideal storage facilities may damage and/or affect the quality of the cartridge and/or components of the cartridge.

In some embodiments, it is desirable to check the fluids and/or reagents stored in the cartridge. A particle larger than cells to be detected or other particles in the sensor array may be placed in a detection system as a control analyte. For example, a control analyte includes any type of particle previously described, including quantum particles or dots. Control analytes may allow assessment of a cartridge and/or equipment used in conjunction with the cartridge, such as, but not limited to, light sources, detectors, analyzers, and/or computer systems. The control analyte may produce a result within a selected range and/or produce a result substantially similar to an expected result from a selected analyte.

In some embodiments a control analyte is a control particle. A control particle may be produced by coupling a known analyte to a particle. Reagents passing over the detection system may interact with the sample and the control particle. When an image of the detection system is captured the control particle is used to determine if the cartridge is functioning properly. For example, if a control particle is not detected, the quality of the reagents may be determined to be poor and the cartridge and assay discarded. In some embodiments, a control particle is distinguishable from other matter in the detection system due to the size of the control particle.

In some embodiments, a control analyte is stored in or on the cartridge. For example, a bead containing a known analyte may be designed to produce a predetermined signal. A weak or non-existent signal from the control analyte may indicate an improperly functioning cartridge.

In certain embodiments, a cartridge control system may be coupled to, positioned in, positioned on or integrated in the cartridge. The cartridge-control system may include, but is not limited to, one or more control analytes, one or more buffer solutions, and one or more reagent pads containing a dried predetermined analyte. In some embodiments, the cartridge-control system includes one or more fluid packages. The fluid packages may include one or more control analytes one or more control solutions, and/or other reagents. Prior to analyzing a sample, a control solution may be released from the fluid packages and pass over detection system.

In some embodiments, the detection system includes a control-detection system and an analyte-detection system. The known or control analyte may be applied to the control-detection system and the sample may be applied to the analyte-detection system. If the known analyte is captured by the control-detection system and a predetermined signal is produced, the cartridge is considered to be operating properly. If the known analyte passes through the control-detection system but does not produce an appropriate signal, it may indicate that the cartridge is not working properly (*e.g.*, due to improper storage and/or age of the cartridge). Improperly working cartridges may be discarded prior to deposition of a sample on the cartridge. Once the quality of the cartridge has been confirmed, the sample is analyzed for analytes.

In some embodiments, a single detection system may be used to analyze the control analyte and the sample analytes. For example, if the known analyte is detectable in a detection system, the detection system may then be washed (*e.g.*, laterally washing matter off the surface and/or back washing matter off the surface) to remove the known analyte from the detection system. After cleaning the detection system, a sample may be introduced to the detection system and a sample analysis performed.

In some embodiments, a detection system may be washed prior to use with fluid from a fluid delivery system. For example, a fluid package is coupled via a channel to a side or bottom surface of the detection system. Fluid from the fluid package washes the detection system such that the wash fluid, and any matter contained in the wash fluid, passes into an outlet channel of the detection region and into a waste region.

In some embodiments, an analyte-detection system is used with different cartridges to detect a plurality of analytes. The analyte-detection system may include a housing. The housing may include a slot for receiving a cartridge. In some embodiments, the housing includes an optical platform and/or an analyzer.

In some embodiments, an analyte-detection system may include an analyzer (e.g., a computer system). The analyzer may analyze images and/or control the one or more components of the analyte-detection system. The analyzer may be coupled to the housing and/or an optical platform of the analyte-detection system. The analyzer and/or analyte-detection system may include a display to show images produced by the detector. The analyzer and/or analyte-detection system may include a temperature controller. A temperature controller may control temperatures of or around the housing or components of the analyte-detection system.

The analyte-detection system may include a cartridge positioning system. In some embodiments, the cartridge positioning system is included in a housing of the analyte-detection system. The cartridge positioning system may automatically position the cartridge so that it is optically coupled to one or more light sources and/or one or more detectors. In some embodiments, one or more detectors and/or one or more light sources are coupled or directly attached to an optical platform.

One or more detectors may include, but are not limited to, a CCD detector, a CMOS detector, a camera, a microscope, or a digital detector. One or more detectors may detect one or more signals from an analyte. For example, a CMOS detector may be used for detection in membrane-based detection systems or for quantitative measurements while a CCD camera detector may be used for detection in particle-based detection systems. A signal may be represented by one or more wavelengths of light absorbed by: the analyte; matter retained on a membrane; a fluorophore; a particle, or combinations thereof. A signal may be represented by the fluorescence of: the analyte; matter retained on a membrane; a fluorophore; a particle; or combinations thereof. The detector may transform the signal to one or more images. The images may be of: one or more analytes in one or more fluids; samples retained on or in one or more membranes; one or more particles of a detection system; or combinations thereof.

In certain embodiments, a monochromatic detector may be used. When a monochromatic detector is used with multiple fluorophores and excitation sources, one or more filters may be used to isolate light emitted in a predetermined spectrum. For example, a green filter may be used to isolate the light emitted from the green fluorophore, and thus an image of the detection system may only include material that emits green light. A red filter may be used to isolate light emitted from a red fluorophore.

In some embodiments, one or more light sources may emit light of different wavelengths. For example, a light source may be capable of emitting two different wavelengths of light. Different wavelengths of lights may enhance detection of various types of analytes. In certain embodiments, different assays require different exposure times when images of the detection systems are obtained. An exposure time from approximately 1-5 seconds may be used.

In some embodiments, two light sources (*e.g.*, blue and red LED light sources) and one or more detectors may be used to assist in detection of an analyte in a fluid and/or sample. Each light source may emit light at a different wavelength. For example, two light sources may be included in an optical platform and different combinations of light sources may be used to detect different analytes. Blue and red light sources may be used for CD4 cell assays, *E. coli* assays, ß-galactosidase assay (BG) assays, and cell based assays. A blue light source may be used for CRP, tumor necrosis factor-$\alpha$ (TNF-$\alpha$), and BG assays. A red light source may be used for interleukin-6 (IL-6) assays.

In some embodiments, an analyte-detection system includes several different lenses for the detection of different analytes. More than one lens may be used in the detection of some analytes. The lenses may be included in an optical platform and/or as part of a detector. Lenses of different magnification levels may be used in the analysis of one or more analytes. Lens magnification levels may include, but are not limited, 4x, 10x, and/or 20x. For example, a 10x lens may be used for CD4 assays, while a 4x lens may be used for CRP, TNF-$\alpha$, and IL-6 assays. Alternatively, a 4x lens and a 10x lens are used in the detection of *E. coli* and/or BG assays.

In some embodiments, fiber optic cables are coupled to a detection system to facilitate image capturing. In certain embodiments, fiber optic cables are coupled to a particle-based membrane detection system to facilitate analyte-detection and reduce the need to adjust magnification between detection regions

In some embodiments, an analyte-detection system includes a motor coupled to a lens and/or a detector. The motor may be coupled to the housing, the optical platform and/or a detector of the analyte-detection system. A motor may move the lens and/or the detector in a direction perpendicular to the plane the cartridge is positioned in, or the z-axis. Moving the lens and/or the detector vertically along the z-axis may focus the image of the detection region.

In some embodiments, a cartridge is coupled to a motor, actuator, or a cartridge positioning system designed to move the cartridge in the z-direction to focus an image of the detection region. A cartridge may be moved to allow more than one image of analytes to be captured in more than one detection system. For example, a cartridge contains more than one detection region. The area of interest in the detection systems may be too large to be captured with one image, thus the cartridge may be moved horizontally or in any direction along the x-y plane to obtain images of the desired areas.

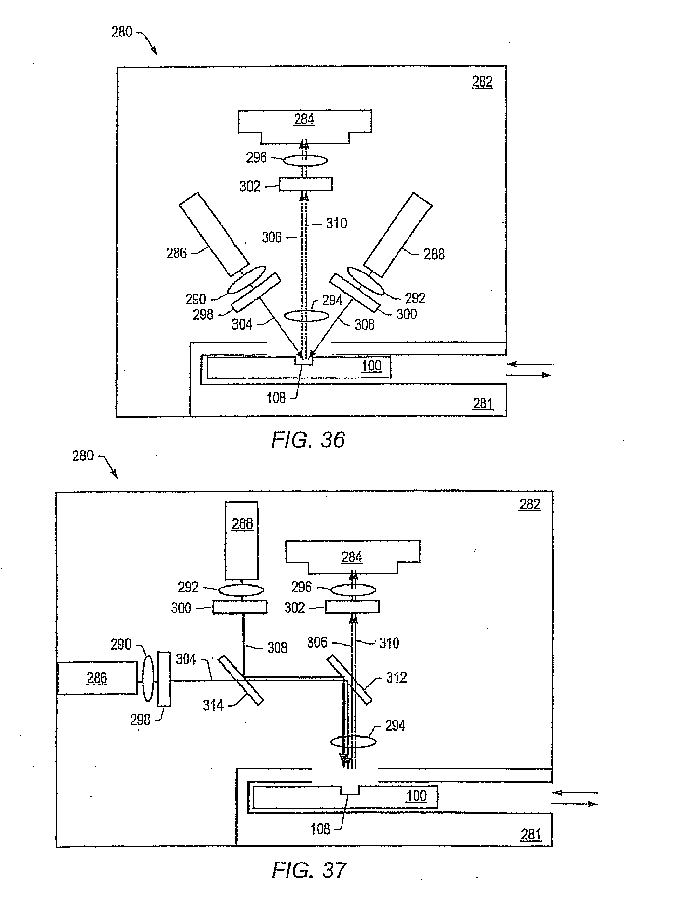

FIG. 36 depicts a cartridge positioned in an analyte-detection system. Analyte-detection system 280 includes cartridge 100, housing 281 and optical platform 282. Optical platform 282 includes detector 284, light sources 286, 288, lenses 290, 292, 294, 296 and filters 298, 300, 302. Cartridge 100 may be positioned automatically and/or manually in housing 281. Light 304 (*e.g.*, a white light) from light source 286 may be collimated with lens 290, filtered to a desired wavelength using filter 298 (*e.g.*, filtered to a wavelength in a blue portion of visible light), and directed in or on a detection system positioned in detection region 108 of cartridge 100. In some embodiments, light from a light source may enter the cartridge at an angle. For example, the light source may be positioned at a 45° angle with respect to the detector and/or the cartridge. Filter 298 (*e.g.*, excitation filters and/or clean-up filters) may be used to narrow excitations from light emitting diodes and/or other light sources. For example, filter 298 may be a D467/20x filter capable of filtering light to a wavelength ranging from about 450 nm to about 480 nm (*e.g.*, 457 nm to about 477 nm). Filter 300 may be a 635/20x filter capable of filtering light to a wavelength ranging from about 625 nm to about 645 nm.

After light is directed into detection region 108, light 306 (*e.g.*, signal) produced from interaction of the analyte with the sample may then be obtained using detector 284. The signal may be transformed into an image representing the desired analyte. In some embodiments, the image represents a membrane of the detection system and/or one or more analytes in the fluid and/or sample. Detector 284 includes, but is not limited to, a digital detector, a CMOS camera, or a CCD device. In some embodiments, moving the optical platform along the axis perpendicular to the cartridge while the cartridge is held static allows images of the cartridge to be brought into focus for the detector. Emission filter 302 may be used with detector 284. For example, light 306 reflected from the detection region 108 passes through lens 294 and/or an emission filter 302. Lens 296 is used to collimate light 306 from detection region 108 and/or focus the light from the detection region to detector 284. Emission filter 302 may be a dual band emission filter that allows transmission between about 504 nm and about 569 nm and between about 670 nm and about 822 nm.

Next, light 308 from light source 288 is collimated with a lens 292, filtered to a desired wavelength with filter 300, and focused on a sample. Emitted light 310 produced by interaction of the analyte with the sample and emitted from detection region 108 passes through lens 294 and/or emission filter 302 and is collimated with lens 296 to detector 284. Detector 284 obtains the signal from illumination of detection region 108 with light source 288. Emitted light 310 is transformed into an image representing an image of the detection region. It should be understood that additional light sources (*e.g.*, a third light source, a fourth light source, a fifth light source, etc.) may also be used. Signals produced from the detection region may then be processed to produce images of a portion of the detection region (e.g., a portion of a membrane) and/or of analytes present in the sample. In some embodiments, an analyzer determines the identity and/or presence of the analytes.

FIG. 37 depicts an alternative arrangement for an analyte-detection system 280. Optical platform 282 includes light sources 286, 288. Light sources 286, 288 emit light in a range from about 460 nm to about 480 nm, from about 465 nm to about 475 nm, or from about 460 nm to about 470 nm. During use, detection region 108 of cartridge 100 may be positioned automatically or manually in housing 281. Detection region 108 contains one or more detection systems (*e.g.*, a membrane-based detection system and/or a particle-base detection system). The detection system includes at least one sample and at least one visualization agent. Light 304 from first light source 286 is collimated with lens 290, filtered to a desired wavelength using filter 298, reflected 90 degrees by dichroic mirror 312, and focused on a detection system in detection region 108 with lens 294. In some embodiments, the dichroic mirror is a combination of dichroic mirrors. The dichroic mirror may include one or more reflection bands and/or one or more transmission bands. For example, dichroic mirror 312 may be a Z502RDC long pass dichroic mirror, which is a dual band dichroic mirror having 2 reflection bands and 2 transmission bands. One reflection band of a dichroic mirror may reflect light at a wavelength ranging from about 463 nm to about 483 nm and transmit light ranging from about 502 nm to about 587 nm. A second reflection band of the dichroic mirror may reflect light at a wavelength ranging from 603 nm to about 637 nm and transmit light at a wavelength ranging from about 656 nm to about 827 nm.

Light 306 reflected and/or emitted from detection region 108 passes through lens 294, is filtered to predetermined wavelengths with filter 302 (*e.g.*, a dual band emission filter), collimated with lens 296, and processed by detector 284 to produce an image of the detected analytes.

Light 308 from second light source 288 is collimated with lens 292, filtered to a desired wavelength with filter 300. Filter 300 is a different filter than filter 298, thus light 308 has a different wavelength than light 304. Filtered light 308 is reflected 90 degrees by dichroic mirror 314, reflected 90 degrees by dichroic mirror 312, and focused on or in detection region 108 using lens 294. Light 310 reflected and/or emitted from detection system 108 passes through lens 294, passes through dichroic mirror 312, is filtered to predetermined wavelengths with filter 302, is collimated by lens 296, and processed by detector 284 to produce an image of the detected analytes. Filter 302 may be a dual band emission filter capable of filtering light at two different ranges of wavelengths (*e.g.*, a first wavelength from about 504 nm to about 569 nm and a second wavelength from about 607 nm to 822 nm).

The signal obtained by detector 284 may then be analyzed (*e.g.* using an analyzer) to determine the presence and/or identity of analytes in the detection region. Any number of light sources may be used in a similar manner as described above. It may be desirable to use a plurality of light sources to substantially simultaneously detect a plurality of analytes.

In some embodiments, a single light source with a beam splitter is used instead of multiple light sources. Using one excitation source may reduce costs. The single light source may excite two or more visualization agents applied to matter captured on a membrane of a detection system of a cartridge. The emission of light from the detection system may be separated using one or more dichroic mirrors and one or more detectors.

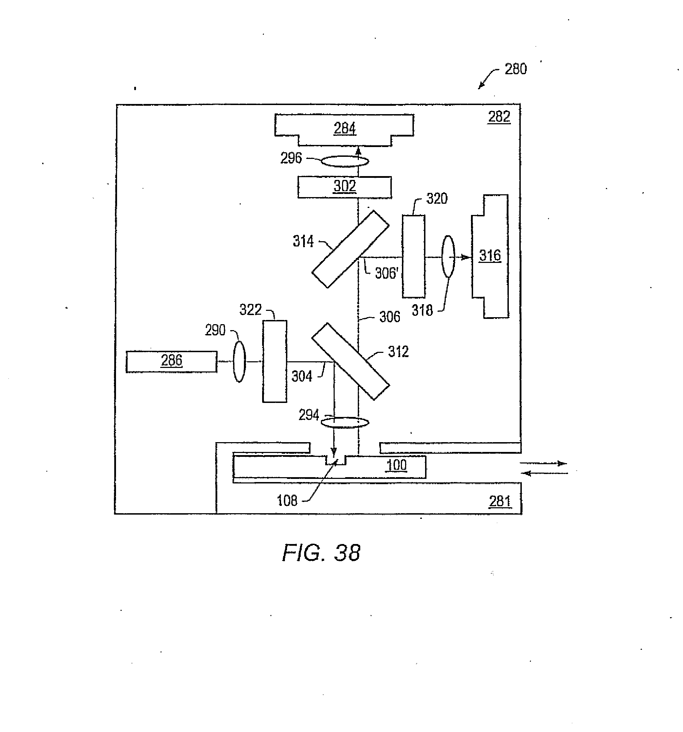

FIG. 38 is a schematic of a cartridge positioned in an analyte-detection system with an optical platform that includes a single light source. Analyte-detection system 280 includes cartridge 100, housing 281, and optical platform 282. Optical platform 282 includes detectors 284, 316, light source 286, lenses 290, 294, 296, 318, filters 302, 320, dichroic mirrors 312, 314 and shutter 322.

Light 304 from single source 286 is collimated with lens 290, passed through shutter 322, reflected 90 degrees by dichroic mirror 312, and focused on detection region 108 of cartridge 100 with lens 294. Shutter 322 is positioned between lens 290 and dichroic mirror 312. Shutter 322 may block light from shining on detection region 108 and/on cartridge 100. Light 306 reflected and/or emitted from a detection system of detection region 108 may pass through lens 294, dichroic mirrors 312, 314, filter 302, and lens 296 where light 306 is collimated onto detector 284. A portion of light 306, depicted as light 306', may be reflected using dichroic mirror 314, pass through filter 320 (*e.g.*, a dual band emission filter), and lens 318 where light 306' is collimated onto detector 316.

In some embodiments, an actuator is used to move a series of different emission filters into the path of light entering a detector. The ability to use different emission filters allows more than one signal from the detection region of the cartridge to be analyzed by one detector. The use of one detector and more than one filter may enhance the sensitivity of a test process, allowing less sample to be used for an analysis of multiple analytes. Determination of the appropriate emission filters to position in front of the detection system may be based on data obtained from a barcode located on the cartridge.

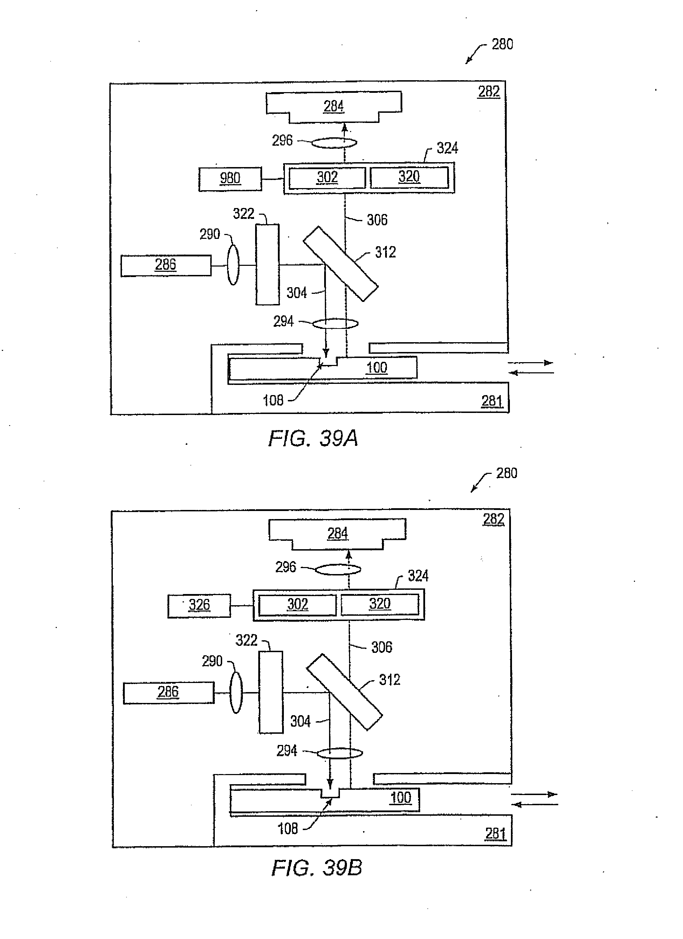

FIG. 39A is a schematic diagram of a cartridge positioned in an analyte-detection system that includes an optical platform equipped with an actuator. The actuator is designed to position a series of filters in front of a detector. Analyte-detection system 280 includes cartridge 100, housing 281, and optical platform 282. Optical platform 282 includes detector 284, light source 286, lenses 290, 294, 296, dichroic mirror 312, shutter 322, filter holder 324, filters 302, 320, and actuator 326. Light 304 from light source 286 is collimated with lens 290, passed through shutter 322, reflected 90 degrees by dichroic mirror 312, and focused onto detection region 108 of cartridge 100 with lens 294. Light 306 reflected and/or emitted from a detection region 108 may pass through lens 294, pass through dichroic mirror 312, pass through filter 302 or filter 320 positioned in filter holder 324, and lens 296 where light 306 is collimated onto detector 284. Filter holder 324 may include additional emission filters depending on the analyte to be analyzed. Filter holder 324 is coupled to actuator 326, which is designed to move filter holder 324. Actuator 326 may move filter holder 324 based on a signal from detector 284 and/or an analyzer of analyte-detection system 280. Filter holder 324 may be positioned between cartridge 100 and detector 284. In some embodiments, actuator 326 may move filter holder 324 such that filter 320 may be positioned between detector 284 and detection region 108 such that light 306 may pass filter 320 and into detector 284, as shown in FIG. 39B, allowing analysis of the detection region using a different wavelength of light. The filter light (*e.g.*, filtered signal) may then be analyzed in the detector to produce an image and/or data of analytes in the fluid and/or sample. A plurality of images and/or data from the fluid and/or sample may be obtained using a plurality of emission filters placed sequentially in front of the detector.

Analyte-detection systems described herein may be used to identify the presence of a plurality of analytes in a sample. Analyte-detection systems may be designed for detection of one or more specific analytes (*e.g.*, cellular components, proteins, or pathogens such as viruses, bacteria, fungi or parasites, or combinations thereof) typically associated with various infections, diseases, illnesses, and/or syndromes. Examples of diseases, illnesses, viruses and syndromes include, but are not limited to, AIDS, malaria, heart disease, atherosclerosis, cancer, tuberculosis, mononucleosis, syphilis, sickle-cell anemia, herpes virus, HIV, Good's syndrome, or Sjogren's syndrome. Examples of herpes viruses include, but are not limited to, Epstein-Barr virus (EBV), cytomegalovirus (CMV), herpes simplex viruses 1 and 2 (HSV1 and HSV2), varicella-zoster virus (VZV), Kaposi's sarcoma-related virus (HHV8), herpes lymphotropic virus (HHV6), and human herpes virus 7 (HHV7).

Analysis of human blood samples may allow for early detection of various diseases, illness, viruses and/or syndromes. For example, WBCs and RBCs may be separated and analyzed to determine specific diseases, illnesses, viruses, and/or syndromes. In some embodiments, WBCs are separated from RBCs and immunotyped to determine the total number of various cell types in a sample and/or their ratio relative to other cell types. A five-part WBC differential, which is part of a typical complete blood count, may be used for general illness assessment. A five part WBC differential may sort out results based on counts of various white blood cells in various classes of diseases and may be used to diagnose viral, bacterial, allergic and immune diseases.

Samples may be analyzed by characterizing one or more components of a blood sample, including the fluid component of whole blood, such as serum or plasma. Samples may also be analyzed by characterizing one or more solid components of a blood sample. Solid components of a blood sample may include, but are not limited to, blood cells, platelets, or pathogenic organisms (*e.g.*, bacteria, viruses, fungi, or blood-borne parasites).

In some embodiments, the cellular components of a sample may be characterized by detecting the presence and/or expression levels of one more molecular groups (*e.g.*, polypeptides, polynucleotides, carbohydrates, lipids) typically known to be associated or correlated with a specific trait for which the test is being performed. For example, a blood sample may be collected to measure the number of one or more specific cell types present in the sample (commonly referred to in the art as "cell counts"), and/or the ratio thereof with respect to one or more different cells types also present in the sample. Examples of the types of blood cells that may be detected in a blood sample include, but are not limited to, erythrocytes, lymphocytes (*e.g.*, T cells and B cells), Natural Killer (NK)-cells, monocytes/macrophages, megakaryocytes, platelets, eosinophils, neutrophils, basophils or mast cells. In some embodiments, various sub-populations of specific cell types within a fluid sample are distinguished. For example, the T cells present in a blood sample may be further categorized into helper (CD4+), cytotoxic (CD8+), memory (CD4/CD8 and/or CD45RO) or suppressor/regulatory (CD4+CD25+FOXP3+) T cells. Alternatively, B cells present in a blood sample may be further categorized into populations of immature, mature, activated, memory, or plasma cells, based on the immunoglobulin isotype expressed on the cell surface, and presence or absence of various additional proteins.

Table I summarizes the surface expression profile of a selection of non-limiting protein markers that may be used to classify the stage of B cell differentiation, where filled circles denote expression, open circles denote lack of expression, and partially filled circles denote partial or limited expression of the indicated surface marker. The presently described systems and methods are not limited to detecting the cell types disclosed in Table 1. It should be understood, that the presently disclosed systems and methods may be suitably adapted to analyze most cell types and/or macromolecules present in a biological sample without departing from the spirit and scope of the presently described embodiments.

**Table I.**

| B cell stage | Surface Immunoglobulin isotype | Marker protein | | | | | | |
|---|---|---|---|---|---|---|---|---|
| | IgM | IgG or IgA | IgD | CD23 | PCA-1 | CD38 | CD25 | CD10 |
| Pre B | ○ | ○ | ○ | ○ | ○ | ● | ○ | ○ |
| Immature | ● | ○ | ○ | ○ | ○ | ○ | ○ | ○ |
| Mature | ● | ○ | ● | ● | ○ | ○ | ● | ○ |
| Activated | ● | ● | ○ | ● | ○ | ○ | ● | ● |

(continued)

| B cell stage | Surface Immunoglobulin isotype | Marker protein | | | | | | | |
|---|---|---|---|---|---|---|---|---|---|
| | IgM | IgG or IgA | IgD | CD23 | PCA-1 | CD38 | CD25 | CD10 |
| Memory | ○ | ● | ○ | ○ | ○ | ○ | ○ | ○ |
| Plasma cell | ○ | ○ | ○ | ○ | ● | ● | ○ | ○ |

Analysis of a cellular composition of a sample may include detecting the presence of one or more "surface markers" known to be expressed on the surface of the population of cells of interest. Certain surface markers useful in the differential identification of cells in a sample (*e.g.*, in particular cells involved in immune responses) and/or diseases are commonly referred to as "cluster of differentiation (CD)" antigens or CD markers, of which over 250 have been characterized. Many of the CD antigens may also be referred to by one or more alternative art-recognized terms. Table II lists several examples of CD antigens, and the cells in which they are expressed, that may be referred to using one or more alternative terms. The system of CD marker nomenclature is widely recognized by ordinary practitioners of the art. General guidance in the system of CD marker nomenclature, and the CD expression profiles of various cells may be found in most general immunology reference textbooks such as, for example, in IMMUNOLOGY, 4th Edition Ed. Roitt, Brostoff and Male chapter 28 and Appendix II (Mosby/Times Mirror International Publication 1998), or in IMMUNOBIOLOGY: THE IMMUNE SYSTEM HEALTH AND DISEASE, 5th Edition, Eds. Janeway et al. Appendices I-IV (Garland Publishing, Inc. 2001).

**Table II.**

| CD Antigen | Identity/function | Expression |
|---|---|---|
| CD3 | T cell receptor ($\gamma,\delta,\varepsilon,\xi,\eta$,) | Thymocytes, T cells |
| CD4 | MHC class II receptor | Thymocyte subsets, T helper cells, monocytes, macrophages |
| CD8 | MHC class I receptor | Thymocytes subsets, cytotoxic T cells |
| CD10 | Neutral endopeptidase/CAALA | T and B-cell precursors, activated B cells, granulocytes |
| CD11a | Integrin $\alpha$ | Lymphocytes, granulocytes, monocytes and macrophages |
| CD11b | Integrin $\alpha$ | Myeloid and NK cells |
| CD13 | Aminopeptidase N | Monocytes, granulocytes |
| CD16 | Fc$\gamma$RIIIA/B | Neutrophils, NK cells, macrophages |
| CD19 | B cell function/activation | B-cells |
| CD20 | $Ca^{2+}$ ion channel | B-cells |
| CD21 | C3d and EBV receptor | Mature B cells |
| CD35 | Complement receptor 1 | Erythrocytes, B cells, monocytes, neutrophils, eosinophils |
| CD41 | $\alpha$IIb integrin | Platelets, megakaryocytes |
| CD45RO | Fibronectin type II | T-cell subsets, B-cell subsets, monocytes, macrophages |
| CD45RA | Fibronectin type II | B cells, T-cell subsets (naive T cells), monocytes |
| CD45RB | Fibronectin type II | T-cell subsets, B cells, monocytes, macrophages, granulocytes |
| CD56 | NKH-1 | NK cells |

In some embodiments, the presently described analyte-detection systems and methods may be used to analyze blood samples on the basis of the expression profile or presence of one or more macromolecules (*e.g.*, proteins, phosphoproteins, glycoproteins, polynucleotides, or variants or isoforms thereof) that are indicative or prognostic of certain pathological states. Types of analytes that may be useful diagnostic or prognostic indicators and whose plasma or cellular expression levels are correlated with various diseases, illnesses, viruses, and/or syndromes include, but are not limited to, chemokine receptor 5 (CCR5), viral DNA or RNA sequences, certain species of plasma RNA, interferon-gamma (IFN-$\gamma$), virus particles, early secreted antigenic target protein-6 (ESAT-6), culture filtered protein-10 (CFP-10), C-reactive protein (CRP), troponin-I, and TNF-$\alpha$.

In some embodiments, an analyte-detection system may be used for prognostic tests for HIV seropositive patients. HIV infects CD4$^+$ cells (*e.g.*, certain populations of T helper cells, monocytes and macrophages) by binding to a co-receptor CCR5. The expression level of certain CCR5 variants in CD4$^+$ cells has been shown to correlate with viral load and progression to AIDS. The presently described analyte-detection systems and methods may be used to, for example, monitor CCR5 expression in CD4$^+$ cells in patient blood samples. This parameter may advantageously be measured simultaneously from a single sample with one or more measures of HIV viral load. In some embodiments, the tests described herein may further measure one or more blood parameters associated with other pathological situations in addition to, or alternatively to, HIV infection.

In certain embodiments, an analyte-detection system may be used to diagnose tuberculosis (TB). In some embodiments, an analyte-detection system may be used to detect reductions in systemic CD3$^+$ and CD4$^+$ cells that typically occur in TB patients. This parameter may be measured alone or in combination with the detection of one or more soluble proteins typically elevated in TB patients (such as IFN-$\gamma$), the mycobacterial proteins ESAT-6, CFP-10, or T cells populations that are reactive to ESAT-6 and CFP-10. Such applications may be particularly suited to certain point-of-care settings and/or in resource scarce countries where HIV and TB comorbidity are common.

In some embodiments, an analyte-detection system as described herein may be used to diagnose viral infections in addition to HIV. Blood samples from both Epstein-Barr virus (EBV) and cytomegalovirus (CMV) infected patients exhibit increases in percentages of total T-cells, suppressor T-cells and activated HLA-DR$^+$ T-cells when compared with healthy, uninfected people. Additionally, as seen in HIV infected patients, individuals infected with EBV and/or CMV typically display significantly decreased levels CD4$^+$ T-cells as well as a decrease in the ratio of CD4/CD8 T cells. Blood samples from individuals infected with EBV may also exhibit elevated levels of NK cells.

The analyte-detection systems described herein may, in some embodiments, be adapted to readily, reproducibly, and cost effectively diagnose a variety of maladies endemic to geographic and/or economically disadvantaged regions. An example of such an application is point-of-care diagnosis of malaria in geographic areas such as, for example, Africa, Latin America, the Middle East, South and Southeast Asia, and China. Currently, reliable diagnosis of malaria is time consuming, labor intensive, and typically involves identifying erythrocytes harboring *Plasmodium* parasites. Identification of such cells is typically made by microscopic examination of uncoagulated Giemsa-stained blood samples, possibly in combination with one or more serological and/or molecular diagnostic tests (*e.g.*, polymerase chain reaction), all of which require highly specialized equipment. In some embodiments, analyte-detection systems described herein may be sued to detect one or more *Plasmodium*-specific antigens that include, but are not limited to, panmalarial antigen (PMA), histidine-rich protein 2 (HRP2) and parasite lactate dehydrogenase (pLDH) in a blood sample. In some embodiments, the analyte-detection systems presently described may be used to monitor one or more physiological parameters associated with malaria. For example, a portion of the hemoglobin from *Plasmodium*-parasitized erythrocytes forms lipidized pigment granules generally referred to as "hemozoin." Phagocytosed hemozoin impairs monocyte/macrophage and hence immune function, at least in part, by reducing the surface expression of MCH class II, CD11c and CD54 in phagocytes. Additionally, low peripheral blood monocyte counts may be associated with patients with severe and complicated malaria. Analyte-detection systems described herein may be used to detect and monitor the presence and/or quantities of these physiological parameters associated with malaria.

In some embodiments, analyte-detection systems described herein may be used to diagnose Good's syndrome, an immunodeficiency disorder secondary to thymoma and characterized by deficiencies of cell-mediated immunity and T-cell lymphopenia.

In some embodiments, an analyte-detection system may be used to identify certain biological markers associated with increased susceptibility to various pathological conditions (*e.g.*, cardiovascular disease, atherosclerosis, inflammation, and/or certain types of cancer). Inflammation has been identified as an underlying cause of atherosclerosis, a condition associated with the deposition of lipids on the lining of arteries that may progressively lead to serious vascular complications such as myocardial infarction (MI) and/or stroke. By measuring the concentration of certain proteins associated with inflammation (*e.g.*, CRP) either alone or in conjunction with cellular profiles (*e.g.*, WBC count), the presently described analyte-detection systems may be used to screen individuals at risk for heart attack, atherosclerosis, or other vascular diseases. Likewise, MI patients with elevated CRP levels or WBC counts are at higher risk for subsequent cardiovascular events. Diagnostic and prognostic tests that provide measurements for these two important biological parameters associated with inflammation and vascular disease may provide powerful diagnostic and prognostic insight, allowing healthcare providers to make timely and appropriate therapeutic interventions. For example, it is recognized by practitioners of the art that individuals having elevated WBC counts and blood CRP levels have a greater risk for heart disease than individuals having WBC counts and CRP levels within normal range.

A low peripheral monocyte count in individuals with high cholesterol is generally predictive of increase risk for developing atherosclerosis. The presently described analyte-detection systems may be readily and advantageously adapted to measure monocyte counts (CD13$^+$CD14$^+$CD45RA) associated with cardiac risk factors. Monocyte counts are also an important physiological parameter in subjects with hypercholesterolemia. Analyte-detection systems described herein may also be used to measure the amounts of other cardiac risk factors such as troponin I and/or TNF-$\alpha$.

A percentage of CD8$^+$ cells and a number of monocytes in blood have been associated with progressive encephalopathy (PE). PE is one of the most common complications of HIV infection in children. As antiretroviral drugs become more available, the number of children with PE has increased, thus it is desired to evaluate risk factors for PE. CD8 stained cells may be identified using an analyte-detection system to monitor the progress of PE.

An analyte-detection system for use in diagnostic and prognostic applications to specific pathologies, such as for example, those described above, may further allow a user of the system to readily identify characteristics in a sample that are associated with the malady. The analyte-detection system may include, for example, various receptor molecules (such as specific antibodies) that bind to cell surface markers (*e.g.*, CD markers or other disease-associated molecules) or any other analyte suspected to be present in a sample that allows rapid characterization of the sample. In some embodiments, one or more antibodies (*e.g.*, monoclonal and/or polyclonal antibodies) that specifically recognize and bind to macromolecules expressed on the surface of cells (*e.g.*, CD or other cell surface markers) may be used in an analyte-detection system.

While certain specific examples of monoclonal or polyclonal antibodies are set forth above, it will be readily understood by ordinary practitioners of the art that the presently described analyte-detection systems may be used, without limitation, in conjunction with any type of antibody that recognizes any antigen, including, but not limited to, commercially available antibodies or antibodies generated specifically for the purpose of performing the tests described herein. Monoclonal and Polyclonal antibody design, production and characterization are well-developed arts, and the methods used therein are widely known to ordinary practitioners of the art (see, *e.g.*, "Antibodies: A Laboratory Manual," E. Howell and D. Lane, Cold Spring Harbor Laboratory, 1988). For example, a polyclonal antibody is prepared by immunizing an animal with an immunologically active composition including at least a portion of the macromolecule to which the desired antibody will be raised and collecting antiserum from that immunized animal. A wide range of animal species may be used for the production of antiserum. Examples of animals used for production of polyclonal anti-sera are rabbits, mice, rats, hamsters, horses, chickens, or guinea pigs.

A monoclonal antibody specific for a particular macromolecule can be readily prepared through use of well-known techniques such as those exemplified in U.S. Patent No. 4,196,265, to Koprowski et al. Typically, the technique involves first immunizing a suitable animal with a selected antigen (*e.g.*, at least a portion of the macromolecule against which the desired antibody is to be raised) in a manner sufficient to provide an immune response. Rodents such as mice and rats are preferred species for the generation of monoclonal antibodies. An appropriate time after the animal is immunized, spleen cells from the animal are harvested and fused, in culture, with an immortalized myeloma cell line.

The fused spleen/myeloma cells (referred to as "hybridomas") are cultured in a selective culture medium that preferentially allows the survival of fused splenocytes. After the fused cells are separated from the mixture of non-fused parental cells, populations of B cell hybridomas are cultured by serial dilution into single-clones in microtiter plates, followed by testing the individual clonal supernatants for reactivity with the immunogen. The selected clones may then be propagated indefinitely to provide the monoclonal antibody of interest. In some embodiments, a membrane-based detection system for use in performing WBC counts on a blood sample may use one or more polyclonal or monoclonal antibodies that specifically recognize various cell types that constitute WBCs to visualize specific blood cells. Antibodies suitable for this purpose include, but are not limited to: anti-CD3; anti-CD4; anti-CD8; anti-CD16; anti-CD56; and/or anti-CD19 antibodies to specifically recognize: T cells; T helper cells and monocytes/macrophages; cytotoxic T cells; neutrophils, NK cells and macrophages; NK cells; and B cells, respectively.

In some embodiments, a membrane-based detection system is used to assess both CD4 cell count and CD4 cells as a percentage of total lymphocytes from a blood sample for diagnosis, staging, and/or monitoring of infections and/or diseases. For example, samples having CD4 counts below 200 cells per microliter may indicate specific drug therapy intervention. In certain embodiments, comparing CD4 cell counts to CD8, CD3, and/or CD19 cell counts may be used to assess the ratio CD4+ T helper cells with respect to cytotoxic T cells, total circulating T cells, B cells, or combinations thereof.

In some embodiments, a sample, such as blood or diluted blood, is applied and/or transported to a membrane of a membrane-based detection system. The membrane may retain portions of the sample, while allowing other portions of the sample to pass through. For example, the membrane may be adapted to retain lymphocytes, while allowing other portions of the sample, such as water or red blood cells, to pass through.

A combination of visualization agents may be applied and/or transported to the membrane to allow a total number and/or different types of lymphocytes (*e.g.*, T cells, NK-cells, and/or B-cells) to be identified. One or more visualization agents may be added to the matter collected on a surface of the detection system. For example, visualization agents may allow the detection of anti-CD3, anti-CD4, anti-CD8, anti-CD16, anti-CD56 and anti-CD19 antibodies bound to their respective CD markers on the surface of target cells. In some embodiments, anti-CD2, anti-CD4, and anti-CD19 antibodies may be coupled to the visualization agent directly. In some embodiments, the visualization agent may be coupled to a second macromolecule that specifically binds to and recognizes the antibody bound to the CD marker.

In some embodiments, a first visualization agent may be used to stain CD4+ cells present in a mixed population of cells. Additional, distinct visualization agents may then be used to stain the NK-cells, B-cells, and/or other T-cells in the mixed population. For example, a mixed population of cells in a sample may be stained with anti-CD4, anti-CD3, anti-CD56, and anti-CD19 antibodies to detect CD4+ T helper cells, total T-cells, NK-cells, and B-cells respectively.

In some embodiments, fluorescent dyes (e.g., AlexaFluor® dyes from Invitrogen Corporation; Carlsbad, CA) may be coupled to antibodies to form fluorophore-labeled antibodies. Use of fluorophore-labeled antibodies to visualize cells may facilitate assessment of the sample. One or more fluorescent dyes may be used to label one or more cell surface markers to facilitate assessment of a desired marker percentage relative to other markers (e.g., a percentage of CD4+ lymphocytes relative to other lymphocytes). An image of the cells stained by the first visualization agent may be provided and one or more additional images of cells stained by the additional visualization agents may be provided. The images may be compared and/or combined to determine the total number of lymphocytes and/or a number of a specific type of lymphocyte in or on the membrane. A detector optically coupled to at least a portion of the membrane may provide the images. An analyzer may automatically compare the images during use. For example, AlexaFluor® 488, which fluoresces green when exposed to light having a wavelength of 488 nm, may be used to visualize anti-CD3 antibodies bound to the surface of all T cells present in a sample. AlexaFluor® 647, which fluoresces red when exposed to light having a wavelength of 647 nm, may be used to visualize anti-CD4 bound to the surface of T helper cells and monocytes. In this way, at least three populations of cells (all T cells stain red, T helper cells stain red and green, the overlap of which shows as yellow, and monocytes which stain green) may be readily and simultaneously identified in a single sample.

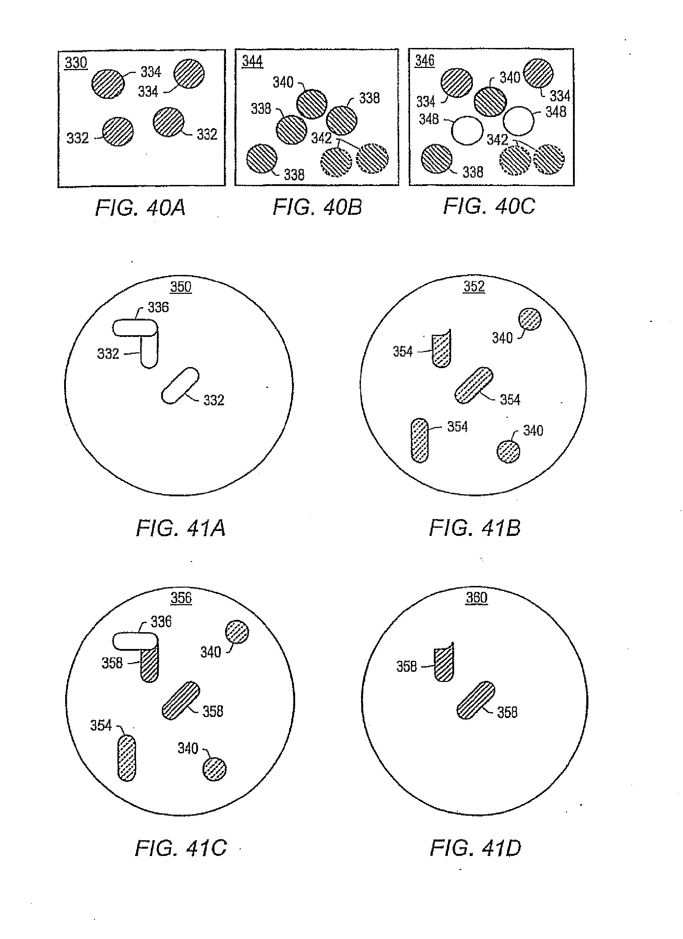

In some embodiments, two fluorophores and two light sources are used to determine types of lymphocytes. The analyte-detection system depicted in FIGS. 36-39 may be used, for example, to determine type of lymphocytes. FIGS. 40A-40C depict representations of images collected using two fluorophores and two light sources. For example, a green fluorophore (e.g., AlexaFluor® 488) may be coupled to anti-CD4 antibodies of a sample. A red fluorophore (e.g., AlexaFluor® 647) may be coupled to the anti-CD56 antibodies, anti-CD3 antibodies, and anti-CD19 antibodies added to the sample. As discussed above and shown in Tables I and II, CD4 is expressed on the surface of T helper cells and monocytes, CD19 is expressed on the surface of B cells, CD56 is expressed on the surface of NK cells, and CD3 is expressed on T cells. Analysis of the samples captured on a membrane using two wavelengths of light may allow differentiation of the types of WBCs captured.

FIG. 40A depicts a representation of image 330 of green cells 332, 334 obtained by exciting the green fluorophore visualization agent with a light source, analyzing the signal generated by the excitation, and producing an image of the cells. Green cells 332, 334 represent CD4+ cells.

FIG. 40B depicts a representation of an image of red cells obtained by exciting the red fluorophore, analyzing the signal produced from excitation, and producing an image of red cells. Red cells 338, 340, and 342, visible in image 344, represent cells expressing CD3, CD19 and CD56 respectively.

In digital detector images, cells that exhibit both green and red light may be combined to emit yellow light. Thus, monocytes (e.g., cells that only emit green light) may be identified and isolated. Combining image 330 and image 344 creates image 346 that includes green cells 334, red cells 338, 340, 342, and yellow cells 348, as shown in FIG. 40C. Green cells 334 are representative of CD4+CD3-CD19-. Yellow cells 348 are representative of CD4+CD3+ T helper cells.

A total number of T-helper cells (cells that express CD4 and CD3 and stain yellow), a total number of lymphocytes (cells that express CD3, CD19 or CD56 and stain red), a total number of CD4 cells (cells that stain green), and a ratio of CD4 cells to a total number of lymphocytes may all be determined from the combination of images 330, 344, 346. A total number of lymphocytes may be obtained from the combined image, as depicted in image 346, since the cells may be identified and isolated (e.g., cells that only emit green light or only emit red light).

An absolute number of CD4+ T helper cells is the total number of yellow cells 348. A ratio of CD4+ T helper cells to the total number of cells may be calculated by dividing the total number of yellow cells 348 (CD4+CD3+) by red cells 338, 340, 342 (CD3+, CD16+, CD456+, or CD19+).

The ratio of T-helper cells to total lymphocytes may be important in determining the progression of diseases, such as HIV, and in the treatment and monitoring of other diseases. Although green and red fluorophores were described, fluorophores of any color may be used without limitation.

In some embodiments, use of one or more visualization agents allows identification of lymphocytes retained on a membrane of a membrane-based detection system. The lymphocytes may contain cell surface markers CD4, CD3, and CD19. Identification of CD4 and CD3 and on the surface of cells identifies T-helper cells. FIGS. 41A through 41D represent images of cells expressing CD4, CD3, and CD19 markers in the presence of two excitation sources.

FIG. 41A depicts an image of cells obtained by excitation of a green fluorophore attached to cells expressing CD4. An excitation source may excite green fluorophores and a detector may analyze the signal produced during excitation and produce image 350 of green cells 332, 336.

FIG. 41B depicts an image of cells obtained by excitation of a red fluorophore attached to cells expressing CD3 or CD19. An excitation source excites red fluorophores bound to the cells and a detector analyzes the signal produced during excitation and produces image 352 of cells 340 containing CD19 and cells 354 containing CD3.

Image 350 may be combined with image 352 to produce image 356 in which green cells 336, red cells 354, 340 and yellow cells 358 are visible. The total number of lymphocytes may be obtained from the combined image of cells stained red, green or yellow, as depicted in FIG. 41C. The total number of T helper cells present on the membrane is identifiable by determining the number of cells that stain yellow (*e.g.*, those cells expressing both CD3 and CD4.

In some embodiments, a filter allows a desired wavelength of light to pass from the detection system to the detector. For example, a filter only allows yellow light to pass, as depicted in FIG. 41D. Thus, T cells 358 may be identified from image 360 collected by the detector. Using a filter may facilitate identification of one or more types of lymphocytes and/or other types of matter.

While a system to identify T cell populations based on differential staining of CD3, CD4, and CD19 markers on cells is described above, it is understood that any combination of CD markers may be used to identify one or more types of lymphocytes and/or total lymphocytes in a sample.

In some embodiments, all cells except a lymphocyte of interest may be stained. A white light image of the membrane may be provided. One or more additional images may be provided in which cells stained with one or more visualization agents are visible. The number of a specific lymphocyte population may be obtained by assessing the number of cells appearing in the first image (*e.g.*, the white light image) but not appearing in the additional images (*e.g.*, images in which only stained cells appear). For example, a sample containing lymphocytes may be retained on a membrane of an analyte-detection system. A first image at a selected wavelength of light of the retained cells is taken. One or more visualization agents may be applied to the retained cells. At least one of the visualization agents stains part of the retained cells, but does not stain CD4+ cells. A second image at one or more wavelengths different than the wavelength for the first image is taken. Such "negative selection" strategies may be employed to determine the number of cells that are depicted in the first image but are not depicted in the second image, to give the number of CD4+ lymphocytes. Such strategies may be particularly suited to applications where additional functional analyses are performed on the cell of interest. For example, it is known in the art that contacting certain CD markers (*e.g.*, CD3, CD19) with certain antibodies (commonly referred to as "cross-linking antibodies") causes profound changes in cellular physiology. Therefore, the negative selection strategy outlined above may be useful when additional biological/functional analyses are to be performed on a particular cell type.

In some embodiments, cells expressing CD4 may be stained red and cells expressing CD45 may be stained green. In certain embodiments, cells with certain surface markers may stain brighter than cells without the surface markers. For example, stained CD45 cells may appear brighter than stained CD4+ cells. A percentage of CD4 to total lymphocytes may be determined from the ratio of CD4+ cells to brighter stained CD45 cells.

It may be desirable to stain various cell subtypes differentially to allow discrimination between various cell types even when the cells are stained with antibodies with the same color tag. For example, CD4+ monocyte population may be differentiated from the CD4+ lymphocyte population. Low and high intensity CD4+ cells may be extracted from images of the detection system obtained by a detector. Weakly stained CD4+ cells may then be stained with a CD14 stain that identifies weakly stained CD4+ cells as monocytes.

Similar principles may be applied to other subsets of the lymphocyte population. A difference in the staining of NK-cells, B cells, and T cells due to the number of surface markers, antibody affinity, or antibody performance may identify a CD8 population. CD8 monitoring and/or a ratio of CD4 to CD8 cells may be important in providing information about the progression of certain diseases, such as, for example, HIV progression and AIDS.

It may be desirable to obtain a CD8 percentage and monocyte count from a sample. Monocytes may exhibit a weaker stain with CD4 antibodies, which allows monocytes to be distinguished from CD4 T-cells, which are characterized by a strong stain with CD antibodies.

Differences in surface marker concentrations on cells may provide a tool for discrimination between cells. In some diseases, cell morphology may be correlated with disease states. Images from assay screening may provide information about the assay and cell morphology and may provide additional information about the disease. For example, the malaria antibody may be localized on a part of the cell to allow a difference in intensity across a cell to be observed. This difference in intensity may provide information about the health of the patient.

Different subpopulations of cells may accept the same stain but emit light at different intensities and so the subpopulations may be differentiated. The antibody binding capacity for various surface antigens may be measured using methods generally known to ordinary practitioners of the art. For example, CD4+ T-cells bind about 50,000 antibody molecules. Protocols for assay development and image analysis can be defined based on the relative amount of antibodies molecules that various cells can bind. Often exposure times may be adjusted to further separate populations. For example, a total T-cell population may be identified with an anti-CD3 antibody. Even though CD3 cells are stained with the same color as NK-cells and B-cells, the populations can be determined based on the differential staining characterizing these cells. As the CD3 population becomes separated from the rest of the cell count (*e.g.*, by increasing exposure time when taking the image), the percentage of CD8 cells may be determined by subtracting the number of CD4+ cells and CD3+ cells from the total CD3 cell count. In some embodiments, when cells are stained with anti-CD8 antibody, there exists a strong intensity differential to discriminate CD8 cells from other cells such as NK-cells and B-cells. The strong intensity may accentuate the differential seen in a single color containing CD8$^+$ cytotoxic T cells, NK-cells, and B-cells. A ratio of CD8$^+$ cells may be calculated by dividing the total number of CD3$^+$ cells minus the total number of CD4$^+$ cells and CD3$^+$ cells by the total number of CD3$^+$ cells.

An analyte-detection kit including at least one cartridge designed for performing a pre-determined analysis, a sample collection device and disinfectant wipes may be opened. In some embodiments, the cartridge, wipes, sample collection devices are individually obtained. In certain embodiments, the cartridge is checked for viability prior to use. In some embodiments, a portion of a human may be wiped with one of the disinfectant wipes and a blood sample may be obtained with the sample collection device. A portion of the collected sample may be deposited on or in a collection region of the cartridge. For example, a finger may be pricked with a lancet and a drop of blood transferred to the cartridge using disposable tubing, a pipette, or a fluid bulb. In some embodiments, the sample may be deposited directly onto a membrane of a membrane-detection system. After the sample is introduced into a collection region of a cartridge, the collection region may be capped or sealed with, for example, an adhesive strip, a rubber plug, or a cover.

In some embodiments, one or more reagents may be provided to the sample. For example, anti-coagulant and/or fixative may be added to the blood sample. Fixatives include, but are not limited to, paraformaldehyde, ethanol, sodium azide, colchicine, Cyto-Chex® (Streck, Inc., Omaha, NE), and Cyto-Chex® BCT. In some embodiments, a reagent may be provided to the sample. The reagent may be mixed with the sample during or after collection of the sample. Alternatively, a reagent may be added to a sample after the sample is introduced into a cartridge. In certain embodiments, a reagent may be provided to the sample by, for example, one or more pumps, fluid packages, and/or reagent regions coupled to, positioned in, and/or positioned on a cartridge.

The cartridge may be positioned, automatically or manually, in a housing of the analyte-detection system. The cartridge may substantially contain all fluids used for the analysis.

In some embodiments, a check of the cartridge may be performed. For example, the cartridge includes one or more particles having the desired analyte to be determined. An image of the particles may be obtained by one of the detectors. Analysis of the image is performed to determine if the known analyte can be detected. If the known analyte is detected, the cartridge is deemed suitable for use. If the known analyte is not detected, the cartridge may be disposed of and a new cartridge obtained. In some embodiments, the new cartridge is obtained from the kit or a supply of cartridges.

At least a portion of the sample may be provided to a metered volume portion of the cartridge. In some embodiments, the sample may be drawn by capillary action into the metered volume portion. In certain embodiments, the sample may be delivered by a fluid delivery system disposed in or coupled to the cartridge. After the sample has filled the metered volume portion, a portion of the sample may travel toward an overflow reservoir. In some embodiments, the sample may not be measured.

A fluid delivery system that includes a reagent may be actuated. Flow of fluid from the fluid delivery system may push a metered volume of sample from the metered volume portion towards a detection region that includes one or more detection systems (*e.g.*, a particle-based detection system and/or a membrane-based detection system). The reagent and sample may combine during passage of the sample toward the one or more detection regions to form a sample/reagent mixture. A portion of the sample/reagent mixture flows through or is collected in the detection region. The remaining portion of sample/reagent mixture may flow over or through the detection region to a waste region of the cartridge.

In some embodiments, the fluid delivery system is not necessary to push the sample towards the detection region. Capillary forces may transport the sample towards the detection region. In some embodiments, capillary forces that transport the sample are enhanced with hydrophilic materials (*e.g.*, plastic or glass) to coat a channel for aqueous samples. Certain portion of channels may include hydrophilic materials positioned proximate the collection region, in the metered volume chamber, and/or proximate the overflow reservoir to direct flow of aqueous samples through a cartridge.

In some embodiments, the sample may be drawn into a channel via negative pressure in the channel. For example, suction created by a passive valve or a negative pressure source may create negative pressure in a portion of a channel and draw fluids towards the detection region. In some embodiments, valves may be used to direct the flow of fluid and/or sample through the cartridge.

One or more additional fluid delivery systems may be actuated to release one or more additional fluids (*e.g.*, additional PBS, water, or other buffers). One or more of the additional fluids may flow over or through one or more reagent regions (*e.g.*, a reagent pad or through a channel containing reagents). One or more reagents (*e.g.*, one or more antibodies or a visualization agent) in or on the reagent regions may be reconstituted by the additional fluids. The reconstituted reagents may be transported to the detection region of the cartridge. Transport of the reconstituted reagents may be accomplished by continued actuation of the fluid delivery systems or through other methods described herein. The reconstituted reagents may label and wash a portion of the sample collected in one or more detection regions of the cartridge (*e.g.*, wash WBCs retained on a membrane).

Portions of a sample and/or fluids may be provided to a detection region in a cartridge sequentially, successively, or substantially simultaneously. In some embodiments, a portion of the sample moves towards a detection region as a portion of the fluid from the second fluid delivery system flows towards a reagent region. Fluid from the second fluid delivery system may reconstitute and/or collect one or more reagents from the reagent region and deliver the reagents to the detection region after the sample has passed through the detection region. The collected reagents may then be added to an analytes that have been collected by the detection region.

Valves (*e.g.*, pinch valves) and/or vents may be use to regulate flow of the sample. For example, a valve proximate the collection region may inhibit additional sample from flowing towards the detection region. In some embodiments, one or more changes in elevation of a channel may inhibit the sample form entering other channels.

In some embodiments, a reagent (e.g., a visualization agent or one or more antibodies) may be directly added to the matter on a membrane of a membrane-based detection system. The sample may then be washed with fluid remaining in the first fluid delivery system or with the fluid from one or more of the fluid delivery systems.

In some embodiments, only one fluid delivery system is used. For example, one or more syringes may be at least partially coupled to, positioned in, or positioned on the cartridge. Each syringe may contain one or more fluids to be used during the analysis. The syringes may be actuated and the fluids delivered sequentially, successively, or substantially simultaneously to the collection region, the reagent regions and/or the detection region.

In some embodiments, analytes collected on a membrane of a membrane-detection system may be viewed through a viewing chamber of the membrane-detection system. Light sources may be activated and light may be directed towards the membrane-based detection system. Light may enter the membrane-detection system through a viewing chamber and/or a top layer of the membrane-detection system. A detector may collect a signal produced from interaction of light with one or more analytes in the detection region. In some embodiments, the detector may be optically aligned with the viewing chamber of the membrane to allow the membrane and/or detection region to be viewed by detector.

The detector processes the produced signal to produce images representative of the analytes collected by the detection system. Images may be obtained concurrently or simultaneously. Images may be analyzed and the analytes in the sample assessed.

The cartridge may then be removed from the analyzer and discarded. The above-described method may then be repeated for the next sample. In certain embodiments, portions of the analyzer may be disinfected between samples. In some embodiments, the cartridge is self-contained such that all fluids remain in the cartridge and the analyzer may not need to be disinfected.

Interaction of a sample with light produces a signal that is received by the detector. The detector may produce images from the signal. Images may be analyzed by an analyzer (*e.g.*, automatically with a computer or manually by a human) to determine the analytes present in the sample.

A third fluid delivery system may be activated to allow a wash solution to flow through or over the detection region. The detection region may be washed repeatedly to clear the detection region and prepare for additional use.

The first fluid delivery system may be actuated, or a fourth fluid delivery system may be used, to push a second portion of sample towards the membrane. The analysis may be repeated to determine different and/or duplicate sample analysis.

The procedure may be repeated as necessary to obtain the needed data. Additional samples may also be obtained and used. In some embodiments, one or more membranes may be used in a membrane-based detection system. After all analyses have been completed, the cartridge may be properly discarded.

In some embodiments, an analyte-detection system may be used to test for two or more analytes. The first and second analytes may include a wide range of cellular and/or chemical/biochemical components. Chemical/biochemical components may include, but are not limited to, electrolytes, proteins, nucleic acids (*e.g.*, DNA and/or RNA), steroids and other drugs. In certain embodiments, an analyte-detection system may be designed to test for indications of cancer (*e.g.*, types of cancerous cells and/or levels of related biochemicals) as well as one or more diseases. For example, an analyte-detection system may be designed to test for cervical cancer and sexually transmitted diseases.

In some embodiments, one or more cellular components of blood and/or one or more proteins may be assessed concurrently in an analyte-detection system including particle- and/or membrane-based detection systems coupled to one or more fluid flow systems. The proteins may include protein cardiac biomarkers. Protein cardiac biomarker targets may include, but are not limited to, proteins related to risk assessment, prognosis, and/or diagnosis. Protein cardiac biomarker targets related to necrosis, thrombosis, plaque rupture, endothelial dysfunction, inflammation, neurohormone activation, ischemia, arrhythmias, and/or other conditions may be assessed. Protein cardiac biomarker targets assessed by particle-based detection systems may include, but are not limited to, cardiac troponin T (cTNT), cardiac troponin I (cTNI), myoglobin (MYO), fatty acid binding protein (FABP), myeloperoxidase (MPO), plasminogen activator inhibitor-1 (PAI-1), tissue factor, soluble CD40 ligand (sCD40L), von Willebrand factor (vWF), D-dimer, matrix metalloproteins (MMPs), pregnancy associated plasma protein (PAPP), placental growth factor (PIGF), soluble intercellular adhesion molecules (sICAM), P-selectin, CRP, high sensitivity C-reactive protein (hs-CRP), oxidized low-density lipoprotein (ox-LDL), monocyte chemotactic protein-1 (MCP-1), interleukin-18 (IL-18), IL-6, TNF-$\alpha$, B-type natriuretic peptide (BNP), norepinephrine (NE), ischemia modified albumin (IMA), free fatty acids (uFFA), and combinations thereof.

The cellular components may include cellular cardiac biomarkers. Cellular cardiac biomarkers may include, but are not limited to, white blood cells, circulating endothelial cells, platelets, and/or combinations or subsets thereof. In some embodiments, for example, a white blood cell subset may include lymphocytes. Identification of ESAT-6 and CFP-10 specific T-cells may be desirable. ESAT-6 and CFP-10 may be tagged with a fluorophore and passed through a membrane of a detection system where they bind with T-cells. In certain embodiments, fluid is directed to a particle-based detection system after passage through the membrane, where the particle-based detection system includes a particle derivatized with anti-IFNy.

Tests targeting CRP and WBCs are widely available in clinical settings; they are typically administered separately on different instruments. These tests may require large sample volumes, additional sample preparation steps, and longer assay times. In addition, the clinical instruments and methodologies currently used to complete these tests are not suitable for point of care testing, such as in the doctor's office, in an emergency room, or in an ambulance. The diagnostic and prognostic value of these biomarkers may be enhanced if these two tests could be administered concurrently on the same instrument, in a convenient, accurate and highly accessible manner.

In some embodiments, an analyte-detection system is used to analyze two or more analytes in a fluid and/or sample. A first analyte may be cellular matter and a second analyte may be a one or more protein components. For example, the first analyte may be WBCs and the second analyte may be CRP. A sample (*e.g.*, whole blood) may be obtained using the methods described herein or other sampling techniques known in the art. A portion of the sample may be provided to a collection region of a multi-functional cartridge.

At least a portion of the sample may be provided to a metered volume portion of the cartridge. In some embodiments, the sample may be drawn by capillary action into the metered volume portion. In certain embodiments, the sample may be delivered to a metered volume portion using a fluid delivery system. As the sample fills the metered volume portion, an excess portion of the sample may travel toward an overflow reservoir. The metered portion of the sample may be advanced toward one or more regions including, but not limited to, a particle-based detection system, a membrane-based detection system, a cell-lysing chamber, a processing chamber, a polymerase chain reaction chamber, or combinations of these regions. In some embodiments, a metered volume portion of the cartridge may not be necessary.

Portions of the sample may be provided to detection systems in the cartridge sequentially, successively, or substantially simultaneously through pathways (*e.g.*, channels) described previously. In some embodiments, a portion of the sample may be provided to a membrane-based detection system, passed through the membrane-based detection system, and the remaining sample is provided to a particle-based detection system. In some embodiments, a portion of the sample may be provided to a particle-based detection system before a portion of the sample is provided to a membrane-based detection system. In certain embodiments, portions of the sample may be provided to a particle-based detection system and a membrane-based detection system via separate pathways (*e.g.* channels) substantially simultaneously. In some embodiments, a sample from a single collection region may be provided to two or more pathways. In certain embodiments, samples may be provided to two or more collections regions and processed independently. After the collection region is filled, the collection region may be capped or sealed with a cover. At least a portion of the sample may be delivered to a membrane-based detection system by methods including, but not limited to, activation of a fluid delivery system.

In some embodiments where the cartridge is designed for analysis of blood samples, one or more membranes may be used to achieve separation of various whole blood components. For example, after the whole blood sample is provided to the membrane, WBCs may remain on the surface of the membrane, while other components of the blood sample (*e.g.*, RBCs and/or plasma) move through the membrane toward a waste reservoir or along one or more paths for further analysis. Cellular components (*e.g.*, WBCs) on the surface of the membrane may be washed or otherwise treated or assessed (*e.g.*, counted). In some embodiments, one or more reagents (*e.g.*, one or more WBC-specific antibodies labeled with an indicator molecule) may be provided to the membrane by one or more fluid delivery systems. In certain embodiments, reagents provided to a sample may be filtered, reconstituted, or otherwise processed in a portion of the cartridge. The portion of the blood sample that passes through the membrane may be directed toward an additional membrane for filtering. For example, a second membrane may remove RBCs from the blood sample. In some embodiments, RBCs may be further processed (*e.g.*, lysed or recovered) and assessed by polymerase chain reaction (PCR), hematocrit count/calculation, and/or other tests.

In some embodiments, a portion of the blood sample that is substantially free of particulate (*e.g.*, cellular) components may be directed toward a particle-based detection system for further analysis. For example, plasma may be directed toward a particle-based detection system that includes particles designed to detect specific proteins in the plasma. For example, particles designed to detect CRP may include CRP-capturing antibodies coupled to the particles. In some embodiments, one or more reagents may be delivered to the particle-based detection system by mechanisms including, but not limited to, fluid packages, reagent pads, or mini-pumps. In certain embodiments, a reagent delivered to a particle-based detection system may include one or more labeled antibodies. The amount and/or identity of the analytes may be assessed using an analyte-detection system. In some embodiments, the cartridge may be positioned, manually or automatically, to allow an analyte-detection system to analyze a membrane-based detection system. The cartridge may then be repositioned, manually or automatically, in the analyte-detection system to allow analytes in the particle-based detection system to be assessed.

As a non-limiting example of a multi-functional detection system, an analyte-detection system was used for the concurrent measurement of both CRP and WBCs. The analyte-detection system included a multi-functional cartridge. The cartridge included a particle-based membrane detection system and a membrane-based detection system. The membrane-based detection system was configured to capture and detect blood cells, while the particle-based detection system was configured to interact with blood proteins. The detection systems were each coupled to a fluid delivery system. The two detection systems shared a common computer. The computer controlled fluid delivery systems and optical components. The fluid delivery systems provided fluids for the analysis. The optical components assisted in microscopic evaluation of signals collected from the two detection systems.

The particle-based detection system of the cartridge was used to perform a CRP-specific immunoassay. The particle based detection system included porous agarose microparticles positioned in a micro-etched array (3x3 array) of wells on a silicon wafer microchip. Three particles, coated with antibodies irrelevant to CRP, were used as negative controls. The other six particles were dedicated to CRP capture and detection. Rabbit CRP-specific antibodies were coupled to the particle to capture the CRP antigen. This level of particle redundancy increased the statistical significance and, hence, the precision and accuracy of the CRP measurements. AlexaFluor® 488 labeled antibodies were employed to visualize the particle-captured protein.

A portion of the blood sample was introduced to the particle-based detection system, and the particles were washed with PBS. Low internal volumes of each particle (about 2 nL to about 30 nL per bead) used in conjunction with high effective flow rates (1-5 mL/min) allowed for the completion of highly stringent washes (>5000 effective washes per minute). The wash efficiently reduced nonspecific binding of antigens and detecting antibody reagents to the particles.

After washing, an image of the particle array was acquired in the following manner. Using standard epi-illumination geometry, white light from a 100-W mercury lamp was collimated, passed through a filter to select the excitation wavelengths centered at 480 nm with a 40 nm spectral bandwidth, reflected by a dichroic mirror (505 nm long pass mirror), and focused onto the particle array using a 4x microscope objective (NA of about 0.13). The fluorescence from the particles was collected by the microscope objective, transmitted through the dichroic mirror, passed through an emission filter centered at 535 nm with a 50 nm spectral width and detected by a CCD camera. The image was digitally processed and analyzed, and the signal intensity converted for each particle into a quantitative CRP measurement with the aid of a calibration curve. The time required to process the sample was approximately 12 minutes.

The particle-based detection region was washed with PBS and another image was acquired. Each assay of the sample was followed by a wash with PBS.

The particle-based CRP assay generally exhibited a detection range of at least 1 ng/ML up to 10,000 ng/mL. With the appropriate choice of assay conditions, use of particles coated with varying concentrations of capturing antibody, and/or use of sample dilution, the detection range for CRP was estimated to be expandable up to 100,000 ng/mL.

The above-described particle-based CRP assay was validated against a commercial high sensitivity-CRP enzyme limited immunosorbent assay (ELISA). CRP values from 9 human blood samples evaluated in parallel by ELISA and the particle-based method were in determined to be in agreement with each other.

A portion anti-coagulated blood sample was fixed with 4% paraformaldehyde, and then incubated for 5 minutes with an AlexaFluor® 488 labeled anti-CD45 antibody specific for WBCs. Coagulation of blood may be inhibited by adding an anti-coagulating agent to the blood sample (*e.g.*, heparin or ethylenediaminetetraacetic acid (EDTA)). The mixture was diluted with PBS and introduced to a membrane of the membrane-detection system with the use of an external peristaltic pump equipped with an injection valve. The membrane was a supported 13 mm track-etched polycarbonate membrane. Image acquisition was performed as described above for the particle-based detection system. Analysis of the scanning electron micrographs of the filtered whole blood revealed that RBCs, with roughly the same diameter as the WBCs, deformed and passed through the 3.0 micrometer pores of the membrane while WBCs were captured on the membrane. After removal of the RBCs, the WBCs were stained with anti-CD45 antibody. Two populations of cells were observed. One population of cells was brighter than the second population of cells captured on the membrane.

To evaluate the linearity and analytical range of the membrane WBC assay, increasing volumes of a CD45-stained whole blood suspension were delivered to the membrane-based detection system. Following a rinse with PBS, images of the WBCs on the membrane were captured at 3 different fields of view (FOV) on the membrane. A pixel analysis algorithm, as described in U.S. Patent Application No. 10/522,499, was applied to identify and count individual WBC based on size, shape, and fluorescence intensity thresholding within the image J environment. From the images, it was determined that the WBC counts increased in a linear fashion with an increasing volume of blood delivered to the flow cell. The coefficient of variation (CV) of the counts measured in different FOVs (intra-assay precision) was found to be within the range of 5% to 15%, and was dependent on the volume of blood delivered on the membrane. Optimal precision with the above-described cell structure was achieved for volumes of blood between 0.81 $\mu$L and 14.3 $\mu$L.

To evaluate the inter-assay precision of the WBC assay, the equivalent of 2.1 $\mu$L of stained whole blood was delivered to the membrane-based detection system. For healthy donors with 5000 to 11,000 WBCs/$\mu$L, this volume of

blood includes 10,500 to 23,100 WBCs. With the optical instrumentation described above, one FOV represented an area of 0.60 mm$^2$. Given that the total surface area of the membrane utilized for cell capture is 78.54 mm$^2$, the current membrane element was estimated to yield about 130 FOVs. Consequently, while the entire sample volume yields 10,500 to 23,100 FOVs, the single FOV collected a fluorescence signature of about 80 to about 176 cells, assuming that the cells were evenly distributed across the entire membrane.

Images from 5 non-overlapping FOVs were captured to get the preliminary mean WBC count. The preliminary count was converted to an absolute count after application of a scaling factor that incorporated the volume of blood delivered to the flow cell, as well as the number of FOVs covering the membrane-based detection system onto which WBCs are captured. The experiment was repeated 5 times using different membrane-based detection systems of the same configuration. The inter-assay coefficient of variation of the counts from one membrane-based detection system to another membrane-based detection system was determined to be 4.3 %.

Additionally, the WBC counts achieved by the membrane counting method were in agreement (95%) with those determined by flow cytometry. Flow cytometry requires a larger blood sample size (100 μL) and an additional processing step to lyse the red blood cells. The excellent agreement between flow cytometry and membrane-based detection indicates that the assumption of even cell distribution on the membrane of the membrane-based detection system was accurate.

As shown by this example, an analyte-detection system that includes a particle-based detection system and a membrane-based detection system allows for enhanced CRP detection levels in whole blood and for separation, isolation and detection of white blood cells from whole blood.

This technique also provides an optical biosensor for rapid, non-invasive analysis of oral epithelial tumor biomarkers. The lab-on-a-chip membrane-based sensor is designed to capture cells from biological fluids or biopsy suspensions directly into a flow cell imaging chamber where the cells undergo assay-specific immunolabeling and optical imaging. Intensity contouring using custom image analysis macros identifies cells for measurement and correlation of multiple parameters on a single-cell basis. The detection assay integrates recently identified early tumor biomarkers, such as epidermal growth factor receptor and DNA aneuploidy, with traditional cellular examination, to provide a cancer-risk profile that encompasses a large range of tumor progression phenotypes, potentially increasing the method's sensitivity over conventional pathology.

Unlike immunophenotyping using a flow cytometer, the lab-on-α-chip optical biosensor can provide both a molecular and morphological pattern within individual cells and improve spatial resolution for high complexity measurements using subcellular features. In addition, the fixed position of cells on the membrane allows individual cells to be relocated and reanalyzed following additional staining procedures, thereby expanding the capacity of the biosensor for multiplexing. Ongoing efforts at sensor and analyzer miniaturization would make these immunophenotyping assays rapid, highly sensitive, and cost effective for point-of-care use, thus increasing access to diagnostic testing for optimal cancer treatment and recovery.

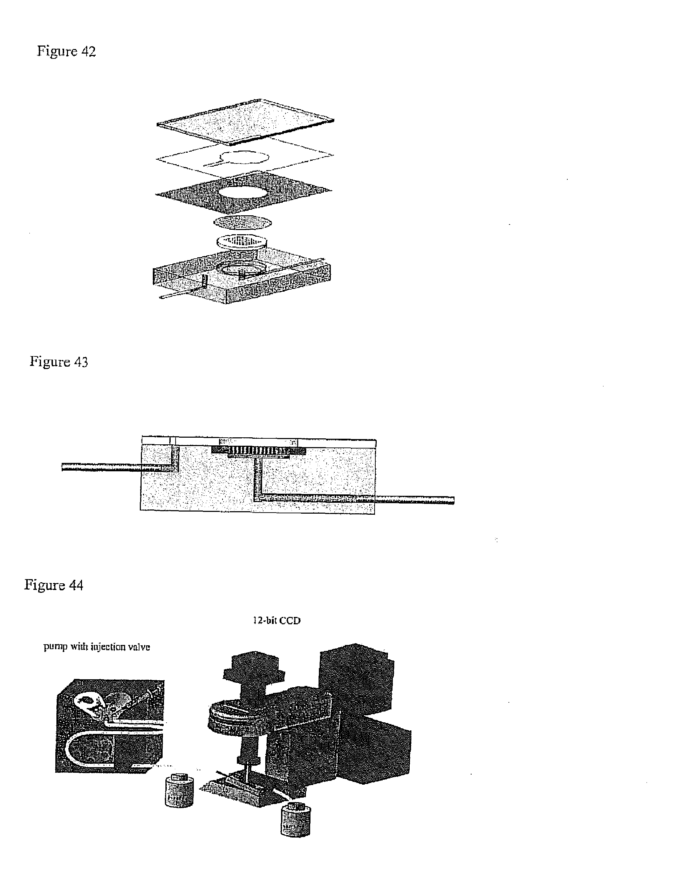

Referring to Figure 42, the membrane-based cell capture device is a multi-layered structure built upon a PMMA base with two fluid inlet and outlet channels. A 0.4mm Isopore™ membrane and support are placed within the base, sealed with adhesives containing precision cut fluid delivery channels and topped with a coverslip.

Referring to Figure 43, this flow-through design allows cell samples and reagents, delivered using a peristaltic pump injection valve, to enter through the side inlet in the PMMA base. From here, fluid travels up to the adhesive layers where a narrow channel directs fluid over the membrane then out using a bottom drain.

Referring to Figure 44, cells retained on the membrane are analyzed for protein and/or nucleic acids using assay-specific fluorescent labeling techniques while automated microscopy and digital imaging with a CCD camera allows quantitation of the fluorescent signal in an X,Y,Z membrane scan.

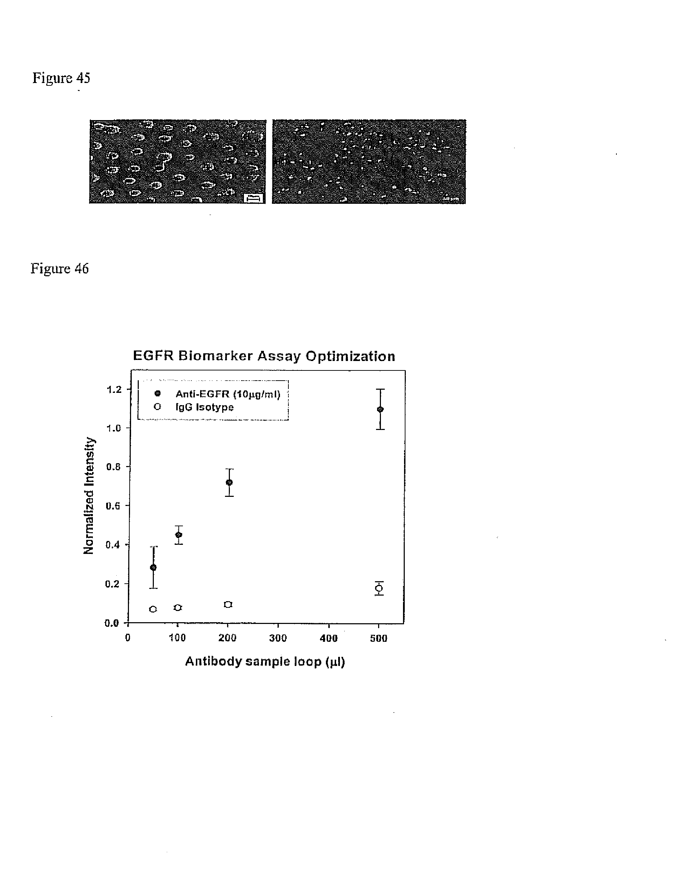

Referring to Figure 45, together, the LOC sensor and instrument system combine cell capture, processing, and analysis onto a unified platform for rapid detection of disease-associated biomarkers in multi-parameter single-cell assays.

Referring to Figure 46, early tumor targets of the lab-on-α-chip sensor include EGFR which is significantly elevated in 80-100% of all head and neck cancers including oral epithelial cancer.

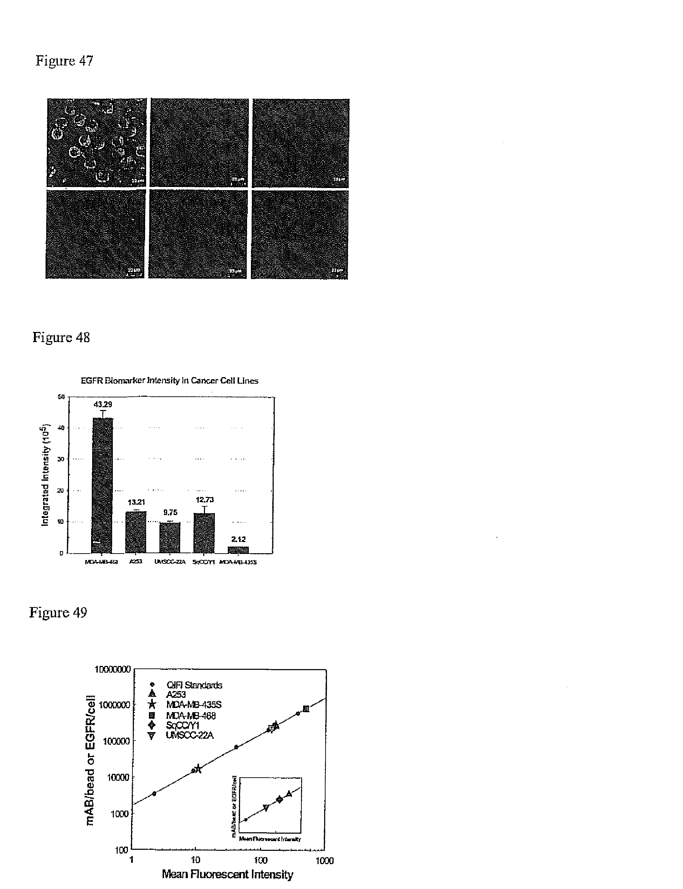

Referring to Figure 47, optimized EGFR biomarker detection in the LOC sensor is accomplished in <15 min. with a labeling intensity equivalent to that of cells processed using a flow cytometry protocol requiring a minimum of 2 hrs.

Referring to Figure 48, image intensity analysis is performed using binary segmentation and custom intensity contouring to measure total EGFR labeling on a single-cell basis, which is directly proportional to EGFR expression.

Referring to Figure 49, the number of EGFR receptors per cell was determined for all tumor cell lines by quantitative flow cytometry using QIFI bead standards (Dako Cytomation). Approximately 300,000 receptors per cell were labeled for the oral epithelial cell lines A253, SqCC/Y1 and UMSCC-22A. Positive control MDA-MB-468 cells are shown to possess over 800,000 EGFR/cell and MDA-MB-435S only 15,000 EGFR/cell, similar to that previously reported for normal epithelium.

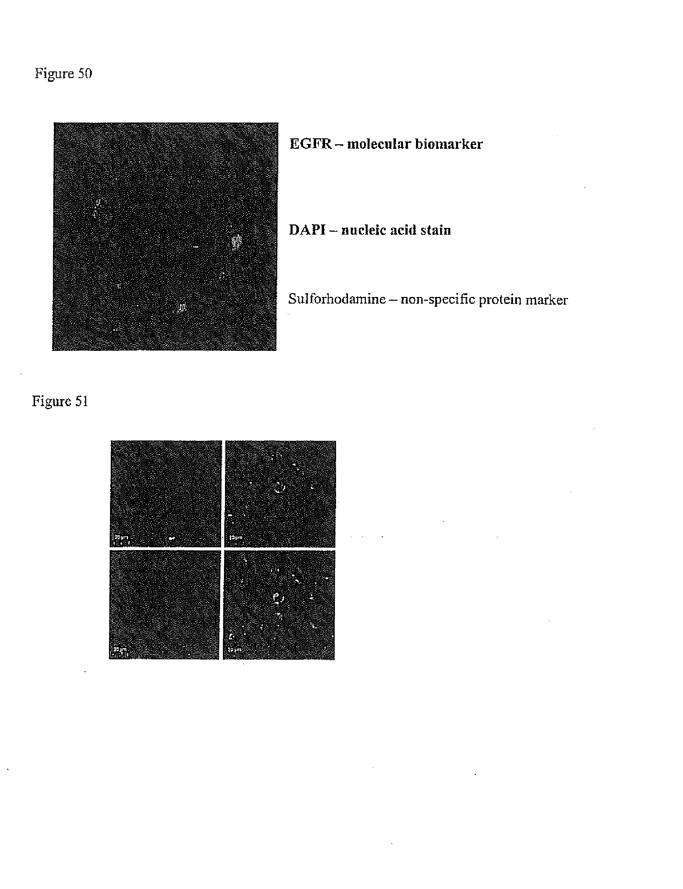

Referring to Figure 50, multi-spectral labeling expands the LOC sensor capacity for multi-parameter analysis combining cutting-edge biomarker discovery with traditional pathology for tumor detection at early developmental stages. Immediately following biomarker immunolabeling (EGFR-green) cells are treated with a staining cocktail of sulforhodamine (SR101- red) and DAPI (blue).

Referring to Figure 51, together, SR101 protein marker and DAPI nucleic acid stain provide a direct measure of cellular morphology including nuclear and cellular area, diameter, and density. Particularly important is the nuclear/cytoplasm ratio which is significantly elevated in hyperplastic cells as analyzed in traditional pathology.

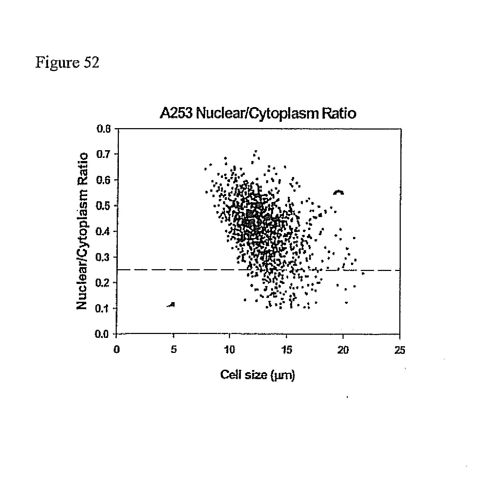

Referring to Figure 52, morphological analysis of A253 oral squamous cell carcinoma cells using the LOC sensor identifies 88.1% of these cells with significantly elevated nuclear/cytoplasm ratio compared to normal cells with a typical ratio of 1:4.

Invasive oral squamous cell carcinoma is often preceded by premalignant lesions appearing as white or red patches with varying degrees of epithelial dysplasia. Malignant transformation occurs in about 5-18% of these dysplasias; however, many are asymptomatic and innocuous in appearance.

Screening these lesions using the membrane-based sensor from a non-invasive brush biopsy for morphological characteristics of dysplasia and molecular biomarkers, such as EGFR, could improve identification of high-risk premalignant lesions.

Additional molecular parameters include which can be analyzed are the following: cell proliferation markers - PCNA, DNA content and ploidy status, Human Papillomavirus (high-risk HPV-16 and HPV-18)

The membrane-based sensor detects elevated levels of EGFR biomarker in oral squamous cell carcinoma cell lines using a rapid, highly-sensitive immunoassay. The LOC sensor provides several benefits over conventional immunophenotyping via flow cytometry such as:

1. Reduced assay time,

2. Analysis of small sample sizes (<10,000 cells),

3. Elimination of cell loss during centrifugation and washing.

Multi-parameter molecular and morphological analysis identifies tumor-derived cells with an elevated nuclear/cytoplasm ratio for further correlation on a single-cell basis.

In a non-limiting embodiment, studies were under taken using the principles described herein. The materials and methods, and results of the study are provided below.

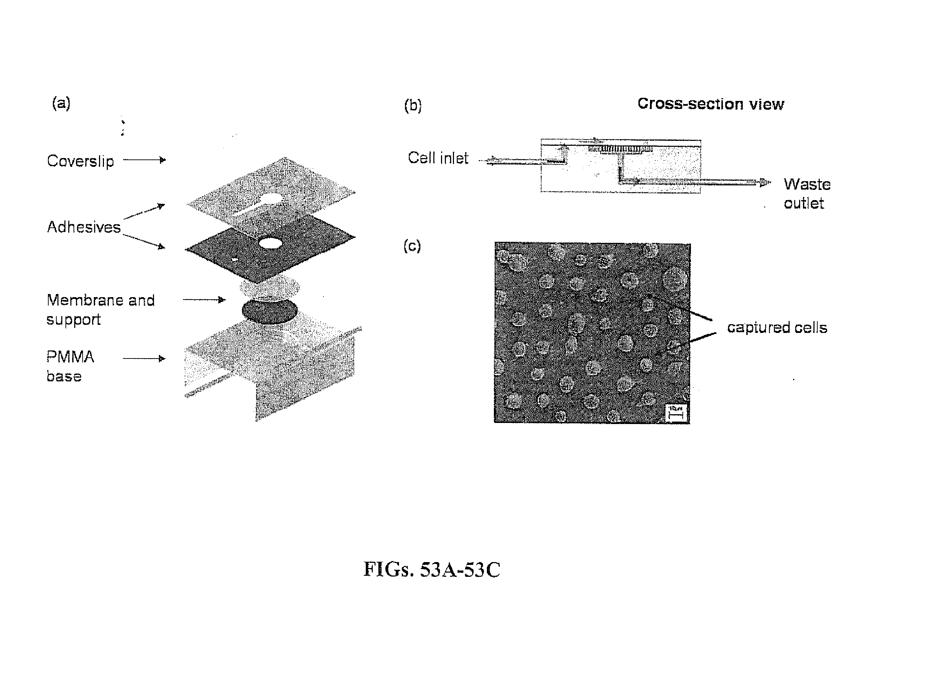

**Sensor Design and Instrumentation** - The cell-based LOC sensor is a multi-layered structure built upon a poly-methyl methacrylate (PMMA) base containing two fluid inlet and outlet channels (FIG. 53A). A 0.4 $\mu$m Isopore™ (Millipore, Billerica, MA) polycarbonate, track-etch membrane and support were embedded within the base, sealed with laminate adhesives containing precision cut fluid delivery channels and topped with a glass coverslip. Fluid and sample delivery was facilitated by a peristaltic pump with 6-port injection valve at flow rates between 250 $\mu$l/min-725 $\mu$L/min. Cells retained on the membrane were analyzed for protein and/or nucleic acids using assay-specific fluorescent labelling techniques as described below.

Digital micrographs were obtained in an (X, Y) membrane scan using a 20X (0.4 NA) or 10X (0.3 NA) objective, on an automated Olympus BX-61 modified epifluorescent microscope with motorized X,Y,Z stage (Prior, Rockland, MA), and 12-bit monochrome CCD camera (Q-Imaging, British Columbia) controlled via Simple PCI software (Compix Inc., Sewickley, PA).

Automated image analysis was performed using ImageJ (Rasband 1997-2006) open-source software with custom written macros for quantitative intensity standardization and cell contouring. All fluorescent images were shade corrected for uneven illumination using FITC/GFP Fluor-Ref reference slide (Microscopy Education, Allen, TX) with homogeneous intensity according to Varga *et al.* (2004) using equation 1:

$$I'_{x,y} = I_{x,y} * W_{mean}/W_{x,y}$$

where I' and I are the corrected image and the original image respectively; W is the bright (or white) reference image and x,y denotes the pixel location in each image. Relevant objects in each field were identified using a basic screen according to size and intensity to generate individual 8-bit single-object images. Next, auto-segmentation with an Otsu thresholding algorithm was performed to define the intensity contour and area-of-interest (AOI) of each object followed by local background subtraction. Measurement parameters included AOI area, perimeter, circularity, minimum and maximum intensity, mean, standard deviation, mode, and integrated intensity. For statistical analysis, data was exported to Microsoft® EXCEL® with Analyse-It® (Analyse-It Software Ltd., Leeds, UK) software and SigmaPlot 9.0 (Systat Software Inc., San Jose, CA) for graphical representation.

*In vitro* **Cell Culture** - Three human oral tumor-derived cell lines were utilized in this study including A253 adenocarcinoma from the submaxillary saliva gland, obtained from the American Type Culture Collection (ATCC); SqCC/Y1 from the bucal mucosa; and UMSCC-22A from the hypopharynx, both squamous cell carcinomas generously provided by Dr. Rebecca Richards-Kortum at Rice University. Cells were maintained in recommended culture media (McCoy's 5A, DMEM-F12, and EMEM respectively) containing 2 mM L-glutamine, 10% fetal bovine serum and 50 $\mu$g/mL penicillin/streptomycin at 37°C with 5% $CO_2$ in a humidified environment. Two breast adenocarcinoma cell lines, MDA-MB-468 and MDA-MB-435S (ATCC), were maintained in Leibovitz's L-15 media, supplemented as above, at 37°C under atmospheric conditions. All cells were seeded at 0.5 - 1x10$^4$ cells/cm$^2$ in T-75 flasks and allowed to grow for 3-5 days until cells reached ~80% confluency. Adherent cells were harvested using 0.25% Trypsin/EDTA solution, washed twice in 3mL PBS by centrifugation at 150 g for 5 minutes and fixed in 1 mL Histochoice™MB fixative (Electron Microscopy Sciences, Hatfield, PA) for 20 minutes at room temperature then stored at 4°C for up to two weeks.

**Flow Cytometry and Pre-labelling EGFR Protocol** - EGFR labelling was performed using an indirect flow cytometry protocol for cell surface antigens as previously described by Hsu *et al.* (2004). Briefly, 1 x10$^6$ - 2x10$^6$ cells were incubated in anti-EGFR mouse monoclonal antibody (10 $\mu$g/ml in PBS with 0.1% BSA, NeoMarkers, Freemont, CA) for 1 hr. at room temp. followed by goat antimouse IgG F(ab')$_2$ conjugated to AlexaFluor®488 (20 $\mu$g/ml in PBSA, Molecular Probes, Eugene, OR) for 45 minutes. Intermediate washing was carried out twice in 2 mL PBSA by centrifugation at 150 x g for 5 minutes. Negative controls replaced anti-EGFR with an irrelevant antibody of the same isotype (10 $\mu$g/ml Mouse IgG$_1$ in PBSA, Sigma, St. Louis, MO). Optimal primary and secondary antibody concentrations for use in flow cytometry and LOC assays were determined by titration (data not shown). All labelled cell samples were analyzed on a Beckman Coulter FC500 flow cytometer or delivered directly to the LOC sensor serving as "pre-labelled" samples for comparison of immunoassay techniques.

**Assay Development in LOC Sensor** - In order to determine the optimal assay parameters for the LOC sensor, a labelling intensity curve was generated using A253 cells incubated for various periods of time (0.2-2 minutes) in primary and secondary antibodies with a 2 minute intermediate buffer wash. Incubation time was manipulated by increasing the antibody sample loop size from 50-500 $\mu$l while maintaining a constant flow rate of 250 $\mu$l/min to avoid introducing variations in flow dynamics within the microfluidic channels and membrane chamber. Mean EGFR intensity was expressed as a percentage of the intensity obtained from pre-labelled A253 cells imaged and analyzed in LOC sensor under the same conditions. All assays were performed in triplicate with matched isotype controls.

In order to ensure homogeneous labelling across the membrane surface, the sample distribution and variation in integrated intensity of bead standards labelled in LOC sensor versus those pre-labelled in centrifuge tube was examined. Bead standards were obtained from QIFI quantitative flow cytometry kit (see below) set solution. Data acquisition and analysis was performed as described using a 10X objective with an 8 x 8 raster pattern scan of the membrane surface. Events were gated according to AOI area and max pixel intensity followed by single-parameter histogram analysis of log-transformed integrated intensity values to obtain the geometric mean and CV of each population.

**EGFR in Tumor Cell Lines** - Capacity of the LOC sensor to detect EGFR over-expression was examined in oral tumor cell lines using the membrane-based sensor immunoassay protocol above. The MDA-MB-486 cells known to overexpress EGFR at approximately 1x10$^6$ receptors/cell (Hsu *et al.,* 2004; Anido *et al.,* 2003) served as a positive immunolabelling control while MDA-MB-435S cells which express no or low levels of EGFR served as a negative control (Anido *et al.,* 2003; Kimmig *et al.* 1997; Cowley *et al.,* 1986). Data acquisition and analysis was performed as described using a 20X objective with a 5 x 10 membrane scan pattern. Integrated intensity data was not normally distributed; therefore, log transformation was applied to data prior to statistical analysis and presented in original scale for convenience. Mean integrated intensity values of triplicate biomarker assays were reported and 1-way pairwise ANOVA performed to determine statistically significant ($p < 0.05$) differences among cell populations. Parallel cell samples were labelled and analyzed via standard flow cytometry. Correlation of mean fluorescent intensity (MFI) obtained from flow cytometry and LOC sensor (calculated as mean integrated intensity in image cytometry) was assessed using linear regression with 95% confidence interval.

**Quantitative Flow Cytometry** - Quantitative flow cytometry using QIFIKIT® (Dako Cytomation, Denmark) was performed on all cell lines to determine how many receptors per cell were being labelled. The QIFIKIT® consists of a series of bead standards with defined amounts of anti-CD5 monoclonal antibody immobilized on the surface to mimic cells labelled with primary antibody to surface antigens. Binding of fluorescently labelled secondary antibodies generated an intensity calibration curve from which the number of receptors per cell were interpolated. Bead standards and cell samples were labelled in parallel under the same conditions according to the flow cytometry protocol above. Data acquisition and analysis, including linear regression of calibration curve and calculation of antigen density, was completed according to manufacturer's instructions.

**Membrane Cell Capture -** The LOC sensor utilized an embedded size-selective membrane, functioning as a microsieve, to capture and screen epithelial cells from culture suspensions or brush biopsy (FIG. 53A). The flow-through design allowed cell samples and reagents to enter through the side inlet in the PMMA base; from here, fluid travelled up to the adhesive layers where a narrow channel directed fluid over the membrane then out using a bottom drain (FIG. 53B). Cell capture was verified by scanning electron microscopy (SEM) of the membrane surface (FIG. 53C). Once captured, cells retained on the membrane were available for protein expression analysis using assay-specific fluorescent labelling. This simple yet elegant integrated microfluidic system is shown to be functional for both cell collection and cell staining steps provided as an example below.

**EGFR Biomarker Assay in LOC Sensor** - Mean fluorescence intensity of oral cancer cells immunolabelled in the LOC sensor for EGFR biomarker at increasing antibody incubation times under saturating conditions is demonstrated in FIG. 54A. At a constant flow rate of 250 $\mu$l/min, 2 minutes of sequential primary and secondary antibody incubation resulted in an EGFR labelling intensity of 110 $\pm$ 10 percent of pre-labelled EGFR intensity. Thus, these conditions were established as the optimal sensor immunolabelling parameters comparable to standard protocols. Ultimately, the EGFR assay was established in the LOC sensor as follows: (1) cell capture of 5,000-10,000 cells at 725 $\mu$l/min for 1 min.; (2) PBS wash for 2 min.; (3) 10 $\mu$g/ml anti-EGFR at 250 $\mu$l/min for 2 min.; (4) PBS wash for 2 min.; (5) 20 $\mu$l/ml AlexaFluor-488 conjugate at 250 $\mu$l/min for 2 min. immediately followed by automated imaging and analysis.

A surface plot of cell subsets from the LOC sensor assay at 2 min. and pre-labelled cells further demonstrates equivalent EGFR intensity obtained using the "on-membrane" sensor immunolabelling technique (FIG. 54B). In addition, the surface contour reveals cellular localization of EGFR primarily at the cell surface, as expected for a membrane-bound receptor, using both methods. Direct comparison of immunolabelling schemes exhibits a greater than 10-fold reduction in assay labelling time using the integrated microfluidic cell collector-detector methodology. Here EGFR biomarker detection was completed in 9 minutes, which accounts for cell capture, antibody incubation, and brief buffer washes between reagents, whereas the pre-labelling/flow cytometry protocol required 2 hrs and 5 min (FIG. 54C).

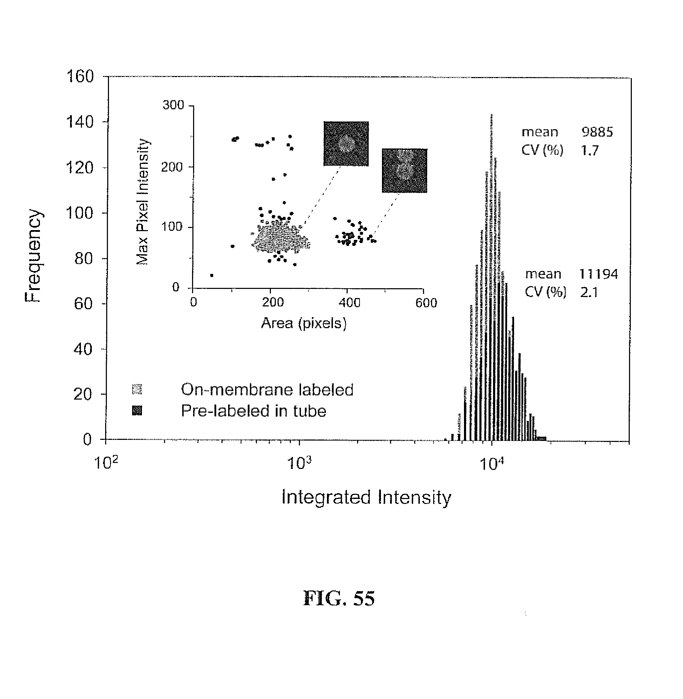

**Homogeneous Labeling in Sensor** - With the "on-membrane" sensor immunoassay technique it is important that all cells are adequately labelled regardless of their spatial location on the membrane. In order to ensure homogeneity, single-parameter intensity histograms of EGFR pre-labelled bead standards versus those labelled within LOC sensor were examined (FIG. 55). A gating scheme using area and maximum pixel intensity parameters excluded doublets and higher level aggregates along with debris from each data set as demonstrated in FIG. 55 inset. Comparison of the histogram CVs, which provide a measure of variation within each sample population, shows narrow peak distributions with a slightly lower CV found for the LOC sensor labelled beads (1.7%) versus beads pre-labelled in centrifuge tube (2.1%) (not analyzed for statistical significance). Hence, the LOC sensor provided homogeneous immunolabelling of the bead population across the membrane surface comparable to current in-tube labelling methods.

**EGFR in OSCC Cell Lines** - Fluorescent micrographs of EGFR detected in tumor cell lines using the LOC sensor are presented in FIG. 56A. Visually, EGFR expression was demonstrated in all oral cancer cell lines (ii-iv) and in MDA-MB-468 cells (i) known to overexpress EGFR. The MDA-MB-435S cells, previously reported to express no or very low levels of EGFR, demonstrated slight staining intensity (v) while isotype controls were indistinguishable from background fluorescence (vi). Subsequent automated image analysis revealed quantitative differences in EGFR intensity with significantly elevated levels found in MDA-MB-468, A253, SqCC/Y1, and UMSCC-22A cells compared to MDA-MB-435S negative control cells (FIG. 56B). The three oral cancer cell lines appeared to cluster at similar EGFR expression levels, although A253 cells exhibited a significant increase 1.5-fold over UMSCC-22A cells. In order to validate the LOC sensor assay methodology, parallel cell samples were stained and analyzed using flow cytometry, the gold standard in cellular protein expression analysis. The lab-on-$\alpha$-chip sensor EGFR immunoassay results displayed a high degree of correlation ($r^2$ = 0.98) with 95% confidence interval to conventional flow cytometry (FIG. 56C).

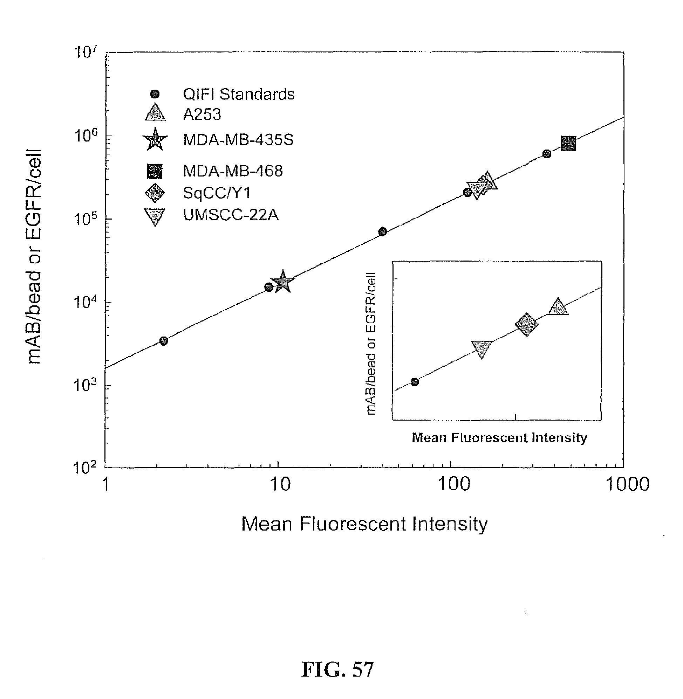

**Quantitative Flow Cytometry** - In order to further understand the cellular differences detected by the LOC sensor, the number of EGFR receptors per cell was determined in all cell lines by quantitative flow cytometry using QIFIKIT® analysis (FIG. 57). In agreement with the results achieved on the LOC system, MDA-MB-486 cells exhibited the highest EGFR expression at a density of 8.1 x $10^5$ receptors per cell, followed by A253 (2.7 x $10^5$ EGFR/cell), SqCC/Y1 (2.5 x $10^5$ EGFR/cell), UMSCC-22A (2.3 x $10^5$ EGFR/cell), and MDA-MB-435S cells with less than 0.2 x $10^5$ EGFR/cell. Although normal oral epithelium was not examined in this study, previous investigators have reported ~0.3 x $10^5$ EGFR binding sites per cell in normal cervical squamous epithelium (Kimming *et al.,* 1997) similar to MDA-MB-435S cells presented here.

Further details may be found in a U.S. Provisional Application 60/693,613, entitled "ANALYTE-DETECTION SYSTEMS AND METHODS INCLUDING SELF-CONTAINED CARTRIDGES WITH DETECTION SYSTEMS AND FLUID DELIVERY SYSTEMS," filed June 24, 2005 and naming John T. McDevitt, Karri L. Ballard, Nicolaos J. Christodoulides, Pierre N. Floriano and Glennon W. Simmons as co-inventors. Subsequently, a PCT application was filed claiming priority to this provisional (International Application No. PCT/US2006/024603),.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as the presently preferred embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

**Claims**

1. A membrane-based detection system 220 comprising a membrane 226, a bottom member 234 suitable to receive fluids flowing through membrane 226 and to conduct the fluids to outlet 240, a channel 238 in bottom member 234, an inlet 246 coupled to a top member 244 allowing fluids to enter membrane-based detection system 220, and a fastening member 250.

2. The membrane-based detection system according to claim 1, the system further comprising any one of the following:

   - a membrane support 228,
   - a bottom spacer 232,
   - a housing 230 positioned on a cartridge 100,
   - a gasket 242 suitable to reduce leaks from membrane-based detection system 220, wherein gasket 242 is optionally positioned between top member 244 and membrane 226,
   - a top spacer 248, optionally positioned between top member 244 and fastening member 250,
   - layers to direct fluid flow, preferably a plurality of layers which are positioned in cartridge 100 or on a surface of cartridge 100,
   - a cavity which is formed between top member 244 and membrane 226, wherein the cavity is formed with top member 244 which is spaced at a distance above membrane 226,
   - top member 244 has a shape such that a cavity is formed between top member 244 and membrane 266.

3. The membrane-based detection system according to any one of claims 1 or 2, wherein any one or more of the following features apply:

   - the thickness of membrane 226 is selected such that membrane 226 remains planar,
   - membrane 226 is porous,
   - membrane 226 captures or retains matter in the sample, such as particles or cells including lymphocytes, epithelial cells, precancer cells, cancer cells, $CD4^+$ cells, T-cells, NK cells, and B-cells, preferably on its surface and/or in membrane 226,
   - membrane 226 includes a thin film or layer capable of separating one or more components from a liquid passing through the film or layer, wherein the surface of membrane 226 is hydrophilic to promote cell proliferation across the surface of membrane 226,
   - membrane 226 is square, rectangular, circular, oval, and/or irregularly shaped,
   - membrane 226 includes openings, such as pores that inhibit an analyte of interest from passing through membrane 226,
   - membrane 226 is a monolithic microchip with a plurality of high-density holes, wherein the monolithic microchip membrane is preferably formed from materials selected from the group consisting of glass, silica/germanium oxide doped silica, inorganic polymers, organic polymers, titanium, silicon, silicon nitride, and/or mixtures thereof, and wherein the organic polymers are selected from the group consisting of PMMA, polycarbonate (PC) such as NUCLEOPORE® membranes (Whatman, Florham Park, NJ), and resins, such as Delrin®,
   - membrane 226 is formed of polymeric material and includes pores of a selected range of dimensions,
   - membrane 226 is an acrylic frit,
   - membrane 226 is formed of multiple layers, such as at least 2 layers, at least 3 layers, at least 4 layers, or at least 5 layers, of etchable and/or non-etchable glass,
   - membrane 226 and/or membrane support 228 are/is formed from an anti-reflective material and/or a material that does not reflect light in the ultraviolet-visible light range,
   - membrane 226 includes one or more locking mechanisms to assist in securing placement of membrane 226 in or on cartridge 100 or membrane support 228,
   - membrane 226 is a microsieve, preferably a layered plastic microsieve and/or a microsieve manufactured from silicon materials and/or plastic materials,
   - membrane 226 has a thickness from about 0.001 mm to about 25 mm, from about 1 mm to about 20 mm, or from about 5 mm to 10 mm, preferably a thickness ranging from about 0.001 mm to about 2 mm,
   - membrane 226 has a diameter from about 1 mm to 500 mm, from about 5 mm to about 100 mm, or from about 10 mm to about 50 mm,
   - the pores of membrane 226 have various dimensions, such as diameter and/or volume, preferably the pores of membrane 226 have approximately the same dimensions, or the pores have a diameter ranging from about 0.0001 mm to about 1 mm; from about 0.0002 mm to about 0.5 mm; from about 0.002 mm to about 0.1 mm, most preferably the pores have a diameter of at most 0.005 mm or at most 0.01 mm,

- the pores of membrane 226 are randomly arranged or arranged in a pattern, such as a hexagonal close-packed arrangement, preferably the pores of membrane 226 occupy at least 10 percent, at least 30 percent, at least 50 percent, or at least 90 percent of the surface area of membrane 226,
- membrane 226 is positioned from about 0.3 mm to about 0.5 mm below a top surface of cartridge 100.

4.   The membrane-based detection system according to any one of claims 1 to 3, wherein bottom member 234 further comprises indentation 236, optionally to receive membrane 226 and/or membrane support 228.

5.   The membrane-based detection system according to any one of claims 1 to 4, wherein bottom spacer 232 is suitable to position bottom member 234 in housing 230.

6.   The membrane-based detection system according to any one of claims 1 to 5, wherein top member 244 comprises a viewing window 142, preferably viewing window 142 is opaque or translucent to visible light and/or ultraviolet light, and/or top member 244 is at least partially transparent to visible light and/or ultraviolet light, optionally top member 244 is formed of PMMA.

7.   The membrane-based detection system according to any one of claims 1 to 6, wherein fastening member 250 is suitable to keep the components of membrane-based detection system 220 coupled during use and/or wherein fastening member 250 is machined to mate with housing 230.

8.   The membrane-based detection system according to any one of claims 1 to 7, wherein the plurality of layers comprise top layer 252, middle layer 254, and bottom layer 256, which are positioned according to the following order within membrane-based detection system 220: top member 244, top layer 252, middle layer 254, membrane 226, bottom layer 256, and membrane support 228; preferably the layers are coupled to each other, preferably top layer 252, middle layer 254, and bottom layer 256 include openings 258, 260, and 262, respectively; preferably top layer 252 is partially of a material or materials capable of coupling top layer 252 to middle layer 254, preferably the material is vinyl material and/or adhesive; preferably top layer 252 directs flow of fluid from top member 244 through opening 258 and towards membrane 226; preferably middle layer 254 is partially of a material or materials capable of coupling middle layer 254 to top layer 252 and/or bottom layer 256, preferably the material is a vinyl material and/or an adhesive; preferably middle layer 254 is opaque or translucent to visible light and/or ultraviolet light; preferably middle layer 254 directs fluid to flow through opening 260 toward membrane 226; preferably bottom layer 256 directs fluid flow through opening 262; preferably bottom layer 256 is partially of a material or materials capable of coupling bottom layer 256 to middle layer 254, preferably the material is a vinyl material and/or an adhesive; preferably opening 262 in bottom layer 256 has a size similar to the size of opening 260 allowing fluid to be retained in the area of membrane 226 between middle layer 254 and bottom layer 256; preferably one or more layers separate membrane 226 and membrane support 228, preferably membrane support 228 facilitates positioning of membrane 226 in or on cartridge 100.

9.   The membrane-based detection system according to any one of claims 1 to 8, wherein gasket 242 is positioned below bottom layer 256 to inhibit leaks from membrane-based detection system 220.

10.  The membrane-based detection system according to any one of claims 1 to 9, wherein any one or more of the following features apply:

- membrane support 228 inhibits sagging of membrane 226,
- membrane support 228 is positioned in bottom member 234 and/or in an opening of cartridge 100,
- membrane support 228 is coupled to cartridge 100 or integrated within cartridge 100,
- membrane support 228 is used to maintain membrane 226 in a substantially planar orientation,
- membrane support 228 is integrated with one or more membranes 226,
- membrane support 228 is formed of the same material as membrane 226,
- membrane support 228 is formed of materials selected from the group consisting of glass, polymers, metal, silicon, PC, cyclic olefin copolymer (COC), nylon, and/or nitrocellulose,
- membrane support 228 is a stainless steel filter or a plastic mesh,
- membrane support 228 is coupled to a support assembly to allow membrane 226 and membrane support 228 to withstand backpressures of at least 10 psi, preferably membrane support 228 is selected to produce a predetermined backpressure,
- membrane support 228 includes open areas, such as pores or holes, preferably the open areas in membrane support 228 have any shape, such as substantially square and/or substantially circular, wherein the shape of the open areas in membrane support 228 is preferably different than the shape of the pores in membrane 226,
- the diameter of the open areas of membrane support 228 is equal to or greater than the diameter of the pores of membrane 226, preferably membrane support 228 has open areas with diameters ranging from about 0.0001 mm to about 1 mm, from about 0.0002 mm to about 0.5 mm, from about 0.002 mm to about 0.1 mm, particularly preferred open area diameters are at most 0.005 mm or at most 0.01 mm.

11. A cartridge 100 including one or more collection regions, one or more fluid delivery systems, one or more channels, one or more reagent regions, one or more reservoirs, a detection region, or combinations thereof.

12. The cartridge according to claim 11, wherein the detection region includes one or more detection systems, in particular the detection system of any of claims 1 to 10.

FIG. 1

FIG. 2

FIG. 3

FIG. 4

FIG. 5

FIG. 6

FIG. 7

*FIG. 8*

*FIG. 9*

*FIG. 10*

*FIG. 11*

*FIG. 12*

FIG. 13

FIG. 14

*FIG. 15*

*FIG. 16*

*FIG. 17*

150

152

188

FIG. 18

152

150

198

194

188

106

190

189

FIG. 19

152

204

204

FIG. 20

150

154

205

208

206

202

FIG. 21

FIG. 22A

FIG. 22B

FIG. 23

FIG. 24

218

*FIG. 25A*

218

*FIG. 25B*

218

*FIG. 25C*

218

*FIG. 25D*

218

*FIG. 25E*

218

*FIG. 25F*

218

*FIG. 25G*

218

*FIG. 25H*

FIG. 26A

FIG. 26B

FIG. 27

FIG. 28

FIG. 29

FIG. 30

FIG. 31

228

W

264    266    268

L

*FIG. 32*

228

264

260    268

*FIG. 33*

228

270

266

272

T

*FIG. 34*

228

270

274

266

272

*FIG. 35*

FIG. 36

FIG. 37

FIG. 38

FIG. 39A

FIG. 39B

*FIG. 40A*  *FIG. 40B*  *FIG. 40C*

*FIG. 41A*  *FIG. 41B*

*FIG. 41C*  *FIG. 41D*

Figure 42

Figure 43

Figure 44

12-bit CCD

pump with injection valve

Figure 45

Figure 46

**EGFR Biomarker Assay Optimization**

Figure 47

Figure 48

Figure 49

Figure 50

**EGFR – molecular biomarker**

**DAPI – nucleic acid stain**

Sulforhodamine – non-specific protein marker

Figure 51

Figure 52

(a)

Coverslip ⟶

Adhesives

Membrane and support ⟶

PMMA base ⟶

(b)          **Cross-section view**

Cell inlet

Waste outlet

(c)

captured cells

FIGs. 53A-53C

(a)

(b)

(c)

FIGs. 54A-54C

FIG. 55

FIGs. 56A-56C

**FIG. 57**

**European Patent Office**
Europäisches Patentamt
European Patent Office
Office européen des brevets

# EUROPEAN SEARCH REPORT

Application Number

EP 12 16 6973

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | WEIGUM SHANNON E ET AL: "Cell-based sensor for analysis of EGFR biomarker expression in oral cancer", LAB CHIP, vol. 7, 2007, pages 995-1003, XP002561398, * the whole document * | 1-8 | INV. C12Q1/00 G01N33/574 C12Q1/02 |
| X,P | S. E. WEIGUM ET AL: "Nano-Bio-Chip Sensor Platform for Examination of Oral Exfoliative Cytology", CANCER PREVENTION RESEARCH, vol. 3, no. 4, 1 April 2010 (2010-04-01), pages 518-528, XP55030662, ISSN: 1940-6207, DOI: 10.1158/1940-6207.CAPR-09-0139 * the whole document * | 1-12 | |
| X | WO 2005/085796 A2 (UNIV TEXAS [US]; MCDEVITT JOHN T [US]; ANSLYN ERIC V [US]; SHEAR JASON) 15 September 2005 (2005-09-15) * p. 5, line 27 - p. 12, line 29, p. 46, line 17 - p. 74, line 19, Fig. 1A-C, 19A-D, 27-30, 42-45 * | 1-12 | TECHNICAL FIELDS SEARCHED (IPC) G01N |
| X | WO 2004/009840 A1 (UNIV TEXAS [US]; MCDEVITT JOHN T [US]; FLORIANO PIERRE [US]; CHRISTODO) 29 January 2004 (2004-01-29) * p. 13, line 6 - p. 24, line 10, Fig. 1-3 * | 1-12 | |
| X | US 2004/245102 A1 (GILBERT JOHN R [US] ET AL) 9 December 2004 (2004-12-09) * p. 5, paragraph [0094] - p. 17, paragraph [0198], Fig. 1-8 * | 1-3,7, 10-12 | |

-/--

The present search report has been drawn up for all claims

| Place of search | Date of completion of the search | Examiner |
|---|---|---|
| Munich | 22 June 2012 | R. von Eggelkraut-G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt
European
Patent Office
Office européen
des brevets

**EUROPEAN SEARCH REPORT**

Application Number

EP 12 16 6973

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2005/036167 A1 (UNIV SINGAPORE [SG]; LIU WEN-TSO [SG]; ANG SIMON S [US]; ZHU LIANG [SG]) 21 April 2005 (2005-04-21) * the whole document * ----- | 1-12 | |

TECHNICAL FIELDS
SEARCHED      (IPC)

The present search report has been drawn up for all claims

| Place of search | Date of completion of the search | Examiner |
|---|---|---|
| Munich | 22 June 2012 | R. von Eggelkraut-G. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

................................................................................
& : member of the same patent family, corresponding
    document

EPO FORM 1503 03.82 (P04C01)

**ANNEX TO THE EUROPEAN SEARCH REPORT
ON EUROPEAN PATENT APPLICATION NO.**

EP 12 16 6973

This annex lists the patent family members relating to the patent documents cited in the above-mentioned European search report.
The members are as contained in the European Patent Office EDP file on
The European Patent Office is in no way liable for these particulars which are merely given for the purpose of information.

22-06-2012

| Patent document cited in search report | | Publication date | Patent family member(s) | | Publication date |
|---|---|---|---|---|---|
| WO 2005085796 | A2 | 15-09-2005 | US | 2006257993 A1 | 16-11-2006 |
| | | | WO | 2005085796 A2 | 15-09-2005 |
| WO 2004009840 | A1 | 29-01-2004 | AU | 2003256742 A1 | 09-02-2004 |
| | | | CA | 2494727 A1 | 29-01-2004 |
| | | | EP | 1546367 A1 | 29-06-2005 |
| | | | EP | 2107120 A1 | 07-10-2009 |
| | | | JP | 2005533502 A | 10-11-2005 |
| | | | US | 2006073585 A1 | 06-04-2006 |
| | | | US | 2006079000 A1 | 13-04-2006 |
| | | | WO | 2004009840 A1 | 29-01-2004 |
| US 2004245102 | A1 | 09-12-2004 | NONE | | |
| WO 2005036167 | A1 | 21-04-2005 | EP | 1682891 A1 | 26-07-2006 |
| | | | US | 2006046305 A1 | 02-03-2006 |
| | | | WO | 2005036167 A1 | 21-04-2005 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20020197622 A, McDevitt
- US 02217604 A, McDevitt
- US 11020443 B
- US 11020442 B
- US 11022365 B
- US 11021123 B
- US 11022219 B
- US 5096660 A, Lauks
- US 5837199 A, Dumschat
- US 6010463 A, Lauks
- US 020442 A
- US 11022365 A
- US 11021123 A
- US 11022219 A
- US 6908770 B, McDevitt
- US 6680206 B
- US 6602702 B
- US 6589779 B
- US 6649403 B
- US 6713298 B

- US 20020160363 A
- US 20040029259 A
- US 20030064422 A
- US 20030186228 A
- US 20040053322 A
- US 20050136548 A
- US 20050164320 A
- US 20050214863 A
- US 61673100 A
- US 10522499 B
- US 10470646 B
- US 10522926 B
- US 10544864 B
- US 10544954 B
- US 616731 A
- US 20020064422 A
- US 4196265 A, Koprowski
- US 522499 A
- US 69361305 P
- US 2006024603 W

**Non-patent literature cited in the description**

- IMMUNOLOGY. Mosby/Times Mirror International Publication, 1998
- IMMUNOBIOLOGY: THE IMMUNE SYSTEM HEALTH AND DISEASE. Garland Publishing, Inc, 2001

- **E. HOWELL ; D. LANE.** Antibodies: A Laboratory Manual. Cold Spring Harbor Laboratory, 1988